# EUROPEAN PATENT APPLICATION

(11) **EP 4 704 022 A1**
(43) Date of publication of application: **04.03.2026**
(21) Application number: 24797153.4
(22) Date of filing: 25.04.2024
(51) Int. Cl.: G06Q 50/10, A63H 3/02, A63H 5/00, A63H 11/00, A63H 13/00, B25J 13/00, B25J 13/08, G06F 3/01, G06F 3/16, G06F 16/9035, G06N 3/008, G06N 5/04, G06Q 10/00, G06Q 10/04, G10L 13/00, G10L 13/08, G10L 15/00, G10L 15/22, G10L 25/63, G16H 20/00, G16H 20/70

(54) **BEHAVIOR CONTROL SYSTEM AND ELECTRONIC DEVICE**

(30) Priority: 25.04.2023 JP 2023071547; 26.04.2023 JP 2023072215; 26.04.2023 JP 2023072562; 27.04.2023 JP 2023073620; 27.04.2023 JP 2023073756; 27.04.2023 JP 2023073783; 28.04.2023 JP 2023074278; 28.04.2023 JP 2023074279; 28.04.2023 JP 2023074460; 28.04.2023 JP 2023074461; 28.04.2023 JP 2023074938; 28.04.2023 JP 2023074939; 28.04.2023 JP 2023074940; 28.04.2023 JP 2023075014
(71) Applicant: SoftBank Group Corp., Tokyo 105-7537 (JP)
(72) Inventor: SON, Masayoshi, Tokyo 105-7537 (JP)
(74) Representative: SSM Sandmair
(86) International application number: PCT/JP2024/016353
(87) International publication number: WO 2024/225408

(57) **Abstract**

An action control system includes: a detection unit that detects occurrence of an event which is an event occurring in a circumstance of a user and which is an event that causes a negative emotion in the user; and an output controller that controls a robot including a text generation model to output information corresponding to the event detected by the detection unit to the user.

## Description

### Field

The present invention relates to an action control system and an electronic device.

### Background

Patent Literature 1 discloses a technique of deciding an appropriate action of a robot in accordance with a state of a user. The known technology of Patent Literature 1 recognizes user's reaction when the robot executes a specific action, and in a case where an action of the robot with respect to the recognized reaction of the user cannot be decided, information related to an action suitable for the recognized state of the user is received from the server to update the action of the robot.

### Citation List

### Patent Literature

Patent Literature 1: JP 6053847 A

### Summary

### Technical Problem

However, the known technology has room for improvement in output of information from an electronic device.

### Solution to Problem

According to a first embodiment, an action control system is provided. The action control system includes a detection unit that detects occurrence of an event related to a circumstance of a user; and an output controller that controls an electronic device including a text generation model to output information for adapting to the circumstance to the user in accordance with the event detected by the detection unit.

According to a second embodiment, an electronic device is provided. The electronic device includes an estimation unit that estimates an emotion of a user; an acquisition unit that acquires information related to health and medical care of at least one of the user and a family member of the user, and an assisting unit that performs assist corresponding to the emotion of the user estimated by the estimation unit, based on the information related to health and medical care of at least one of the user and a family member of the user acquired by the acquisition unit.

According to a third embodiment, an electronic device is provided. The electronic device includes a recognition unit that recognizes a state of a user, a physical condition estimation unit that estimates a physical condition of the user based on the state of the user recognized by the recognition unit, and an assisting unit that performs assist based on the physical condition of the user estimated by the physical condition estimation unit.

According to a fourth embodiment, an action control system is provided. The action control system includes a detection unit that detects occurrence of an event which is an event related to a circumstance of a user and which is an event that causes a negative emotion in the user; and an output controller that controls an electronic device including a text generation model to output information corresponding to the event detected by the detection unit, to the user.

According to a fifth embodiment, an electronic device is provided. The electronic device includes a control unit that recognizes an action of a user having a brain dysfunction, decides an own action corresponding to the recognized action of the user, and controls a control target based on the own action decided.

According to a sixth embodiment, an electronic device is provided. The electronic device includes a control unit that recognizes an action of an artist, decides an own action corresponding to the recognized action of the artist, and controls a control target based on the own action decided, wherein the control unit decides an action related to a performance of the artist.

According to a seventh embodiment, an electronic device is provided. The electronic device includes a recognition unit that recognizes a state of a child; an emotion estimation unit that estimates an emotion of the child based on the state of the child recognized by the recognition unit, and an assisting unit that performs assist corresponding to the emotion of the child estimated by the emotion estimation unit.

According to an eighth embodiment, an action control system is provided. The action control system includes an estimation unit that estimates a social situation of a user based on user information related to the user; and an output controller that controls an electronic device including a text generation model to output, to the user, information corresponding to the situation estimated by the estimation unit.

According to a nineth embodiment, an electronic device is provided. The electronic device includes a control unit that recognizes study information of a user and determines an emotion of the user, has a conversation with the user based on the emotion determined, and controls a control target to provide the user with information corresponding to the study information recognized.

According to a 10th embodiment, an electronic device is provided. The electronic device includes a control unit that recognizes a mental state of a user, determines an emotion of the user, has a conversation with the user based on the emotion determined, and controls a control target so as to perform care in accordance with the mental state recognized.

According to a 11th embodiment, an electronic device is provided. The electronic device includes an action decision unit that decides a personalized countermeasure for a user in accordance with a state of an emotion of the user.

According to a 12th embodiment, an electronic device is provided. The electronic device includes a control unit that recognizes an action of a user performing a training, determines an emotion of the user, and controls a control target to provide the user with information related to the training based on the recognized action and the determined emotion.

According to a 13th embodiment, an electronic device is provided. The electronic device includes a control unit that recognizes at least one of a state of a user constituting a family and a state of a pet kept by the user, decides an own action corresponding to the recognized state, and controls a control target based on the own action decided.

According to a 14th embodiment, an action control system is provided. The action control system includes a control unit that recognizes a state related to mind and body of a user, decides an own action corresponding to the recognized state, and controls a control target based on the own action decided.

### Brief Description of Drawings

FIG. 1 is a diagram schematically illustrating an example of a system 5 according to a first embodiment.
FIG. 2 is a diagram schematically illustrating a functional configuration of a robot 100.
FIG. 3 is a diagram schematically illustrating a data structure of character data 223.
FIG. 4 is a diagram schematically illustrating an example of an operation flow related to setting of a character.
FIG. 5 is a diagram schematically illustrating an example of an operation flow performed by the robot 100.
FIG. 6 is a diagram schematically illustrating a functional configuration of an event detection unit 290.
FIG. 7 is a diagram schematically illustrating an example of an operation flow performed by the event detection unit 290.
FIG. 8 is a diagram schematically illustrating an example of a hardware configuration of a computer 1200.
FIG. 9 is a diagram illustrating an emotion map on which a plurality of emotions is mapped.
FIG. 10 is a diagram illustrating another example of the emotion map.
FIG. 11 is a diagram illustrating an example of an emotion table.
FIG. 12 is a diagram illustrating an example of an emotion table.
FIG. 13 is an external view and an internal structural view of a stuffed toy according to another embodiment.
FIG. 14 is a rear-front view of a stuffed toy according to another embodiment.
FIG. 15 is a diagram schematically illustrating an example of an operation flow performed by a robot 100 according to a second embodiment.
FIG. 16 is a diagram schematically illustrating an example of an operation flow performed by a robot 100 according to a third embodiment.
FIG. 17 is a diagram schematically illustrating an example of an operation flow performed by a robot 100 according to a fifth embodiment.
FIG. 18 is a diagram schematically illustrating an example of a system 1 according to a seventh embodiment.
FIG. 19 schematically illustrates a functional configuration of an output unit 290a according to an eighth embodiment.
FIG. 20 schematically illustrates an example of an operation flow performed by the output unit 290a according to the eighth embodiment.
FIG. 21 schematically illustrates an example of a specific operation flow performed by a robot 100 according to an eleventh embodiment.

### Description of Embodiments

Hereinafter, the present invention will be described through embodiments of the invention, but the following embodiments do not limit the invention according to the claims. In addition, not all combinations of features described in the embodiments are essential to the solution of the invention.

### [First embodiment]

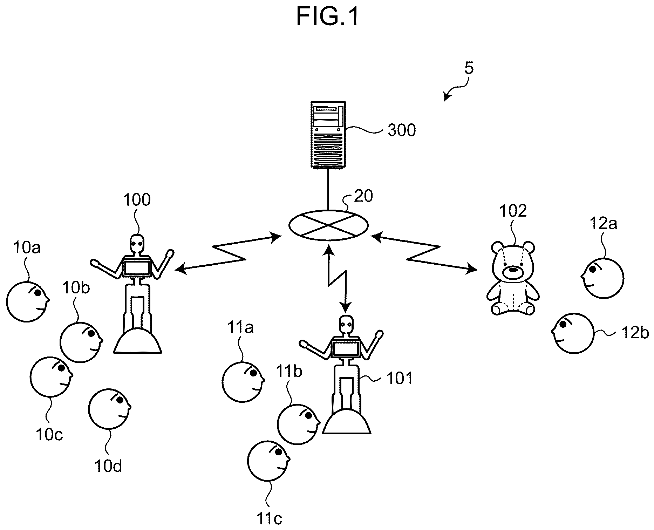

FIG. 1 schematically illustrates an example of a system 5 according to a first embodiment. The system 5 includes a robot 100, a robot 101, a robot 102, and a server 300. A user 10a, a user 10b, a user 10c, and a user 10d are users of the robot 100. A user 11a, a user 11b, and a user 11c are users of the robot 101. A user 12a and a user 12b are users of the robot 102. In the description of the first embodiment, the user 10a, the user 10b, the user 10c, and the user 10d may be collectively denoted as a user 10. Furthermore, the user 11a, the user 11b, and the user 11c may be collectively denoted as a user 11. The user 12a and the user 12b may be collectively denoted as a user 12. The robot 101 and the robot 102 have substantially the same functions as those of the robot 100. Therefore, the system 5 will be described mainly focusing on the function of the robot 100.

The appearance of the robot may imitate a figure of a person like the robot 100 and the robot 101, or may be a stuffed toy like the robot 102. The robot 102 has an external appearance of a stuffed toy, and thus is considered to be familiar to children in particular.

The robot 100 has a conversation with the user 10 and provides a video to the user 10. At this time, the robot 100 performs conversation with the user 10 and provides a video, etc. to the user 10 in cooperation with the server 300 and the like communicable via a communication network 20. For example, the robot 100 not only performs self-learning of appropriate conversations, but also performs learning in cooperation with the server 300 so as to achieve more appropriate conversations with the user 10. Furthermore, the robot 100 causes the server 300 to record the captured video data, etc. of the user 10, requests the video data, etc. from the server 300 as necessary, and provides the video data, etc. to the user 10.

Furthermore, the robot 100 has emotion values indicating types of its own emotions. For example, the robot 100 has emotion values indicating the intensity of each emotion of "delighted", "angry", "sad", "joyful", "pleasant", "unpleasant", "secure", "anxious", "sorrowful", "excited", "worried", "relieved", "fulfilled", "empty", and "neutral". For example, when the robot 100 has a conversation in a state of having a high emotion value of excitement with the user 10, the robot emits a voice at a high speed. In this manner, the robot 100 can express its own emotion by action.

Furthermore, the robot 100 may be configured to decide the action of the robot 100 corresponding to the emotion of the user 10 by using matching of a text generation model (also referred to as an Artificial Intelligence (AI) chat engine) with an emotion engine. Specifically, the robot 100 may be configured to recognize an action of the user 10, determine an emotion of the user 10 behind the action of the user, and decide an action of the robot 100 corresponding to the determined emotion.

More specifically, when having recognized an action of the user 10, the robot 100 uses a preset text generation model to automatically generate action content to be taken by the robot 100 with respect to the action of the user 10. The text generation model may be construed as an algorithm and an operation for automatic dialog processing with texts. Since the text generation model is known as disclosed in JP 2018-081444 A, for example, a detailed description thereof will be omitted. Such a text generation model is configured by a Large Language Model (LLM). As described above, in the first embodiment, by combining the Large Language Model and the emotion engine, it is possible to reflect the emotions of the user 10 and the robot 100 and various linguistic information in the action of the robot 100. That is, according to the first embodiment, a synergistic effect can be obtained by combining the text generation model and the emotion engine.

Here, the robot 100 detects occurrence of an event related to a circumstance of the user. In addition, the robot 100 outputs information corresponding to the detected event. For example, the events related to the circumstance of the user include user's becoming an international student, the job change of the user (starting working with a new company, changing to another job, getting employed again), the birth of a child of the user, the growth of the child of the user to reach a certain age, and the like.

The robot 100 holds an emotion corresponding to the occurrence of the event. Specifically, in a case where an event is detected, among a plurality of types of emotions prepared in advance as the emotions of the robot, in which a value can be set for each of the emotions, a value of the emotion of being secure (for example, "secure" in an emotion map to be described below) is to be changed. Subsequently, the robot 100 performs output corresponding to the value of the emotion.

This is for the purpose of giving advice or the like, by the robot, related to the circumstance of the user in a case where the robot 100 has detected the event described above. In order to give convincing advice, the robot 100 itself needs to be calm. The robot 100 may decrease a plurality of types of emotion values comprehensively instead of increasing the emotion value of secure, and may give advice with a calm and cool attitude without revealing its emotion excessively.

For example, the robot 100 outputs, by utterance, a text describing a method of adapting to a circumstance. This makes it possible for the robot 100 to promote the emotion of the user to change in the positive direction (toward secure and relieved).

Furthermore, the robot 100 has a function of recognizing an action of the user 10. The robot 100 recognizes the action of the user 10 by analyzing a face image of the user 10 acquired by a camera function and the voice of the user 10 acquired by a microphone function. The robot 100 decides an action to be executed by the robot 100 based on the recognized action of the user 10 or the like.

The robot 100 stores a rule defining an action to be executed by the robot 100 based on the emotion of the user 10, the emotion of the robot 100, and the action of the user 10, and takes various actions in accordance with the rule.

Specifically, the robot 100 has a reaction rule for deciding an action of the robot 100 based on the emotion of the user 10, the emotion of the robot 100, and the action of the user 10. The reaction rule defines, for example, that in a case where the action of the user 10 is "smiling", the action of the robot 100 is to be an action of "smiling". In addition, the reaction rule defines, for example, that in a case where the action of the user 10 is "getting angry", the action of the robot 100 is to be an action of "apologizing". Furthermore, the reaction rule defines, for example, that in a case where the action of the user 10 is "asking a question", the action of the robot 100 is to be an action of "answering the question". The reaction rule defines, for example, that in a case where the action of the user 10 is "expressing sorrow", the action of the robot 100 is to be an action of "offering words".

Based on the reaction rule, having recognized that the action of the user 10 is "getting angry", the robot 100 selects an action of "apologizing" prescribed in the reaction rule, as the action to be executed by the robot 100. For example, when selecting the action of "apologizing", the robot 100 takes an action of "apologizing" and outputs a voice expressing a word of "apologizing".

Furthermore, it is prescribed that, when a condition that the emotion of the robot 100 is "neutral" (that is, "delighted" = 0, "angry" = 0, "sad" = 0, and "joyful" = 0) and the state of the user 10 is "alone and looks sad" is satisfied, it is possible to execute an emotional change in which the emotion of the robot 100 turns to "worried" and an action of "offering words".

When the robot 100 recognizes that the current emotion of the robot 100 is "neutral" and the user 10 is alone and looks sad, the emotion value of "sad" of the robot 100 is increased based on the reaction rule. Furthermore, the robot 100 selects an action of "offering words" prescribed in the reaction rule as an action to be executed on the user 10. For example, when the action of "offering words" is selected, the robot 100 outputs a word "What's wrong?" indicating a concern in a concerned voice obtained by voice conversion.

Furthermore, the robot 100 transmits, to the server 300, user reaction information indicating that a positive reaction has been obtained from the user 10 by this action. The user reaction information includes, for example, a user action of "getting angry", an action of the robot 100 of "apologizing", a positive reaction of the user 10, and an attribute of the user 10.

The server 300 stores the user reaction information received from the robot 100. The server 300 receives and stores the user reaction information not only from the robot 100 but also from the robot 101 and the robot 102 individually. Subsequently, the server 300 analyzes the user reaction information from the robot 100, the robot 101, and the robot 102, and updates the reaction rule.

The robot 100 inquires the server 300 about the updated reaction rule to receive the updated reaction rule from the server 300. The robot 100 incorporates the updated reaction rule into the reaction rule stored in the robot 100. With this configuration, the robot 100 can incorporate the reaction rule acquired by the robot 101, the robot 102, or the like into its own reaction rule.

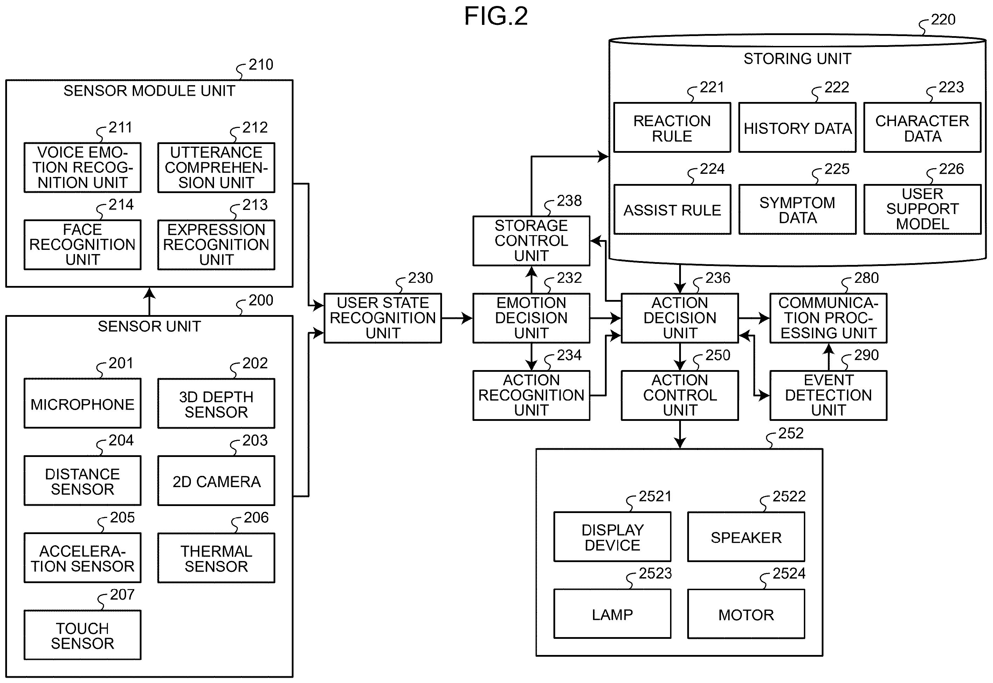

FIG. 2 schematically illustrates a functional configuration of a robot 100. The robot 100 includes a sensor unit 200, a sensor module unit 210, a storing unit 220, a user state recognition unit 230, an emotion decision unit 232, an action recognition unit 234, an action decision unit 236, a storage control unit 238, an action control unit 250, a control target 252, a communication processing unit 280, and an event detection unit 290.

The control target 252 includes a display device 2521, a speaker 2522, a lamp 2523 (for example, the LED for the eyes), and motors 2524 that drive parts such as arms, hands, and legs. The posture and gesture of the robot 100 are controlled by controlling the motors 2524 in the parts such as an arm, a hand, and a leg. Some of the emotions of the robot 100 can be expressed by controlling these motors 2524. The expression of the robot 100 can also be expressed by controlling the light emission state of the LED for the eyes of the robot 100. The posture, gesture, and expression of the robot 100 are examples of attitude of the robot 100.

The sensor unit 200 includes a microphone 201, a 3D depth sensor 202, a 2D camera 203, a distance sensor 204, an acceleration sensor 205, a thermal sensor 206, and a touch sensor 207. The microphone 201 continuously detects a voice and outputs voice data. The microphone 201 may be provided on the head of the robot 100 and may have a function of performing binaural recording. The 3D depth sensor 202 detects the contour of an object by continuously projecting an infrared pattern and analyzing the infrared pattern from the infrared image continuously captured by the infrared camera. The 2D camera 203 is an example of an image sensor. The 2D camera 203 captures an image with visible light and generates video information of visible light. The distance sensor 204 detects a distance to an object by projecting a laser and an ultrasonic wave, for example. The sensor unit 200 may further include a clock, a gyro sensor, a touch sensor, a sensor for motor feedback, and the like.

Among the components of the robot 100 illustrated in FIG. 2, the components other than the control target 252 and the sensor unit 200 are examples of components included in the action control system in the robot 100. The action control system of the robot 100 controls the control target 252 as a target.

The storing unit 220 includes a reaction rule 221, history data 222, character data 223, an assist rule 224, symptom data 225, and a user support model 226. The history data 222 includes past emotion values and action history of the user 10. The emotion value and the action history are recorded for each user 10 by being associated with identification information of the user 10, for example. At least a part of the storing unit 220 is implemented by a storage medium such as memory. A person DB that stores a face image of the user 10, attribute information of the user 10, and the like may be included. Among the components of the robot 100 illustrated in FIG. 2, the functions of the components other than the control target 252, the sensor unit 200, and the storing unit 220 can be implemented by the CPU operating based on a program. For example, the functions of these components can be implemented as the operation of the CPU by basic software (OS) and a program operating on the OS.

The character data 223 is data in which a character and an age are associated with each other. For example, the character is a person or the like appearing in a piece of content such as an existing animation, a video game, a cartoon, or a movie. Furthermore, the character may be an animal and a plant having a personality, or may be an inanimate object (such as a robot).

For example, the age (use age) associated with the character in the character data 223 is decided based on the age group of the viewer assumed as the target of the content in which the character appears.

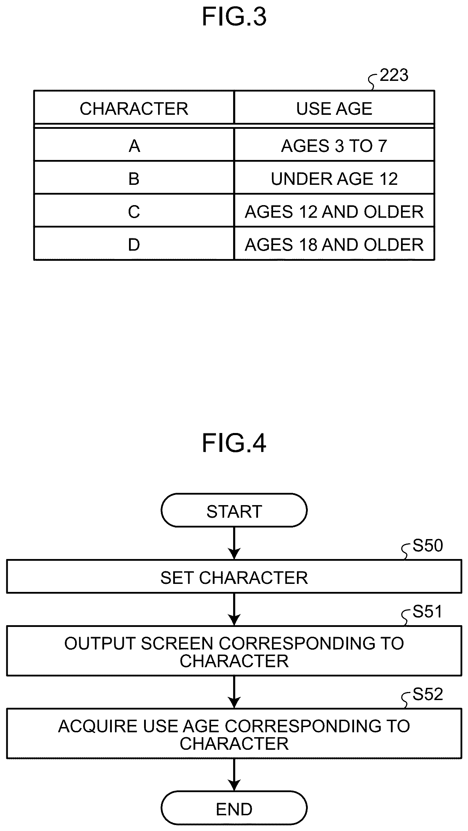

For example, it is assumed that a character "A" appears in an animation for kindergarten children. In this case, as illustrated in FIG. 3, the character "A" is associated with a use age of "ages 3 to 7".

Furthermore, for example, it is assumed that a movie in which a character "C" appears includes a violent scene and is not suitable for viewing by young children. In this case, as illustrated in FIG. 3, the character "C" is associated with a use age of "ages 12 and older".

The age in the character data 223 may be defined based on an age rating by a rating organization such as Pan European Game Information (PEGI), a Film Classification and Rating Organization, or a Computer Entertainment Rating Organization (CERO). Furthermore, the use age may be determined by a range such as "ages 3 to 5" or "ages 12 and older", or may be determined by one value such as "age 10" or "age 15".

The assist rule 224 is a rule defining an action to be assisted by the robot 100 in accordance with the physical condition of the user 10. The symptom data 225 stores information related to the symptom for each disease. The user support model 226 is reference data for deciding a personalized countermeasure for the target user in accordance with the emotional state of the target user.

The sensor module unit 210 includes a voice emotion recognition unit 211, an utterance comprehension unit 212, an expression recognition unit 213, and a face recognition unit 214. Information detected by the sensor unit 200 is input to the sensor module unit 210. The sensor module unit 210 analyzes the information detected by the sensor unit 200 and outputs an analysis result to the user state recognition unit 230.

The voice emotion recognition unit 211 of the sensor module unit 210 analyzes the voice of the user 10 detected by the microphone 201 to recognize the emotion of the user 10. For example, the voice emotion recognition unit 211 extracts a feature such as a frequency component of a voice and recognizes an emotion of the user 10 based on the extracted feature. The utterance comprehension unit 212 analyzes the voice of the user 10 detected by the microphone 201 and outputs textual information indicating utterance content of the user 10.

The expression recognition unit 213 recognizes the expression of the user 10 and the emotion of the user 10 from the image of the user 10 captured by the 2D camera 203. For example, the expression recognition unit 213 recognizes the expression and emotion of the user 10 based on the shapes, positional relationships, and the like of the eyes and the mouth.

The face recognition unit 214 recognizes the face of the user 10. The face recognition unit 214 recognizes the user 10 by checking the match between a face image stored in a person DB (not illustrated) and a face image of the user 10 captured by the 2D camera 203.

The user state recognition unit 230 recognizes the state of the user 10 based on the information analyzed by the sensor module unit 210. For example, processing mainly related to perception is performed using the analysis result of the sensor module unit 210. For example, perception information such as "Daddy is alone" and "Daddy is not smiling with probability of 90%" is generated. Processing of understanding the meaning of the generated perception information is performed. For example, semantic information such as "Daddy is alone and looks sad." is generated.

The emotion decision unit 232 decides an emotion value indicating the emotion of the user 10 based on the information analyzed by the sensor module unit 210 and the state of the user 10 recognized by the user state recognition unit 230. For example, the information analyzed by the sensor module unit 210 and the recognized state of the user 10 are input to a neural network trained in advance, and an emotion value indicating the emotion of the user 10 is acquired.

Here, the emotion value indicating the emotion of the user 10 is a value indicating whether the emotion of the user is positive/negative. For example, the emotion of the user is a bright emotion accompanied with pleasure or comfort, such as "delighted", "joyful", "pleasant", "secure", "excited", "relieved", and "fulfilled", the emotion value indicates a positive value which becomes larger as the emotion is brighter. When the user's emotion is a negative emotion, such as "angry", "sad", "unpleasant", "anxious", "sorrowful", "worried", and "empty", the value indicates a negative value, and the absolute value of the negative value is larger as the user feels more unpleasant. In a case where the user's emotion is not any of the above ("neutral"), the value indicates a value of 0.

In addition, the emotion decision unit 232 decides an emotion value indicating the emotion of the robot 100 based on the information analyzed by the sensor module unit 210 and the state of the user 10 recognized by the user state recognition unit 230.

The emotion value of the robot 100 includes the emotion value for each of a plurality of emotion classifications, and is, for example, a value (0 to 5) indicating the intensity of each of items of "delighted", "angry", "sad", and "joyful".

Specifically, the emotion decision unit 232 decides an emotion value indicating the emotion of the robot 100 in accordance with a rule for updating the emotion value of the robot 100 prescribed in association with the information analyzed by the sensor module unit 210 and the state of the user 10 recognized by the user state recognition unit 230.

For example, in a case where the user state recognition unit 230 recognizes that the user 10 looks sad, the emotion decision unit 232 increases the emotion value of "sad" of the robot 100. In a case where the user state recognition unit 230 recognizes that the user 10 is now smiling, the emotion value of "delighted" of the robot 100 is increased.

The emotion decision unit 232 may decide the emotion value indicating the emotion of the robot 100 in further consideration of the state of the robot 100. For example, in a case where the remaining battery level of the robot 100 is low, a case where the surrounding environment of the robot 100 is completely dark, or the like, the emotion value of "sad" of the robot 100 may be increased. Furthermore, in the case of the user 10 who desires to continue the dialog even though the remaining battery level is low, the emotion value of "angry" may be increased.

The action recognition unit 234 recognizes an action of the user 10 based on the information analyzed by the sensor module unit 210 and the state of the user 10 recognized by the user state recognition unit 230. For example, the information analyzed by the sensor module unit 210 and the recognized state of the user 10 are input to a neural network trained in advance to acquire the probability of each of a plurality of predetermined action classifications (for example, "smile", "get angry", "ask a question", and "expressing sorrow"), and the action classification having the highest probability is to be recognized as the action of the user 10.

As described above, in the first embodiment, the robot 100 acquires the utterance content of the user 10 after specifying the user 10. In the acquisition and use of the utterance content, the action control system of the robot 100 according to the first embodiment considers protection of personal information and privacy of the user 10 in addition to acquisition of necessary consent according to laws and regulations from the user 10.

Based on the current emotion value of the user 10 decided by the emotion decision unit 232, the history data 222 of the past emotion values decided by the emotion decision unit 232 before the current emotion value of the user 10 is decided, and the emotion value of the robot 100, the action decision unit 236 decides an action corresponding to the action of the user 10 recognized by the action recognition unit 234. While the first embodiment will describe a case where the action decision unit 236 uses one most recent emotion value included in the history data 222 as the past emotion value of the user 10, the disclosed technology is not limited to this aspect. For example, the action decision unit 236 may use a plurality of most recent emotion values as the past emotion values of the user 10, or may use emotion values that are earlier by a unit period such as a day before. Furthermore, the action decision unit 236 may decide an action corresponding to the action of the user 10 in further consideration of the history of the past emotion values of the robot 100 in addition to the current emotion value of the robot 100. The action decided by the action decision unit 236 includes a gesture performed by the robot 100 or utterance content of the robot 100.

The action decision unit 236 according to the first embodiment decides the action of the robot 100 as the action corresponding to the action of the user 10 based on a combination of the past emotion value and the current emotion value of the user 10, the emotion value of the robot 100, the action of the user 10, and the reaction rule 221. For example, in a case where the past emotion value of the user 10 is a positive value and the current emotion value is a negative value, the action decision unit 236 decides an action for changing the emotion value of the user 10 to a positive value as the action corresponding to the action of the user 10.

The action decision unit 236 may decide an action corresponding to the action of the user 10 based on the emotion of the robot 100. For example, when the emotion value of "angry" or "sad" of the robot 100 has increased in a case where the robot is abused by the user 10, in a case where the user 10 takes an arrogant attitude (that is, in a case where the user's reaction is unfavorable), in a case where the voice of the user 10 cannot be detected due to surrounding noise, in a case where the remaining battery level of the robot 100 is low, or the like, the action decision unit 236 may decide an action corresponding to the increase in the emotion value of "angry" or "sad" as the action corresponding to the action of the user 10. In addition, in a case where the emotion value of "delighted" or "joyful" of the robot 100 has increased in a case where the user's reaction is favorable, a case where the remaining battery level of the robot 100 is high, or the like, the action decision unit 236 may decide an action corresponding to the increase in the emotion value of "delighted" or "joyful" as an action corresponding to the action of the user 10. The action decision unit 236 may decide an action different from the action toward the user 10 that has increased the emotion values of "angry" and "sad" of the robot 100, as an action toward the user 10 that has increased the emotion values of "delighted" and "joyful" of the robot 100. In this manner, the action decision unit 236 may decide various actions depending on the emotion itself of the robot 100 itself or how the user 10 has changed the emotion of the robot 100 by the action of the user 10.

The reaction rule 221 defines the action of the robot 100 corresponding to the combination of the past emotion value and the current emotion value of the user 10, the emotion value of the robot 100, and the action of the user 10. For example, in a case where the past emotion value of the user 10 is a positive value, the current emotion value is a negative value, and the action of the user 10 is expressing sorrow, a combination of a gesture and utterance content to be used at the time of offering words with gesture to encourage the user 10 is prescribed as the action of the robot 100.

For example, in the reaction rule 221, the action of the robot 100 is determined for all combinations of the pattern of the emotion value of the robot 100 (1296 patterns being the fourth power of six values, namely, values "0" to "5" of "delighted", "angry", "sad", and "joyful"), the pattern of the combination of the past emotion value and the current emotion value of the user 10, and the action pattern of the user 10. That is, for each pattern of the emotion value of the robot 100, the action of the robot 100 corresponding to the action pattern of the user 10 is determined for each of the plurality of combinations such as the case where the combination of the past emotion value and the current emotion value of the user 10 include combinations of a negative value and a negative value, a negative value and a positive value, a positive value and a negative value, a positive value and a positive value, a negative value and a neutral value, and a neutral value and a neutral value. In a case where the user 10 has made an utterance that intends to have a conversation continued from a past topic such as "I want to talk about the topic I discussed earlier", for example, the action decision unit 236 may transition to the operation mode of deciding the action of the robot 100 using the history data 222.

The reaction rule 221 may prescribe at least one of a gesture and statement content as an action of the robot 100 for each of patterns (1296 patterns) of the emotion value of the robot 100 at the maximum. Alternatively, the reaction rule 221 may prescribe at least one of a gesture and statement content as an action of the robot 100 for each of the groups of the patterns of the emotion values of the robot 100.

The strength of a gesture is prescribed for each gesture included in the action of the robot 100 prescribed in the reaction rule 221. The strength of utterance content is prescribed for each utterance content included in the action of the robot 100 prescribed in the reaction rule 221.

The storage control unit 238 decides whether to store data including the action of the user 10 in the history data 222 based on the strength of the action prescribed for the action decided by the action decision unit 236 and the emotion value of the robot 100 decided by the emotion decision unit 232.

Specifically, in a case where the total value of the sum of the emotion values for each of the plurality of emotion classifications of the robot 100 and the strength that is the sum of the strength predetermined for the gesture included in the action decided by the action decision unit 236 and the strength predetermined for the utterance content included in the action decided by the action decision unit 236 is a threshold or more, it is decided to store data including the action of the user 10 in the history data 222.

Having decided to store the data including the action of the user 10 in the history data 222, the storage control unit 238 stores the action decided by the action decision unit 236, the information (for example, any peripheral information including data such as a voice, an image, and a smell of the place) analyzed by the sensor module unit 210 from the current time point to a certain period before, and the state of the user 10 (for example, the expression and emotion of the user 10) recognized by the user state recognition unit 230 in the history data 222.

The action control unit 250 controls the control target 252 based on the action decided by the action decision unit 236. For example, when the action decision unit 236 has determined an action including utterance, the action control unit 250 controls to output a voice from a speaker included in the control target 252. At this time, the action control unit 250 may decide the speech speed of the voice based on the emotion value of the robot 100. For example, the action control unit 250 decides the speech speed such that the larger the emotion value of the robot 100, the higher the utterance speed will be. In this manner, the action control unit 250 decides the execution mode of the action decided by the action decision unit 236 based on the emotion value decided by the emotion decision unit 232.

The action control unit 250 may recognize a change in emotion of the user 10 about the execution of the action decided by the action decision unit 236. For example, the change in emotion may be recognized based on the voice or expression of the user 10. In addition, a change in emotion of the user 10 may be recognized based on detection of an impact by the touch sensor included in the sensor unit 200. In a case where an impact is detected by the touch sensor included in the sensor unit 200, it is allowable to recognize that the emotion of the user 10 is worsened, or in a case where it is determined that the reaction of the user 10 is smiling or delighted from the detection result of the touch sensor included in the sensor unit 200, it is allowable to recognize that the emotion of the user 10 is improved. Information indicating the reaction of the user 10 is output to the communication processing unit 280.

Furthermore, after the action control unit 250 executes the action decided by the action decision unit 236 in the execution mode decided in accordance with the emotion of the robot 100, the emotion decision unit 232 further changes the emotion value of the robot 100 based on the user's reaction to the execution of the action. Specifically, in a case where the user's reaction to the action decided by the action decision unit 236 performed on the user in the execution mode decided by the action control unit 250 is not bad, the emotion decision unit 232 increases the emotion value of "delighted" of the robot 100; in a case where the user's reaction to the action decided by the action decision unit 236 performed on the user in the execution mode decided by the action control unit 250 is bad, the emotion decision unit 232 increases the emotion value of "sad" of the robot 100.

Furthermore, the action control unit 250 expresses the emotion of the robot 100 based on the decided emotion value of the robot 100. For example, when having increased the emotion value of "delighted" of the robot 100, the action control unit 250 controls the control target 252 to cause the robot 100 to make a gesture of delight. Furthermore, when having increased the emotion value of "sad" of the robot 100, the action control unit 250 controls the control target 252 such that the posture of the robot 100 takes a head lowering posture.

The communication processing unit 280 is responsible for communication with the server 300. As described above, the communication processing unit 280 transmits the user reaction information to the server 300. Furthermore, the communication processing unit 280 receives the updated reaction rule from the server 300. When having received the updated reaction rule from the server 300, the communication processing unit 280 updates the reaction rule 221.

The event detection unit 290 implements the above-described output function. Details of the event detection unit 290 will be described below.

The server 300 performs communication between the robots (the robot 100, the robot 101, and the robot 102) and the server 300, receives the user reaction information transmitted from the robot 100, and updates the reaction rule based on the reaction rule including the action for which a positive reaction has been obtained.

### (Action decision based on character)

The above has described a case where the action decision unit 236 decides the action of the robot 100 based on the state recognized by the user state recognition unit 230. On the other hand, the action decision unit 236 may decide the action of the robot 100 based on not only the state of the user but also a character that has been set. At this time, the action decision unit 236 may acquire the age (use age) associated with the character from the character data 223, and decide the action of the robot 100 based on the acquired use age.

That is, the action decision unit 236 decides the action of the robot 100 based on the state recognized by the user state recognition unit 230 and on the character that has been set or the age associated with the character. This configuration enables the robot 100 to execute an appropriate action according to the age of the user. In particular, it is possible to restrict actions by the robot 100 that are not suitable for younger users (for example, outputting violent content).

In the system 5, a character is set in advance. The character setting is input as a prompt (instruction). The input of the prompt may be performed via an input device provided in the robot 100 or may be performed via an external device such as a server communicably connected to the robot 100. Furthermore, in the prompt, a name of a character may be specified, or an ID determined for each character may be designated.

For example, the action decision unit 236 decides an action to output a screen indicating the appearance of the character or a color according to the character on the display device 2521 (an example of an output device) provided on the robot. The color corresponding to the character is a theme color or the like that is associated with the character. This makes it possible for the user to obtain a feeling of having a dialog with the character.

Furthermore, for example, the action decision unit 236 decides an action to output information to the display device 2521 or the speaker 2522 (an example of an output device) provided in the robot 100 by the mode according to the use age. For example, the action decision unit 236 changes the voice of the robot 100 emitted from the speaker 2522 to the tone of the character.

Furthermore, for example, the action decision unit 236 decides an action of outputting a voice or a message by a text using words corresponding to the use age. Here, it is assumed that usable words for each age are set in advance. The action decision unit 236 acquires the use age from the character data 223.

For example, it is assumed that words "What's wrong?" and "Is there anything I can do for you?" are stored in the storing unit 220 in advance as words to be output when the robot 100 selects the action of "offering words". Furthermore, it is assumed that the age of "ages under 12" is associated with "What's wrong?" and the age of "ages 12 and older" is associated with "Is there anything I can do for you?". For example, the action decision unit 236 decides to output the word "Is there anything I can do for you?" when the use age corresponds to "ages 18 and older". For example, the action decision unit 236 decides to output the word "What's wrong?" when the use age corresponds to "ages 3 to 7".

In this manner, by changing the tone of the voice and the words to be output in accordance with the use age, it is possible to improve the familiarity for the user of the younger age while restricting the action not suitable for the user of the younger age in particular.

Furthermore, the action decision unit 236 decides an action of outputting content corresponding to the character to an output device (such as the display device 2521) provided in the robot 100. For example, the action decision unit 236 decides an action to display, on the display device 2521, video content (such as movies and animations) in which a character appears.

Furthermore, the action decision unit 236 may decide an action of outputting educational content according to the use age. Here, the educational content is text, video, and voice related to learning subjects such as English, arithmetic, National language, science, and society. Furthermore, the educational content may be interactive content that allows the user to input their answer to a problem. For example, the action decision unit 236 decides an action to display the text of the calculation problem corresponding to the grade corresponding to the use age on the display device 2521. For example, the action decision unit 236 decides to display an addition problem when the use age is "ages under 8", and decides to display a multiplication problem when the use age is "ages 8 and older".

Furthermore, the action decision unit 236 may decide an action of outputting content corresponding to the use age rather than character to the output device provided in the robot 100. The content in this case may be a piece of content in which a character appears, or may be a piece of content that does not depend on a character, such as a generally known folk tale or fairy tale.

The content corresponding to a character, and the grade and educational content according to the use age may be stored in the storing unit 220 in advance, or may be acquired from an external device such as a server communicably connected to the robot 100.

FIG. 4 schematically illustrates an example of an operation flow related to setting of a character. Note that "S" in the operation flow represents a step to be executed.

In step S50, the robot 100 receives character setting. In step S51, the robot 100 outputs a screen (for example, a screen displaying an appearance of a character) corresponding to the character.

In step S52, the action decision unit 236 acquires the use age corresponding to the character that has been set, from the character data 223.

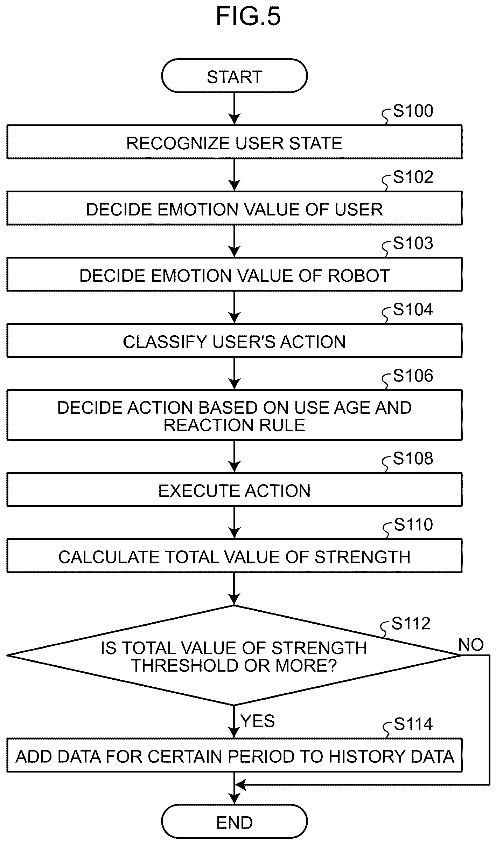

FIG. 5 schematically illustrates an example of an operation flow related to an operation of deciding an action in the robot 100. The operation flow illustrated in FIG. 5 is repeatedly executed. At this time, it is assumed that information analyzed by the sensor module unit 210 is input. Note that "S" in the operation flow represents a step to be executed.

First, in step S100, the user state recognition unit 230 recognizes the state of the user 10 based on the information analyzed by the sensor module unit 210.

In step S102, the emotion decision unit 232 decides an emotion value indicating the emotion of the user 10 based on the information analyzed by the sensor module unit 210 and the state of the user 10 recognized by the user state recognition unit 230.

In step S103, the emotion decision unit 232 decides an emotion value indicating the emotion of the robot 100 based on the information analyzed by the sensor module unit 210 and the state of the user 10 recognized by the user state recognition unit 230. The emotion decision unit 232 adds the decided emotion value of the user 10 to the history data 222.

In step S104, the action recognition unit 234 recognizes the action classification of the user 10 based on the information analyzed by the sensor module unit 210 and the state of the user 10 recognized by the user state recognition unit 230.

In step S106, the action decision unit 236 decides the action of the robot 100 based on the use age acquired in step S52 in FIG. 4, the combination of the current emotion value of the user 10 decided in step S102 in FIG. 5 and the past emotion value included in the history data 222, the emotion value of the robot 100, the action of the user 10 recognized by the action recognition unit 234, and the reaction rule 221.

In step S108, the action control unit 250 controls the control target 252 based on the action decided by the action decision unit 236.

In step S110, the storage control unit 238 calculates a total value of the strength based on the strength of the action predetermined for the action decided by the action decision unit 236 and the emotion value of the robot 100 decided by the emotion decision unit 232.

In step S112, the storage control unit 238 determines whether the total value of the strength is a threshold or more. In a case where the total value of the strength is less than the threshold, the data including the action of the user 10 is not stored in the history data 222, and the processing ends. In contrast, when the total value of the strength is the threshold or more, the processing proceeds to step S114.

In step S114, the action decided by the action decision unit 236, the information analyzed by the sensor module unit 210 from the current time point to a certain period before, and the state of the user 10 recognized by the user state recognition unit 230 are to be stored in the history data 222.

As described above, according to the robot 100, the emotion value indicating the emotion of the robot 100 is decided based on the user state, and whether to store data including the action of the user 10 in the history data 222 is decided based on the emotion value of the robot 100. This makes it possible to suppress the capacity of the history data 222 that stores data including the action of the user 10. In addition, for example, when the robot 100 determines, after 10 years, that the user state is the same as the user state at 10 years before, the robot 100 reads the history data 222 of 10 years before, and thus, can present, to the user 10, the state of the user 10 at 10 years before (for example, the expression, emotion, and the like of the user 10), and can further present any peripheral information such as data of a voice, an image, a smell, and the like at the situation.

Furthermore, according to the robot 100, it is possible to cause the robot 100 to execute an appropriate action in response to the action of the user 10. In known technologies, an action of the user is classified to determine an action of the robot including an expression or an appearance of the robot. In contrast, the robot 100 decides the current emotion value of the user 10, and executes an action on the user 10 based on the past emotion value and the current emotion value. Therefore, for example, in a case where the user 10 who looked happy the day before is depressed the next day, the robot 100 can perform utterance "You looked happy yesterday. What's wrong with you today?". Furthermore, the robot 100 can also perform an utterance with a gesture. Furthermore, for example, in a case where the user 10 who was depressed the day before looks happy the next day, the robot 100 can perform utterance, "You were depressed yesterday, but you look happy today, don't you?". Furthermore, for example, in a case where the user 10 who looked happy yesterday looks happier the next day than the day before, the robot 100 can perform utterance such as "You look happier today than yesterday. Did something good happen to you more than yesterday?" Furthermore, when the user 10 has an emotion value of 0 or more and is continuously in a state where the fluctuation range of the emotion value is within a certain range, for example, the robot 100 can make an utterance such as "Recently, you are stably in good mood." to the user 10.

Furthermore, for example, in a case where the robot 100 asks the user 10 a question "Have you finished the homework you mentioned yesterday?" and an answer of "I have finished it" is obtained from the user 10, the robot 100 can make a positive utterance such as "Good for you!" and make a positive gesture such as applause or thumbs-up. Furthermore, for example, when the user 10 utters "The presentation we discussed the day before yesterday went well", the robot 100 can make a positive utterance such as "Good job!" and also make the above positive gesture. In this manner, by the action taken by the robot 100 based on the history of the state of the user 10, it is expected that the user 10 feels a sense of closeness to the robot 100.

While the above embodiment is a case where the robot 100 recognizes the user 10 using the face image of the user 10, the disclosed technology is not limited to this aspect. For example, the robot 100 may recognize the user 10 using a voice uttered by the user 10, a mail address of the user 10, an ID of an SNS of the user 10, an ID card incorporating a wireless IC tag possessed by the user 10, or the like.

### (Detection of event)

The event detection unit 290 will be described in detail. Here, the event detection unit 290 is provided in the robot 100 and causes the robot 100 to output information corresponding to the detected event.

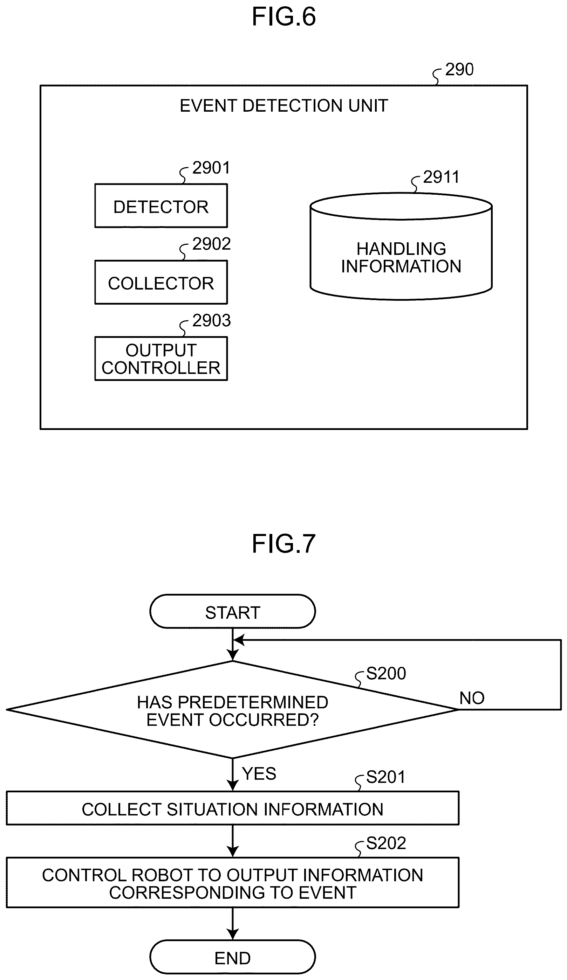

As illustrated in FIG. 6, the event detection unit 290 includes a detection unit 2901, a collection unit 2902, and an output controller 2903. The event detection unit 290 also stores handling information 2911.

Each component of the event detection unit 290 is implemented by the CPU operating based on a program. For example, the functions of these components can be implemented as the operation of the CPU by basic software (OS) and a program operating on the OS. The handling information 2911 is implemented by a storage medium such as memory.

The detection unit 2901 detects occurrence of a predetermined event. The output controller 2903 controls the robot 100 including the text generation model to output, to the user 10, information corresponding to the event detected by the detection unit 2901.

In addition, the collection unit 2902 collects situation information indicating a situation of the user 10. Subsequently, the output controller 2903 controls the robot 100 to output information corresponding to the situation information. For example, when an event occurs, the collection unit 2902 collects information indicating a situation of a place where the user 10 is located (voice or the like of the user 10). This makes it possible for the robot 100 to recognize the emotion of the user 10 from the voice or the like of the user 10 and grasp the situation of the place where the user 10 is located, enabling the robot 100 to give an appropriate instruction for the event that has occurred, by using gesture control or the like.

In addition, the detection unit 2901 detects occurrence of an event. Subsequently, the output controller 2903 controls the robot 100 to output information corresponding to the event that has occurred.

The detection unit 2901 can detect an event from an utterance of the user. For example, in a case where the user performs an utterance "I am here in Japan as an international student", the detection unit 2901 detects that the circumstance of the user has changed due to becoming the international student. For example, in a case where the user performs an utterance "I am thinking of changing my job to another company, which is an IT company", the detection unit 2901 detects that the user intends to change their job. For example, in a case where the user performs an utterance "My son, who likes sports, has become an elementary school student", the detection unit 2901 detects that the child of the user has grown up to a certain age.

In addition, the output controller 2903 controls the robot 100 to output information for adapting to the changed circumstance accumulated in the handling information 2911. With this configuration, the robot 100 can assist the adaptation of the user to the circumstance with respect to the event.

FIG. 7 schematically illustrates an example of an operation flow performed by the event detection unit 290. In step S200, the event detection unit 290 determines whether occurrence of a predetermined event has been detected (step S200). In a case where the occurrence of the predetermined event has not been detected (step S200; No), the event detection unit 290 waits until the occurrence of a predetermined event is detected.

In contrast, in a case where the occurrence of the predetermined event has been detected (step S200; Yes), the event detection unit 290 collects situation information indicating the situation of the user 10 (step S201). Subsequently, the event detection unit 290 controls the robot 100 including the text generation model to output information corresponding to the event that has occurred and corresponding to situation information to the user 10 (step S202), and ends the processing.

Note that the collection unit 2902 acquires not only the situation information but also the emotion value of the robot 100 decided by the emotion decision unit 232. The processing content of the emotion decision unit 232 is as described above. That is, in a case where an event is detected, the emotion decision unit 232 increases an emotion value regarding being secure, among a plurality of types of emotions prepared in advance as the emotions of the robot, each of the emotions being able to have an emotion value set for each of the emotions.

The emotion decision unit 232 increases the emotion value of "secure" (intensifies the emotion) in accordance with the occurrence of the event. Alternatively, the emotion decision unit 232 comprehensively lowers the emotion value in accordance with the occurrence of the event. The output controller 2903 controls the output of the robot 100 in accordance with the emotion value of the robot 100. For example, the output controller 2903 causes the robot 100 to perform utterance in a clear and cool tone of voice.

Hereinafter, an example of the operation of the robot 100 will be described together with an example of the event.

### (Event: becoming international student)

For example, in a case where the detection unit 2901 has detected that the user has become an international student as an event, the output controller 2903 controls the robot 100 to output information related to a method of adapting to the culture and environment of the place of residence that have changed by user's becoming the international student. In addition, the output controller 2903 causes, for example, an efficient method of learning a language of the country in which the user stays as an international student to be output as a method of handling problems and difficulties during the stay as an international student.

### (Event: job change)

For example, in a case where the detection unit 2901 has detected the job change of the user (including changing to another job, starting working with a new company, getting employed again) as an event, the output controller 2903 causes the robot 100 to output an occupation training related to the job obtained as a job change, a method of skill improvement, and a specific curriculum corresponding to the user's individual needs or goal.

Furthermore, the emotion decision unit 232 can change the emotion of the robot 100 to a positive emotion, make an utterance to cheer up the user, and improve the motivation of the user.

### (Event: child)

For example, in a case where the detection unit 2901 has detected that a child of the user was born and that the child of the user has grown to a certain age as events, the output controller 2903 controls the robot 100 to output information indicating extracurricular activities best for the child.

Furthermore, the collection unit 2902 collects, from the utterance content, information related to the interest and skills of the child. Subsequently, the output controller 2903 causes the robot 100 to suggest extracurricular activities corresponding to the interest and skills of the child. In addition, the output controller 2903 may receive a report of the progress situation of the extracurricular activities of the child and cause the robot 100 to output advice corresponding to the progress status.

### (Output example based on reaction rule)

The output controller 2903 can control the robot 100 based on the reaction rule 221. The reaction rule 221 stores message to be output in a case where an event related to a circumstance of the user is detected. Note that the message may be stored in the handling information 2911 instead of the reaction rule 221.

For example, the reaction rule 221 stores a message "First, please join a welcome party for international students" as a message for the user who has become an international student. In addition, the reaction rule 221 stores a message "There is an online programming course" as a message to a user who is considering changing job to an IT company, for example. In addition, the reaction rule 221 stores a message "How about going to a soccer class?" as a message for a user having a child who likes sports and has become an elementary school student, for example.

The robot 100 is an example of an electronic device including an action control system. The application target of the action control system is not limited to the robot 100, and the action control system can be applied to various electronic devices. Furthermore, the function of the server 300 may be implemented by one or more computers. At least a part of the functions of the server 300 may be implemented by a virtual machine. In addition, at least a part of the functions of the server 300 may be implemented in a cloud.

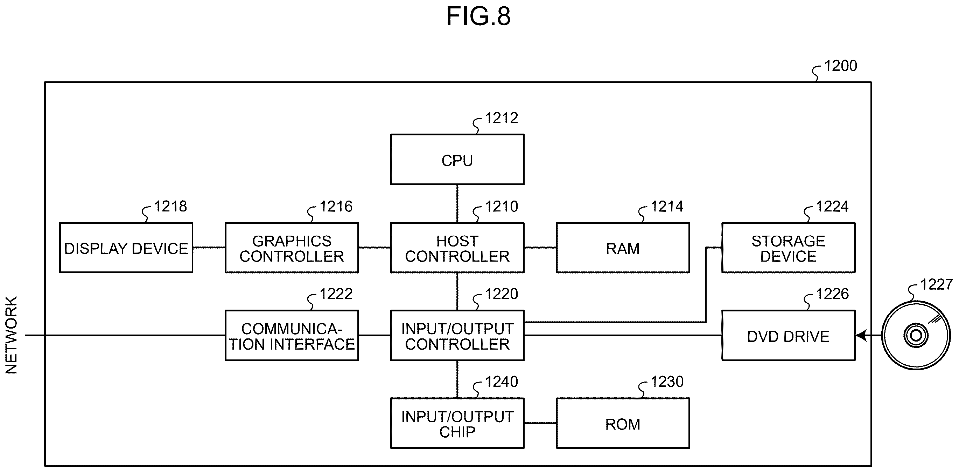

FIG. 8 is a diagram schematically illustrating an example of a hardware configuration of a computer 1200 functioning as the robot 100 and the server 300. A program installed in the computer 1200 can cause the computer 1200 to function as one or more "units" of the apparatus according to the first embodiment, or cause the computer 1200 to execute an operation associated with the apparatus according to the first embodiment or to implement the one or more "units", and/or cause the computer 1200 to execute a process according to the first embodiment or a stage of the process. Such a program may be executed by a CPU 1212 to cause the computer 1200 to perform certain operations associated with some or all of the blocks in the flowcharts and block diagrams described in the present specification.

The computer 1200 according to the first embodiment includes a CPU 1212, RAM 1214, and a graphics controller 1216, which are interconnected by a host controller 1210. The computer 1200 also includes input/output units such as a communication interface 1222, a storage device 1224, a DVD drive 1226, and an IC card drive, which are connected to the host controller 1210 via an input/output controller 1220. The DVD drive 1226 may be a DVD-ROM drive, a DVD-RAM drive, or the like. The storage device 1224 may be a hard disk drive, a solid state drive, or the like. The computer 1200 also includes a legacy input/output unit such as ROM 1230 and a keyboard, which are connected to the input/output controller 1220 via an input/output chip 1240.

The CPU 1212 operates in accordance with programs stored in the ROM 1230 and the RAM 1214, thereby controlling each unit. The graphics controller 1216 obtains image data generated by the CPU 1212 in a frame buffer or the like provided in the RAM 1214 or directly in the RAM 1214, and causes the image data to be displayed on a display device 1218.

The communication interface 1222 communicates with other electronic devices via a network. The storage device 1224 stores programs and data used by the CPU 1212 in the computer 1200. The DVD drive 1226 reads a program or data from a DVD-ROM 1227 or the like and provides the read program or data to the storage device 1224. The IC card drive reads programs and data from an IC card and/or writes programs and data to the IC card.

The ROM 1230 stores therein a boot program and the like executed by the computer 1200 at the time of activation, and/or a program dependent on hardware of the computer 1200. The input/output chip 1240 may connect various input/output units to the input/output controller 1220 via a USB port, a parallel port, a serial port, a keyboard port, a mouse port, or the like.

The program is provided by a computer-readable storage medium such as a DVD-ROM 1227 or an IC card. The program is read from a computer-readable storage medium, installed in the storage device 1224, the RAM 1214, or the ROM 1230, which is also an example of a computer-readable storage medium, and executed by the CPU 1212. The information processing described in these programs is read by the computer 1200 so as to provide a linkage between the programs and various types of hardware resources described above. The apparatus or method may be configured by implementing operation or processing of information in accordance with use of the computer 1200.

For example, when communication is performed between the computer 1200 and an external device, the CPU 1212 may execute a communication program loaded in the RAM 1214 and instruct the communication interface 1222 to perform communication processing based on processing described in the communication program. Under the control of the CPU 1212, the communication interface 1222 reads transmission data stored in a transmission buffer area provided in a recording medium such as the RAM 1214, the storage device 1224, the DVD-ROM 1227, or the IC card, transmits the read transmission data to the network, or writes reception data received from the network into a reception buffer area or the like provided on the recording medium.

In addition, the CPU 1212 may allow the RAM 1214 to read all or a necessary portion of a file or database stored in an external recording medium such as the storage device 1224, a DVD drive 1226 (DVD-ROM 1227), or the IC card, and may execute various types of processing on data on the RAM 1214. Next, the CPU 1212 may perform write-back of the processed data to the external recording medium.

Various types of information, such as various types of programs, data, tables, and databases, may be stored in a recording medium and subjected to information processing. The CPU 1212 may execute various types of processing on data read from the RAM 1214, including various types of operations, information processing, condition determination, conditional branching, unconditional branching, information search/replacement, and the like, which are described throughout the present disclosure and designated by a command sequence of a program, and writes back the results of processing to the RAM 1214. In addition, the CPU 1212 may search for information in a file, a database, or the like in the recording medium. For example, when a plurality of entries, each having an attribute value of a first attribute associated with an attribute value of a second attribute, is stored in the recording medium, the CPU 1212 may search for an entry in which the attribute value of the first attribute matches a designated condition from the plurality of entries, read the attribute value of the second attribute stored in the entry, and thereby acquire the attribute value of the second attribute associated with the first attribute satisfying the predetermined condition.

The above-described program or software modules may be stored in a computer-readable storage medium on the computer 1200 or in the vicinity of the computer 1200. Furthermore, a recording medium such as a hard disk or RAM provided in a server system connected to a dedicated communication network or the Internet can be used as a computer-readable storage medium, thereby providing the program to the computer 1200 via the network.

The blocks in the flowcharts and block diagrams in the first embodiment may represent stages of a process in which an operation is performed or "units" of an apparatus that are responsible for performing the operation. Specific stages and "units" may be implemented by dedicated circuits, programmable circuits provided together with computer-readable instructions stored on a computer-readable storage medium, and/or by a processor provided together with computer-readable instructions stored on a computer-readable storage medium. Dedicated circuits may include digital and/or analog hardware circuits, and may include integrated circuits (ICs) and/or discrete circuits. Programmable circuits may include reconfigurable hardware circuits including, for example, logical conjunction, logical disjunction, exclusive OR, NAND, NOR, and other logical operations, flip-flops, registers, and memory elements, such as field programmable gate arrays (FPGA) and programmable logic arrays (PLA).

A computer-readable storage medium may include any tangible device capable of storing instructions for execution by a suitable device, and as a result, the computer-readable storage medium including instructions stored in the device is to have a product including instructions that can be executed to create means for executing operations designated in the flowcharts or block diagrams. Examples of the computer-readable storage medium may include an electronic storage medium, a magnetic storage medium, an optical storage medium, an electromagnetic storage medium, and a semiconductor storage medium. More specific examples of the computer-readable storage medium may include a floppy (registered trademark) disk, a diskette, a hard disk, random access memory (RAM), read-only memory (ROM), erasable programmable read-only memory (EPROM or flash memory), electrically erasable programmable read-only memory (EEPROM), static random access memory (SRAM), compact disc read-only memory (CD-ROM), a digital versatile disk (DVD), a Blu-ray (registered trademark) disk, a memory stick, and an integrated circuit card.

The computer-readable instructions may include either source code or object code written in any combination of one or more programming languages, including assembler instructions, instruction-set-architecture (ISA) instructions, machine instructions, machine-dependent instructions, microcode, firmware instructions, state-setting data, or an object oriented programming language such as Smalltalk, JAVA (registered trademark), and C++, and conventional procedural programming languages, such as the "C" programming language or similar programming languages.

The computer-readable instructions may be provided for a processor or programmable circuits of a general purpose computer, special purpose computer, or other programmable data processing apparatus, either locally or over a local area network (LAN), a wide area network (WAN) such as the Internet so as to cause the processor or programmable circuits of the general purpose computer, special purpose computer, or other programmable data processing apparatus to execute the computer-readable instructions in order to generate means to execute the operations designated in the flowcharts or block diagrams. Examples of the processor include a computer processor, a processing unit, a microprocessor, a digital signal processor, a controller, and a microcontroller.

### (Other embodiments)

The robot 100 described above may be mounted on a stuffed toy, or may be applied to a control device connected by a wireless or wired link to a control target device (speaker or camera) mounted on the stuffed toy.

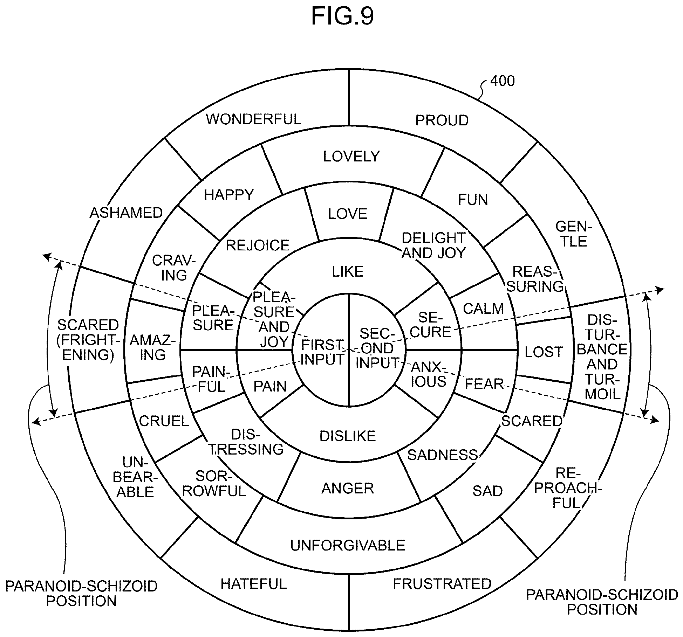

The emotion decision unit 232 may decide the user's emotion in accordance with a specific mapping. Specifically, the emotion decision unit 232 may decide the user's emotion based on an emotion map (refer to FIG. 9) being a specific mapping.

FIG. 9 is a diagram illustrating an emotion map 400 on which a plurality of emotions is mapped. On the emotion map 400, emotions are mapped concentrically radially from the center. The closer to the center of the concentric circle, the more the emotion disposed is toward a primitive state. Emotions indicating states and actions generated from the state of mind are disposed toward the outer side of the concentric circle. The emotion is a concept including an affect and a mental state. The left side of the concentric circle generally includes emotions generated from reactions occurring in the brain. The right side of the concentric circle generally includes emotions induced by situational judgment. The upper and lower directions of the concentric circle generally include emotions generated from reactions occurring in the brain and induced by situational judgment. Furthermore, the upper side of the concentric circle includes "pleasant" emotions while the lower side of the concentric circle includes "unpleasant" emotions. In this manner, the emotion map 400 is a map on which a plurality of emotions is mapped based on a structure of generating emotions, and emotions that are likely to occur at the same time are mapped close to each other.

(1) For example, in a case where the emotion engine, which is the emotion decision unit 232 of the robot 100, detects an emotion at about 100 msec, the decision of the reaction operation (for example, affirmative interjection) of the robot 100 may be performed at a timing at which the frequency is at least similar to the detection frequency (100 msec) of the emotion engine, or may be performed at a timing earlier than this. The detection frequency of the emotion engine may be construed as a sampling rate.

The emotion is detected in about 100 msec, and the reacting operation (for example, affirmative interjection) is immediately performed in conjunction with the detection, making it possible to achieve a dialogue reading the situation instead of giving a strange affirmative interjection. The robot 100 performs a reacting operation (affirmative interjection or the like) in accordance with the directionality and the degree (intensity) of the mandala-like chart of the emotion map 400. The detection frequency (sampling rate) of the emotion engine is not limited to 100 ms, and may be changed depending on the situation (such as when playing sports), the age of the user, or the like.

(2) With reference to the emotion map 400, the directionality and the degree of intensity of the emotion may be set in advance to set the motion of the affirmative interjection and the magnitude of the affirmative interjection. For example, in a case where the robot 100 feels a sense of stability, security, or the like, the robot 100 continues listening to the talk while nodding. When the robot 100 feels anxious, lost, or suspicious, the robot 100 may tilt its head or stop nodding.

These emotions are distributed in the 3 o'clock direction of the emotion map 400, and usually wander between security and anxiety. In the right half of the emotion map 400, situational awareness is stronger than internal sensation, and thus gives a calm impression.

(3) When the robot 100 feels good after being complimented, a filler "Wow" may come before the words; when the robot feels heavy after receiving harsh words, a filler "Ohh!" may come before the words. In addition, a physical reaction such as a gesture of the robot 100 crouching while saying "Ohh!" may be included. These emotions are distributed around 9 o'clock on the emotion map 400.

(4) In the left half of the emotion map 400, internal sensation (reaction) is stronger than situational awareness. Therefore, an impression of unintentional reaction can be given.

In a case where the robot 100 has a favorable feeling in situational awareness while having an internal feeling (reaction) of satisfaction, the robot 100 may nod deeply while looking at the other party, or may utter "Yeah". In this manner, the robot 100 may generate a balanced favorable feeling to the other party, that is, an action such as tolerance or generosity to the other party. Such emotions are distributed around 12 o'clock on the emotion map 400.

On the contrary, when the robot 100 has an internal feeling (reaction) of unpleasant feeling and also has negative feelings as situational awareness, the robot 100 may shake its head when feeling antipathy, and may stare at the other party with LED eyes turned into red when the robot 100 has a feeling close to hatred. Such emotions are distributed around 6 o'clock on the emotion map 400.

(5) Since the inner side of the emotion map 400 represents the inside of the mind and the outer side of the emotion map 400 represents an action, the emotion is more visible (appears in the action) toward the outer side of the emotion map 400.

(6) In a case where the robot 100 listens to a person's speech while feeling the sense of security distributed around 3 o'clock on the emotion map 400, the robot slightly nods with a sound "uh-huh". However, in the direction of love around 12 o'clock, the robot may perform strong and deep nodding.

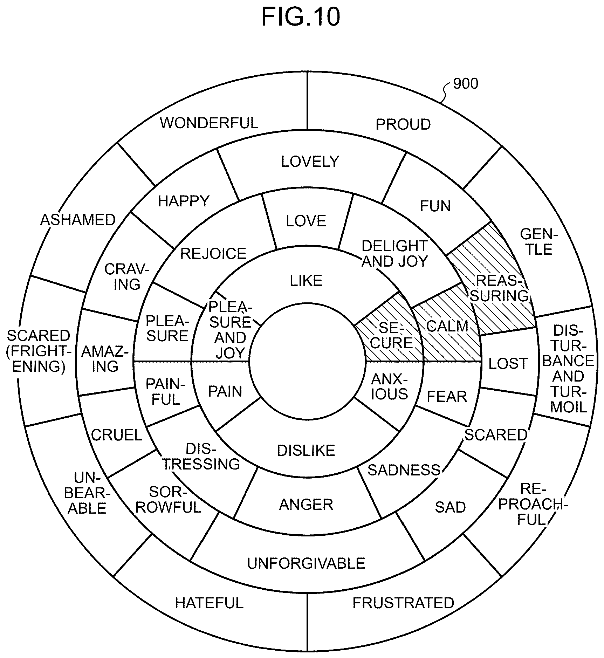

The emotion decision unit 232 inputs the information analyzed by the sensor module unit 210 and the recognized state of the user 10 to a neural network trained in advance, acquires an emotion value indicating each emotion illustrated on the emotion map 400, and decides the emotion of the user 10. This neural network is trained in advance based on a plurality of pieces of learning data including a combination of the information analyzed by the sensor module unit 210 and the recognized state of the user 10 and the emotion value indicating each emotion illustrated on the emotion map 400. Furthermore, as in an emotion map 900 illustrated in FIG. 10, this neural network is trained to allow emotions disposed close to each other to have close values. FIG. 10 is a diagram illustrating another example of the emotion map. FIG. 10 illustrates an example in which a plurality of emotions such as "secure", "calm", and "reassuring" have emotion values close to each other.

Furthermore, the emotion decision unit 232 may decide the emotion of the robot 100 in accordance with a specific mapping. Specifically, the emotion decision unit 232 inputs the information analyzed by the sensor module unit 210, the state of the user 10 recognized by the user state recognition unit 230, and the state of the robot 100 to a neural network trained in advance, acquires an emotion value indicating each emotion illustrated on the emotion map 400, and decides the emotion of the robot 100. This neural network is trained in advance based on a plurality of pieces of learning data including a combination of the information analyzed by the sensor module unit 210, the recognized state of the user 10, and the state of the robot 100, and the emotion value indicating each emotion illustrated on the emotion map 400. For example, the neural network is trained based on learning data indicating that the emotion value "3" of "happy" is obtained in a case where the robot 100 is recognized as being petted by the user 10 from the output of the touch sensor 207, and learning data indicating that the emotion value "3" of "anger" is obtained in a case where the robot 100 is recognized as being hit by the user 10 from the output of the acceleration sensor 205. Furthermore, as in an emotion map 900 illustrated in FIG. 10, this neural network is trained to allow emotions disposed close to each other to have close values.

Furthermore, the emotion decision unit 232 may decide the emotion of the robot 100 based on the action content of the robot 100 generated by the text generation model. Specifically, the emotion decision unit 232 inputs the action content of the robot 100 generated by the text generation model to the neural network trained in advance, acquires the emotion value indicating each emotion illustrated on the emotion map 400, integrates the acquired emotion value indicating each emotion and the emotion value indicating each emotion of the current robot 100, and updates the emotion of the robot 100. For example, the acquired emotion value indicating each emotion and the emotion value indicating each emotion of the current robot 100 are averaged and integrated. This neural network is trained in advance based on a plurality of pieces of learning data that is a combination of text representing the action content of the robot 100 generated by the text generation model and the emotion value indicating each emotion illustrated on the emotion map 400.

For example, there is a case where utterance content of the robot 100 "Good for you. Lucky you." is obtained as the action content of the robot 100 generated by the text generation model. In this case, the emotion of the robot 100 is updated such that, when the text representing the utterance content is input to the neural network, a high value is obtained as the emotion value of the emotion "happy" to increase the emotion value of the emotion "happy".

The action decision unit 236 adds a fixed sentence for asking a question about the action content of the robot corresponding to the user's action to the text representing the user's action, the user's emotion, and the robot's emotion, and inputs the obtained text to the text generation model having a dialogue function, thereby generating the action content of the robot.

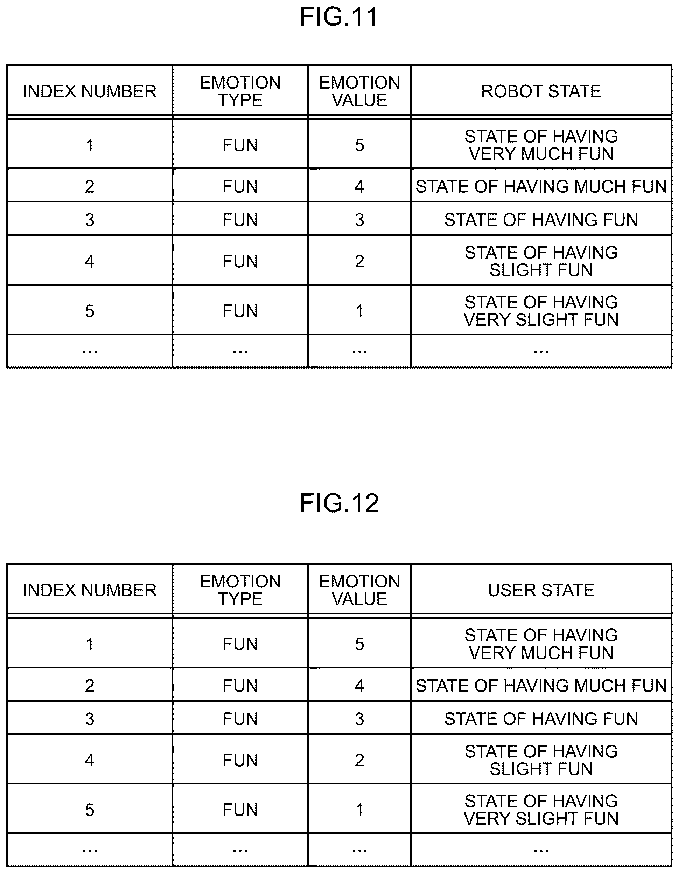

For example, the action decision unit 236 uses an emotion table as illustrated in FIG. 11 to acquire a text indicating the state of the robot 100 from the emotion of the robot 100 decided by the emotion decision unit 232. FIG. 11 is a diagram illustrating an example of the emotion table. Here, in the emotion table, an index number is assigned to each emotion value for each type of emotion, and a text indicating the state of the robot 100 is stored for each index number.

In a case where the emotion of the robot 100 decided by the emotion decision unit 232 corresponds to the index number "2", a text "state of having much fun" is obtained. Note that, in a case where the emotion of the robot 100 corresponds to a plurality of index numbers, a plurality of texts indicating the state of the robot 100 is obtained.

Furthermore, an emotion table as illustrated in FIG. 12 is prepared also for the emotion of the user 10. FIG. 12 is a diagram illustrating an example of the emotion table. Here, in a case where the user's action is to say "AAA.", the emotion of the robot 100 corresponds to the index number "2 ", and the emotion of the user 10 corresponds to the index number "3", the sentences "The robot is in a state of having much fun. The user is having fun. The user said "AAA" to the robot. What will be an answer as a robot?" is to be input to the text generation model and acquires the action content of the robot. THE USER IS HAVING FUN. The action decision unit 236 decides an action of the robot from the action content.

In this manner, since the robot 100 can change the action of the robot according to the index number corresponding to the emotion of the robot, the user has an impression of the robot 100 having a heart, and is prompted to take an action such as talking to the robot.

Furthermore, the action decision unit 236 may generate the action content of the robot by adding not only the text indicating the action of the user, the emotion of the user, and the emotion of the robot but also the text indicating the content of the history data 222, and then adding a fixed sentence for asking a question about the action content of the robot corresponding to the action of the user and inputting the obtained text to the dialogue function. With this configuration, the robot 100 can change the action of the robot according to the history data indicating the emotion of the user and action, and this gives the user an impression of the robot having individuality, and prompts the user to take an action such as talking to the robot. Furthermore, the history data may further include the emotion and action of the robot.

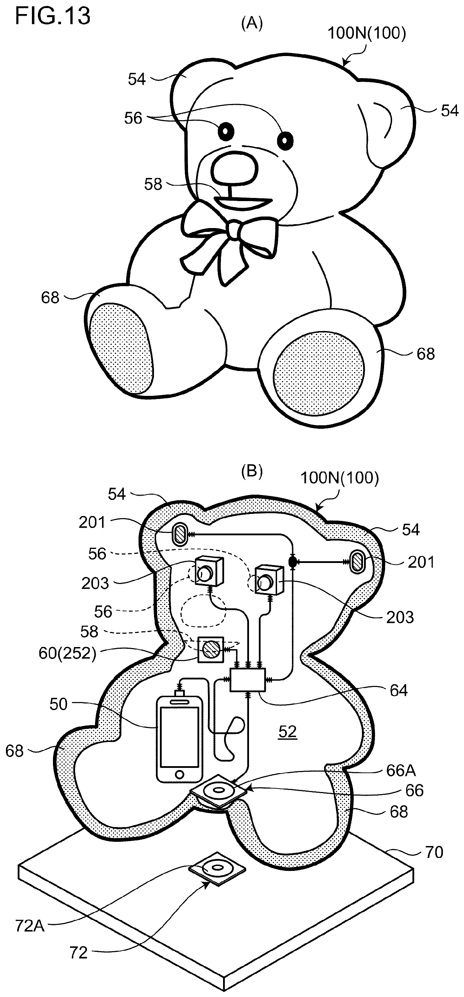

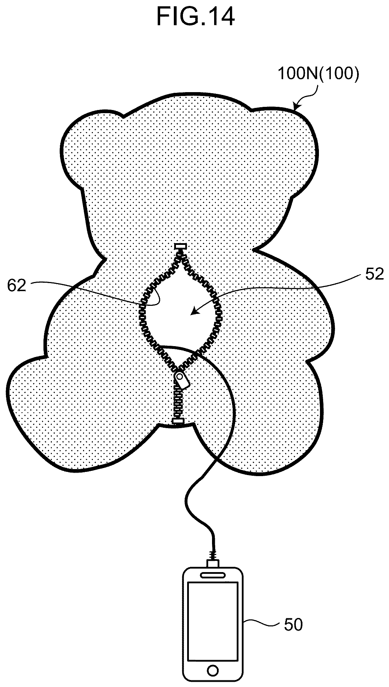

Specifically, the stuffed toy is configured as follows. For example, the robot 100 may be applied to a cohabitant (specifically, a stuffed toy 100N illustrated in FIGS. 13 and 14) who spends daily life with the user 10 and has a dialogue with the user 10 based on information related to daily life, and provides information matching the tastes and preferences of the user 10. In the present embodiment (another embodiment), an example in which the control section of the robot 100 is applied to the smartphone 50 will be described.

The stuffed toy 100N is equipped with a function as an input/output device of the robot 100. The smartphone 50 functioning as a control section of the robot 100 is detachable from the robot 100. Inside the stuffed toy 100N, an input/output device and the smartphone 50 which is accommodated in the stuffed toy 100N are connected to each other.

As illustrated in FIG. 13(A), the stuffed toy 100N has a shape of a bear an external appearance of which is covered with a soft fabric in the present embodiment (the another embodiment). As illustrated in FIG. 13(B), The input/output devices disposed in a space 52 formed inside the stuffed toy 100N include: a microphone 201 (refer to FIG. 2) of the sensor unit 200 disposed at a portion corresponding to the ear 54; a 2D camera 203 of the sensor unit 200 disposed at a portion corresponding to an eye 56 (refer to FIG. 2); and a speaker 60 constituting a part of the control target 252 (refer to FIG. 2) disposed at a portion corresponding to a mouth 58. Note that the microphone 201 and the speaker 60 are not necessarily separated from each other, and may be an integrated unit. In the case of the unit, it is preferable to dispose the unit at a position where the utterance can be heard naturally, such as the position of the nose of the stuffed toy 100N. Although the case where the stuffed toy 100N has an animal shape has been described as an example, the present invention is not limited thereto. The stuffed toy 100N may have a shape of a specific character.

The smartphone 50 has functions illustrated in FIG. 2, namely, a function as the sensor module unit 210, a function as the storing unit 220, a function as the user state recognition unit 230, a function as the emotion decision unit 232, a function as the action recognition unit 234, a function as the action decision unit 236, a function as the storage control unit 238, a function as the action control unit 250, and functions as the communication processing unit 280 and the event detection unit 290.

As illustrated in FIG. 14, a fastener 62 is attached to a part (for example, the back portion) of the stuffed toy 100N. By opening the fastener 62, the outside and the space 52 communicate with each other.

Here, the smartphone 50 is accommodated in the space 52 from the outside and is connected in USB connection to each input/output device via a USB hub 64 (refer to FIG. 13(B)), so as to have a function equivalent to that of the robot 100 illustrated in FIG. 1.

The USB hub 64 is connected to a non-contact power receiving plate 66. The power receiving plate 66 incorporates a power receiving coil 66A. The power receiving plate 66 is an example of a wireless power receiving unit that receives power supply wirelessly.

The power receiving plate 66 is disposed near a root 68 of both legs of the stuffed toy 100N, and is located closest to a mounting base 70 when the stuffed toy 100N is placed on the mounting base 70. The mounting base 70 is an example of an external wireless power transmitter.

The stuffed toy 100N placed on the mounting base 70 can be viewed as a statuette in a natural state.

In addition, this root is formed to be thinner than the surface layer thickness of the stuffed toy 100N in other parts, and thus is held in a state closer to the mounting base 70.

The mounting base 70 includes a charging pad 72. The charging pad 72 incorporates a power transmitting coil 72A. When the power transmitting coil 72A transmits a signal to search the power receiving coil 66A of the power receiving plate 66, and when the power receiving coil 66A is found, a current flows through the power transmitting coil 72A to generate a magnetic field, and the power receiving coil 66A reacts to the magnetic field to start electromagnetic induction. This allows current to flow through the power receiving coil 66A, and power is stored in a battery (not illustrated) of the smartphone 50 via the USB hub 64.

That is, the smartphone 50 is automatically charged by placing the stuffed toy 100N as a statuette on the mounting base 70, making it unnecessary to take out the smartphone 50 from the space 52 of the stuffed toy 100N for charging.

In the present embodiment (the another embodiment), the smartphone 50 is accommodated in the space 52 of the stuffed toy 100N and connected by wired connection (USB connection), but the connection is not limited thereto. For example, a control device having a wireless function (for example, "Bluetooth (registered trademark)") may be accommodated in the space 52 of the stuffed toy 100N, and the control device may be connected to the USB hub 64. In this case, the smartphone 50 and the control device wirelessly communicate with each other without inserting the smartphone 50 into the space 52, and the external smartphone 50 is connected to each input/output device via the control device, making it possible to have a function equivalent to that of the robot 100 illustrated in FIG. 1. Alternatively, the control device accommodated in the space 52 of the stuffed toy 100N and the external smartphone 50 may be connected to each other by wired connection.

While the present embodiment (the another embodiment) has exemplified a toy bear as the stuffed toy 100N, the stuffed toy 100N may be another toy, a doll, or may have a shape of a specific character. In addition, their clothes may be changeable. The material of the skin is not limited to a fabric, and may be other materials such as soft vinyl, but is preferably to be a soft material.

Furthermore, a monitor may be attached to the skin of the stuffed toy 100N to add the control target 252 that provides information to the user 10 through vision. For example, the eye 56 may be used as a monitor to express delight, anger, sadness, and joy by an image projected on the eye 56, or there may be provided, at a belly, a window through which the monitor of the built-in smartphone 50 is visible. Furthermore, the eyes 56 may be used as a projector to express delight, anger, sadness, and joy by an image projected on a wall surface.

According to the another embodiment, the existing smartphone 50 is placed in the stuffed toy 100N, and the 2D camera 203, the microphone 201, the speaker 60, and the like are extended from the smartphone 50 to appropriate positions via the USB connection.

Furthermore, for wireless charging, the smartphone 50 and the power receiving plate 66 are connected via USB, and the power receiving plate 66 is disposed so as to be as outermost as possible from the inside of the stuffed toy 100N.

In order to use the wireless charging of the smartphone 50, it is necessary to dispose the smartphone 50 as outermost as possible when viewed from the inside of the stuffed toy 100N, which would give a rough feeling when the stuffed toy 100N is touched from the outside.

To avoid this, the smartphone 50 is disposed at the center of the stuffed toy 100N as much as possible, while the wireless charging function (power receiving plate 66) is disposed as outermost as possible as viewed from the inside of the stuffed toy 100N. The 2D camera 203, the microphone 201, the speaker 60, and the smartphone 50 receive wireless power supply via the power receiving plate 66.

### [Second embodiment]

Hereinafter, a plurality of embodiments including a second embodiment will be described. Common portions in the above description of the first embodiment and the another embodiment, and in each embodiment, will be omitted, and features specific to each embodiment will be mainly described. Each embodiment can be implemented by replacing part of functions of the first embodiment and the another embodiments and adding functions. The event detection unit may be rephrased as an acquisition unit.

In the second embodiment, the robot 100 executes assist corresponding to the health state of the user 10 or a family member of the user 10. The robot 100 acquires information related to health and medical care of the user 10 or the family member of the user 10. The information related to health and medical care of the user 10 or the family member of the user 10 may be acquired by detection performed by the robot 100. In addition, the information related to health and medical care of the user 10 or the family member of the user 10 may be acquired by using information detected by another device. For example, the information related to the health and medical care of the user 10 or the family member of the user 10 may be acquired from an Internet of Things (Iot) device owned by the user 10 or the family member of the user 10 or a system of a medical institution. For example, the server 300 acquires information related to health and medical care of the user 10 or the family member of the user 10 from an Iot device owned by the user 10 or the family member of the user 10 or a system of a medical institution. The server 300 stores the acquired information related to health and medical care in association with identification information of each user 10. For example, the server 300 stores, as information related to health and medical care, information related to whether the user 10 or a family member of the user 10 is pregnant, or the time of birth, a disease name and symptoms in a case where the user has a disease, and a scheduled hospital stay period in a case where the user is hospitalized. The robot 100 inquires of the server 300 about the information related to health and medical care of the user 10 or the family member of the user 10, thereby receiving (acquiring), from the server 300, the information related to health and medical care of the user 10 or the family member of the user 10.

The event detection unit 290 acquires information related to health and medical care of at least one of the user 10 and a family member of the user 10. For example, the event detection unit 290 acquires, from the server 300, information related to health and medical care of the user 10 and a family member of the user 10. For example, the event detection unit 290 inquires of the server 300 the identification information of the user 10 and the family of the user 10. When the server 300 stores information related to health and medical care corresponding to the identification information, the server 300 transmits the information related to health and medical care corresponding to the identification information. The event detection unit 290 acquires information related to health and medical care of the user 10 and a family member of the user 10 by receiving the information from the server 300. For example, the event detection unit 290 acquires, from the server 300, information related to health and medical care of a family member, whether the user 10 or the family member of the user 10 is pregnant, the time of birth, a disease name and symptoms in a case where the user 10 or their family member has a disease, and a scheduled hospital stay period in a case where the user 10 or their family member is hospitalized.

In addition, in a case where the event detection unit 290 has acquired information related to health and medical care of at least one of the user 10 and a family member of the user 10, the action decision unit 236 provides assist corresponding to an emotion of the user 10 based on the acquired information related to health and medical care of at least one of the user 10 and the family member of the user 10. For example, the action decision unit 236 reads, from the assist rule 224, information related to content that should be provided as response and assist corresponding to information related to health and medical care of the user 10 or a family member of the user 10. The action decision unit 236 decides an action of the robot 100 based on the read information.

The assist rule 224 stores information related to content that should be provided as a response and assist to the person themselves and a family member separately for each problem for each health state and medical situation. For example, in a case where the person themselves and the family member are pregnant and after childbirth, the assist rule 224 stores content provided as an answer and content provided as assist separately for each problem occurring during pregnancy and after childbirth. For example, the assist rule 224 stores content provided as an answer to an emotional problem that occurs during pregnancy and a method of handling worries during pregnancy. In addition, the assist rule 224 stores content as an answer to an emotional problem occurring after childbirth, a method of handling stress after childbirth, and information related to child care. For example, the assist rule 224 stores content as an answer to an emotional problem occurring after childbirth, a method of handling stress, and information related to child care, for each period after the childbirth. In addition, the assist rule 224 stores information related to support of care and end-of-life care of a family member in a case where the family member is a last stage patient. For example, the assist rule 224 stores content that should be performed as care and a method of handling end-of-life care separately for each problem occurring in a terminal patient or a family. In addition, the assist rule 224 stores information related to emotional care and rehabilitation in a case where the patient requires hospitalization. For example, the assist rule 224 stores information related to content that should be performed as daily care and information related to rehabilitation separately for each problem occurring in a patient requiring hospitalization. In addition, the assist rule 224 stores information related to emotional care, vital signs, and medication in a case of a chronic disease patient. For example, the assist rule 224 stores content that should be performed as daily care, vital signs needing attention, and cautions related to medicine to be taken separately for each problem occurring in a chronic disease patient.

In a case where the event detection unit 290 has acquired information related to health and medical care of at least one of the user 10 and a family member of the user 10, the action decision unit 236 provides assist corresponding to an emotion of the user 10 based on the information related to health and medical care of at least one of the user 10 and the family member of the user 10. The action decision unit 236 according to the present embodiment specifies a problem from utterance content of the user 10. The action decision unit 236 analyzes textual information indicating the utterance content of the user 10 and specifies a problem included in the utterance content. The action decision unit 236 reads, from the assist rule 224, information related to content that should be provided as a response and assist corresponding to the specified problem in the health and medical situations acquired regarding the user 10 or the family of the user 10. The action decision unit 236 decides an action of the robot 100 based on the read information.

In a case where the robot 100 has acquired information related to health and medical care of at least one of the user 10 and a family member of the user 10, the robot 100 can provide assist corresponding to an emotion of the user 10 that has been recognized, based on the information related to health and medical care of at least one of the user 10 and the family member of the user 10. For example, the robot 100 can help navigate pregnant and post-partum parents through challenges that arise during pregnancy and after the childbirth. For example, the robot 100 can suggest a method of handling worries during pregnancy and stress after the childbirth, and improve their confidence as a parent. Furthermore, the robot 100 can provide content provided as an answer to an emotional problem, a method of handling stress, and information related to childcare for each period from childbirth, making it possible to support adaptation to a new family life. In addition, the robot 100 can provide mental care to last stage patients and their family members through dialogue. In addition, the robot 100 can provide a method of appropriate daily care and support of end-of-life care, making it possible to help the family to have a peaceful ending. In addition, the robot 100 can provide mental care to a patient requiring hospitalization through dialogue. In addition, the robot 100 can support a patient requiring hospitalization regarding rehabilitation and preparation for life after de-hospitalization, making it possible to set up an environment in which the patient can concentrate on medical treatment at ease.

In step S105, the event detection unit 290 acquires, from the server 300, information related to health and medical care of at least one of the user 10 and a family member of the user 10. For example, the event detection unit 290 inquires of the server 300 the identification information of the user 10 and the family of the user 10. When the server 300 stores information related to health and medical care corresponding to the identification information, the server 300 transmits the information related to health and medical care corresponding to the identification information. The event detection unit 290 acquires information related to health and medical care of at least one of the user 10 and a family member of the user 10 by receiving the information from the server 300.

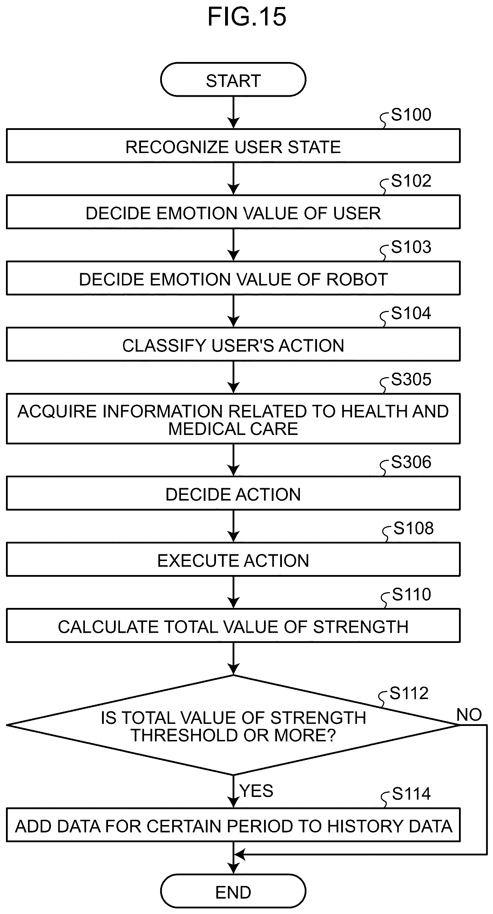

FIG. 15 is a diagram schematically illustrating an example of an operation flow performed by the robot 100 according to the second embodiment. Description of common portions with FIG. 5 will be omitted. In step S306, the action decision unit 236 decides an action of the robot 100. For example, the action decision unit 236 decides the action of the robot 100 based on the combination of the current emotion value of the user 10 decided in step S102 and the past emotion value of the user 10, the emotion value of the robot 100, the action of the user 10 recognized by the action recognition unit 234, and the reaction rule 221. In addition, when having acquired, in step S305, information related to health and medical care of at least one of the user 10 and a family member of the user 10, the action decision unit 236 provides assist corresponding to an emotion of the user 10 based on the acquired information related to health and medical care of at least one of the user 10 and the family member of the user 10. For example, the action decision unit 236 reads, from the assist rule 224, information related to content that should be provided as response and assist corresponding to information related to health and medical care of the user 10 or a family member of the user 10. The action decision unit 236 decides an action of the robot 100 based on the read information.

As described above, the robot 100 includes an estimation unit (the sensor unit 200, the sensor module unit 210, the user state recognition unit 230, and the emotion decision unit 232), the event detection unit 290, and an assisting unit (the action decision unit 236 and the action control unit 250). The estimation unit estimates an emotion of the user 10. The event detection unit 290 acquires information related to health and medical care of at least one of the user 10 and a family member of the user 10. Based on information related to health and medical care of at least one of the user 10 and a family member of the user 10 acquired by the event detection unit 290, the assisting unit provides assist corresponding to the emotion of the user 10 estimated by the estimation unit. This makes it possible for the robot 100 to assist the user 10 in accordance with health states and the medical situations of the user 10 and the family member of the user 10, enabling the support of the user 10.

In addition, in a case where the user 10 or a family member of the user 10 is pregnant or after childbirth, the assisting unit provides information for navigating the emotional problem of the user 10 estimated by the estimation unit. With this configuration, in a case where the user 10 or a family member of the user 10 is pregnant or after childbirth, the robot 100 can assist the user 10, enabling the support of the user 10.

In addition, in a case where the family member of the user 10 is a last stage patient, the assisting unit provides information related to support of daily care and end-of-life care of the family member of the user 10 in accordance with the emotion of the user 10 estimated by the estimation unit. With this configuration, in a case where a family member of the user 10 is a last stage patient, the robot 100 can assist the user 10, enabling the support of the user 10.

In addition, the event detection unit 290 acquires information related to health and medical care of the user 10. In a case where the user 10 is a patient requiring hospitalization, the assisting unit provides information related to at least one of emotional care and rehabilitation. With this configuration, in a case where the user 10 is a patient requiring hospitalization, the robot 100 can assist the user 10, enabling the support of the user 10.

In addition, the event detection unit 290 acquires information related to health and medical care of the user 10. In a case where the user 10 is a chronic disease patient, the assisting unit provides information related to at least one of emotional care, vital signs, and medication. With this configuration, in a case where the user 10 is a chronic disease patient, the robot 100 can assist the user 10, enabling the support of the user 10.

### [Third embodiment]

In the third embodiment, the emotion decision unit 232 functions as a physical condition estimation unit. The emotion decision unit 232 estimates the physical condition of the user 10 based on the recognized state of the user 10. For example, the emotion decision unit 232 estimates the physical condition of the user 10 based on the information analyzed by the sensor module unit 210 and the state of the user 10 recognized by the user state recognition unit 230. For example, the user state recognition unit 230 detects presence/absence of a cough and the presence/absence of voice abnormality in the user 10 from the voice collected by the microphone 201 provided in the sensor unit 200. In addition, the user state recognition unit 230 detects the complexion and the pulse rate of the user 10 from the video captured by the 2D camera 203 provided in the sensor unit 200. In addition, the user state recognition unit 230 detects the body temperature of the user 10 by thermography provided in the sensor unit 200. The emotion decision unit 232 may detect the state of the user 10 from the voice of the user 10 collected by the microphone 201. For example, in a case where the user 10 has performed audio inputs "I have a fever", "I have a sore throat", or "I have a stomachache", the emotion decision unit 232 may detect the state of the user 10 from the recognition result of the voice of the user 10 collected by the microphone 201.

Based on the symptom data 225, the emotion decision unit 232 estimates the physical condition of the user 10 from the detected states of the user 10 such as the presence/absence of a cough, the presence/absence of voice abnormality, the complexion, the pulse rate, and the body temperature. For example, in a case where the symptom data 225 includes a disease corresponding to the state of the user 10, the emotion decision unit 232 estimates that the user 10 is in the state of having the corresponding disease. Note that, in a case where the emotion decision unit 232 has detected the state of the user 10 from the voice of the user 10, the emotion decision unit 232 estimates the physical condition of the user 10 also using the state of the user 10 detected from the voice.

In addition, based on the physical condition of the user 10 estimated by the emotion decision unit 232, the action decision unit 236 decides an action in accordance with the physical condition of the user 10. For example, in a case where the emotion decision unit 232 has estimated that the user 10 is in a state of having a disease, the action decision unit 236 decides the action of the robot 100 in accordance with the disease of the user 10. For example, the action decision unit 236 reads, from the assist rule 224, information related to content that should be provided as response and assist corresponding to the disease of the user 10. The action decision unit 236 decides an action of the robot 100 based on the read information.

The assist rule 224 stores information related to content that should be provided as a response and assist to the user 10 separately for each problem for each physical condition. For example, the assist rule 224 stores content provided as assist, such as necessary emergency treatment and arrangement of an ambulance in the case of an urgent disease. The assist rule 224 also stores information related to an appropriate clinical department of a hospital for each disease. The assist rule 224 also stores information related to a medicine that is likely to be effective for each disease.

In a case where the action decision unit 236 estimates that the user 10 is in a disease state, the action decision unit provides assist corresponding to the disease of the user 10. The action decision unit 236 according to the present embodiment specifies a problem from utterance content of the user 10. The action decision unit 236 analyzes textual information indicating the utterance content of the user 10 and specifies a problem included in the utterance content. The action decision unit 236 reads, from the assist rule 224, information related to content that should be provided as a response and assist corresponding to the disease of the user 10 and the specified problem. The action decision unit 236 decides an action of the robot 100 based on the read information.

The action decision unit 236 may decide an action so as to ask a question related to disease and injury in order to sort out the situations of disease and injury. For example, in a case where there is a plurality of diseases estimated by the emotion decision unit 232, the action decision unit 236 may decide an action so as to ask a question discriminating each disease. The emotion decision unit 232 may estimate the physical condition of the user 10 using an answer of the user 10 to the question.

In addition, the robot 100 performs assist based on the physical condition of the user 10. For example, when having estimated that the user 10 is in a disease state, the robot 100 provides assist corresponding to the emotion of the user 10 based on the physical condition of the user 10. For example, in a case where the user 10 has an urgent disease, the robot 100 performs content provided as assist, such as necessary emergency treatment and arrangement of an ambulance. The arrangement of the ambulance may be performed online via the communication network 20, or may be performed via a mobile communication network when the robot 100 is capable of using the mobile communication. For example, the robot 100 transmits a request for arranging an ambulance by e-mail or the like. In addition, for example, when the physical condition of the user 10 is not good, the robot 100 can notify the symptoms, thereby organizing situations of possible disease and injury, and giving advice on an appropriate clinical department of the hospital. In addition, for example, the robot 100 can introduce a medicine that is likely to be most effective for the symptoms. The robot 100 can also grasp how distressing the user 10 feels from the expression or tone of voice of the user 10, and can change words of concern or gentle words.

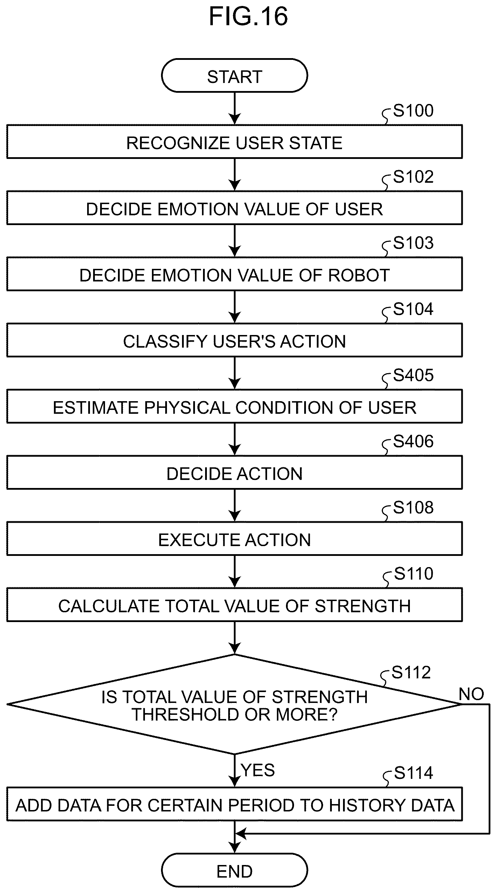

FIG. 16 is a diagram schematically illustrating an example of an operation flow performed by the robot 100 according to the third embodiment. Description of common portions with FIG. 5 will be omitted. In step S405, the emotion decision unit 232 estimates the physical condition of the user 10 based on the recognized state of the user 10. For example, the emotion decision unit 232 estimates the physical condition of the user 10 based on the information analyzed by the sensor module unit 210 and the state of the user 10 recognized by the user state recognition unit 230.

In step S406, the action decision unit 236 decides an action of the robot 100. For example, the action decision unit 236 decides the action of the robot 100 based on the combination of the current emotion value of the user 10 decided in step S102 and the past emotion value of the user 10, the emotion value of the robot 100, the action of the user 10 recognized by the action recognition unit 234, and the reaction rule 221. In addition, based on the physical condition of the user 10 estimated in step S405, the action decision unit 236 decides an action in accordance with the physical condition of the user 10. For example, when having estimated that the user 10 is in a state of having a disease, the action decision unit 236 decides the action of the robot 100 in accordance with the disease of the user 10. For example, the action decision unit 236 reads, from the assist rule 224, information related to content that should be provided as response and assist corresponding to the disease of the user 10. The action decision unit 236 decides an action of the robot 100 based on the read information.

In step S108, based on the action decided by the action decision unit 236, the action control unit 250 controls the control target 252 to execute the action. At this time, when the emotion of the user 10 estimated by the emotion decision unit 232 is a positive state, the action control unit 250 controls the control target 252 to express an emotion corresponding to the estimated emotion of the user 10 in alignment with the state of the user 10 recognized by the user state recognition unit 230. For example, when the emotion of the user 10 is a positive state, the action control unit 250 controls the control target 252 to express an emotion corresponding to the estimated emotion of the user 10 in the same state as the state of the user 10. In addition, when the emotion of the user 10 is in a positive state, the action control unit 250 decides the speech speed of the voice based on the emotion value of the robot 100.

As described above, the robot 100 includes the recognition unit (the sensor unit 200, the sensor module unit 210, and the user state recognition unit 230), the emotion decision unit 232, and the assisting unit (the action decision unit 236 and the action control unit 250). The estimation unit recognizes the state of the user 10. Based on the state of the user 10 recognized by the recognition unit, the emotion decision unit 232 estimates the physical condition of the user 10. Based on the physical condition of the user 10 estimated by the emotion decision unit 232, the assisting unit performs assist. This makes it possible for the robot 100 to assist the user 10 in accordance with the physical condition of the user 10, enabling the support of the user 10.

In addition, the robot 100 further includes an emotion estimation unit (emotion decision unit 232). The emotion estimation unit estimates an emotion of the user 10 based on the state of the user 10 recognized by the recognition unit. Based on the physical condition of the user 10 estimated by the physical condition estimation unit, the assisting unit performs assist corresponding to the emotion of the user 10 estimated by the emotion estimation unit. This makes it possible for the robot 100 to provide assist corresponding to the emotion of the user 10 in accordance with the physical condition of the user 10, enabling the support of the user 10.

In addition, in a case where the user 10 has an urgent disease, the assisting unit performs at least one of provision of information related to emergency treatment and arrangement of an ambulance. With this configuration, the robot 100 can assist the user 10 in which an urgent disease has occurred, enabling the support of the user 10.

In addition, the assisting unit provides information related to a clinical department corresponding to the disease of the user 10. In addition, the assisting unit provides information related to a medicine corresponding to the disease of the user 10. This makes it possible for the robot 100 to assist the user 10 having a disease, enabling the support of the user 10.

### [Fourth embodiment]

In a fourth embodiment, the robot 100 detects occurrence of an event which is related to a circumstance of the user and which causes the user to have a negative emotion. In addition, the robot 100 outputs information corresponding to the detected event. For example, the event related to the circumstance of the user and causing the user to have a negative emotion includes mental illness (Posttraumatic Stress Disorder (PTSD), depression, adjustment disorder, etc.), affection of dependence, death of a close relative, or the like. The negative emotion is an emotion in a case where a user's emotion value described below becomes negative, and includes "angry", "sad", "unpleasant", "anxious", "sorrowful", "worried", "empty", etc.

The robot 100 has an emotion of compassion for the negative emotion caused by the occurrence of the event. Specifically, in a case where an event is detected, a value of the emotion regarding compassion is to be changed, among a plurality of types of emotions prepared in advance as the emotions of the robot, each of the emotions being able to have a value set for each of the emotions. Subsequently, the robot 100 performs output corresponding to the value of the emotion. For example, the emotion related to compassion is "unbearable" on the emotion map described below.

For example, the robot 100 outputs, by utterance, a text describing a method of handling an event. This makes it possible for the robot 100 to promote the emotion of the user to change in the positive direction.

The detection unit 2901 can detect an event from an utterance of the user. For example, in a case where the user performs an utterance "I have PTSD", the detection unit 2901 detects that the user has a mental illness (PTSD). For example, in a case where the user performs an utterance "My grandfather passed away the other day", the detection unit 2901 detects the death of a close relative of the user. For example, in a case where the user performs an utterance "I shake unless I drink alcohol", the detection unit 2901 detects that the user has an addiction (alcohol addiction).

The output controller 2903 controls the robot 100 to output the information related to handling of the event that has occurred, which is accumulated in the handling information 2911. With this configuration, the robot 100 can mentally assist the user for the event.

FIG. 7 schematically illustrates an example of an operation flow performed by the event detection unit 290. In step S200, the event detection unit 290 determines whether occurrence of a predetermined event has been detected (step S200). In a case where the occurrence of the predetermined event has not been detected (step S200; No), the event detection unit 290 waits until the occurrence of a predetermined event is detected.

In contrast, in a case where the occurrence of the predetermined event has been detected (step S200; Yes), the event detection unit 290 collects situation information indicating the situation of the user 10 (step S201). Subsequently, the event detection unit 290 controls the robot 100 including the text generation model to output information corresponding to the event that has occurred and corresponding to situation information to the user 10 (step S202), and ends the processing.

Note that the collection unit 2902 acquires not only the situation information but also the emotion value of the robot 100 decided by the emotion decision unit 232. The processing content of the emotion decision unit 232 is as described above. That is, in a case where an event is detected, the emotion decision unit 232 increases an emotion value regarding compassion, among a plurality of types of emotions prepared in advance as the emotions of the robot, each of the emotions being able to have an emotion value set for each of the emotions.

In a case where the user performs an action (utterance, etc.) with a negative emotion in response to the occurrence of the event, the emotion decision unit 232 increases the emotion value of the emotion (intensifies the emotion) related to compassion (for example, "sad", "unbearable", etc.) based on the action. The output controller 2903 controls the output of the robot 100 in accordance with the emotion value of the robot 100. For example, the output controller 2903 causes the robot 100 to lower the tone of voice and to perform utterance at a lower speed.

Hereinafter, an example of the operation of the robot 100 will be described together with an example of the event.

### (Event: mental illness)

For example, in a case where the detection unit 2901 has detected that the user has suffered from a mental illness as an event, the output controller 2903 controls the robot 100 to output information related to a method of handling the mental illness.

At this time, the output controller 2903 causes the robot 100 to output an utterance describing a personalized handling strategy, a grounding technique, a stress release method, and a relaxation technique for the user, thereby assisting the emotional recovery of the user.

### (Event: loss)

For example, in a case where the detection unit 2901 has detected a death of a close relative of the user as an event, the output controller 2903 controls the robot 100 to output information related to a method of handling the sense of loss caused by the death of the close relative.

At this time, the output controller 2903 increases the emotion value of the compassion to cause the robot 100 to output an utterance resonating with the loss and the sorrow, understanding the emotion, and giving a description of a handling method, and an utterance that encourages consultation with an expert at an appropriate timing, thereby assisting the emotional recovery of the user.

### (Event: addiction)

For example, in a case where the detection unit 2901 has detected that the user is suffering from addiction as an event, the output controller 2903 controls the robot 100 to output information related to a method of handling the addiction.

At this time, the output controller 2903 causes the robot 100 to output an utterance that supports mental health of the user in recovering from the addiction, and an utterance giving descriptions of a stress alleviation method, a cause of the addiction, challenges in a recovery process, and a success experience of another person who has recovered from an addiction, etc., thereby assisting the emotional recovery of the user.

### (Output example based on reaction rule)

The output controller 2903 can control the robot 100 based on the reaction rule 221. The reaction rule 221 stores a message to be output in a case where occurrence of an event which is related to a circumstance of the user and which causes a negative emotion to the user is detected, or in a case where the fact that the user actually has a negative emotion due to such an event is detected. Note that the message may be stored in the handling information 2911 instead of the reaction rule 221.

Examples of the messages stored in the reaction rule 221 as messages related to a handling method in a case where the occurrence of an event that generates negative emotions for the user is detected include messages such as "Please get enough sleep", "Please exercise moderately", "you will get better with time", and "Let's consult an expert".

### [Fifth embodiment]

In the fifth embodiment, the robot 100 recognizes an action of the user 10 having a brain dysfunction, and decides an action of the robot 100 corresponding to the recognized action of the user 10. The robot 100 controls the control target 252 based on the decided action of the robot 100. While the control target 252 will be described below, the control target 252 includes the display device 2521 and the like.

The brain dysfunction includes at least one of a developmental disorder and a memory disorder. Developmental disorders include pervasive developmental disorders, learning disorders, attention deficit hyperactivity disorders, etc. Pervasive developmental disorders include autism spectrum disorders and Asperger's syndrome. Memory disorder occurs due to Alzheimer's disease, dementia, etc.

In a case where the user 10 has been recognized, the user state recognition unit 230 reads information of the user 10. The information of the user 10 is to be stored in the storing unit 220 in association with the face image of the user 10. The information of the user 10 includes the name of the user 10, the age of the user 10, and the like. The information of the user 10 includes the type, disease name, etc. of the brain dysfunction of the user 10. For example, in a case where the user 10 has a memory disorder, the information of the user 10 includes information such as an event related to the user 10 and a related person of the user 10. For example, in a case where the user 10 having a brain dysfunction has been recognized, the user state recognition unit 230 reads information related to the brain dysfunction stored in the storing unit 220.

In addition, the user state recognition unit 230 mainly performs processing related to perception, for example, using the analysis result of the sensor module unit 210. For example, perception information such as "The patient is not smiling with probability of 90%" is generated. Processing of understanding the meaning of the generated perception information is performed. For example, semantic information such as "The patient is alone and looks sad." is generated. The patient is the user 10, being the user 10 having a brain dysfunction, for example.

For example, the reaction rule 221 prescribes actions of the robot 100 corresponding to the brain dysfunction. For example, in a case where the user 10 has a memory disorder such as Alzheimer's disease, the reaction rule 221 prescribes actions of the robot 100 that remind the user 10 of important events, people, and places. In addition, in a case where the user 10 has a memory disorder such as Alzheimer's disease, the reaction rule 221 may prescribe an action of the robot 100 for improving cognitive ability in daily life for the user 10.

For example, in a case where the user 10 has autism spectrum disorder, the reaction rule 221 prescribes, for the user 10, an action of the robot 100 to help the user 10 understand social situations and sort out their own emotions. In addition, in a case where the user 10 has autism spectrum disorder, the reaction rule 221 may prescribe actions of the robot 100 that improve Quality of Life (QOL) of the user 10 by emotion recognition of the user 10 or personalized action support.

For example, in a case where the user 10 is a child having a learning disorder, the reaction rule 221 prescribes actions of the robot 100 for performing learning assist for the user 10 corresponding to the needs of the user 10 or the skills of the user 10. In addition, in a case where the user 10 is a child having a learning disorder, the reaction rule 221 may prescribe actions of the robot 100 to suggest emotional support for the user 10 and an appropriate education method for the user 10. In addition, in a case where the user 10 is a child having a learning disorder, the reaction rule 221 may prescribe actions of the robot 100 to improve the feeling of self-affirmation in the user 10 and the delight of the user 10 with respect to learning.

For example, in a case where the user 10 has memory disorder, the action control unit 250 controls to display a photograph related to a place where the user 10 traveled. Subsequently, in a case where the reaction of the user 10 is a positive reaction, for example, in a case where the user 10 has an expression of smiling, the emotion value of "delighted" of the robot 100 is increased, and the action control unit 250 performs an utterance such as "It was fun in ABC." and controls to execute an action to remind the user 10 of the memory related to the travel. "ABC" is a place where the user 10 traveled. In a case where the reaction of the user 10 is a negative reaction, for example, in a case where there is no change in the expression of the user 10, for example, the emotion value of "sad" of the robot 100 is increased, and the action control unit 250 performs an utterance such as "It was fun in ABC you went with D." and controls to perform an action to remind the user 10 of the memory related to the travel.

In this manner, for example, in a case where the user 10 has a memory disorder, the action control unit 250 controls the control target 252 to perform an action to remind the user 10 of an important event, people, or place. In a case where the user 10 has memory disorder, the action control unit 250 may control to execute an action to improve the cognitive ability of the user 10 in daily life.

In addition, for example, in a case where the user 10 has autism spectrum disorder, the action control unit 250 controls to execute an action to help the user 10 to understand social situations and sort out their own emotions. In addition, in a case where the user 10 has autism spectrum disorder, the action control unit 250 may control to execute an action to improve QOL of the user 10 by emotion recognition of the user 10 or personalized action support for the user 10.

In addition, for example, in a case where the user 10 has a learning disorder, the action control unit 250 controls to execute an action of performing learning assist corresponding to the needs of the user 10 or the skills of the user 10. In addition, in a case where the user 10 has a learning disorder, the action control unit 250 may control to execute an action of suggesting emotional support for the user 10 and an appropriate education method for the user 10. In addition, in a case where the user 10 has a learning disorder, the action control unit 250 may control to execute an action to improve the feeling of self-affirmation in the user 10 and the delight for learning in the user 10.

First, in step S101, the user state recognition unit 230 recognizes the state of the user 10 based on the information analyzed by the sensor module unit 210. In a case where the user 10 has been recognized, the user state recognition unit 230 reads information of the user 10. For example, in a case where information regarding the recognized user 10 is stored in the storing unit 220 in association with information related to brain dysfunction, the user state recognition unit 230 reads the information related to brain dysfunction from the storing unit 220.

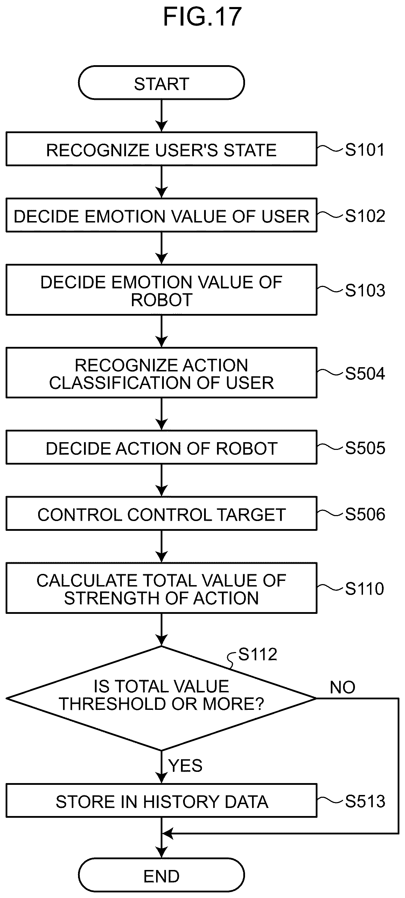

FIG. 17 is a diagram schematically illustrating an example of an operation flow performed by the robot 100 according to the fifth embodiment. Description of common portions with FIG. 5 will be omitted. In step S504, the action recognition unit 234 recognizes the action classification of the user 10 based on the information analyzed by the sensor module unit 210 and the state of the user 10 recognized by the user state recognition unit 230.

In step S505, the action decision unit 236 decides the action of the robot 100 based on the combination of the current emotion value of the user 10 decided in step S102 and the past emotion value included in the history data 222, the emotion value of the robot 100, the action of the user 10 recognized by the action recognition unit 234, and the reaction rule 221.

For example, in a case where the user 10 has memory disorder, the action decision unit 236 decides an action of the robot 100 to display a photograph related to a place where the user 10 has traveled. For example, the action decision unit 236 selects a photograph based on the emotion value indicating the emotion of the user 10, the emotion value of the robot 100, the action of the user, and the reaction rule 221. Note that the photograph data is stored in the storing unit 220 as information regarding the user 10. The photograph data may be acquired from the server 300.

Subsequently, in the next processing, for example, in a case where the reaction of the user 10 to the photograph is a positive reaction, the action of the robot 100 to perform an utterance "It was fun in ABC." or the like is decided.

As described above, the robot 100 includes the control unit that recognizes an action of the user 10 having a brain dysfunction, decides its own action corresponding to the recognized action of the user 10, and controls the control target 252 based on the own action decided. This makes it possible for the robot 100 to execute an appropriate action for the user 10 having a brain dysfunction. For example, the robot 100 can execute assist to the user 10 having a brain dysfunction.

Brain dysfunction includes developmental disorders, such as learning disorder, for example. For example, regarding the user 10 having a learning disorder, the robot 100 recognizes an action of the user 10, decides an own action corresponding to the recognized action of the user 10, and controls the control target 252 based on the own action decided. This makes it possible for the robot 100 to perform learning assist corresponding to the needs of the user 10 and the skills of the user 10. In addition, the robot 100 can suggest emotional support of the user 10 and an appropriate education method to the user 10 having learning disorder. In addition, regarding the user 10 having a learning disorder, the robot 100 can improve the feeling of self-affirmation in the user 10 and the delight for learning in the user 10.

For example, regarding the user 10 having autism spectrum disorder, the robot 100 recognizes an action of the user 10, decides an own action corresponding to the recognized action of the user 10, and controls the control target 252 based on the own action decided. This makes it possible for the robot 100 to help the user 10 understand social situations and sort out their emotions. In addition, the robot 100 can improve QOL of the user 10 by emotion recognition of the user 10 and personalized action support.

Brain dysfunction includes memory disorder. For example, regarding the user 10 having a memory disorder, the robot 100 recognizes an action of the user 10, decides an own action corresponding to the recognized action of the user 10, and controls the control target 252 based on the own action decided. This makes it possible for the robot 100 to remind important events, people, and places. In addition, the robot 100 improves the cognitive ability in daily life for the user 10 having a memory disorder.

The robot 100 decides an emotion value of the robot 100, and decides an action of the robot 100 in accordance with the decided emotion value of the robot 100. This makes it possible for the robot 100 to decide an action corresponding to its own emotion and execute a more appropriate action. Accordingly, the robot 100 can execute a more appropriate action for the user 10 having a brain dysfunction.

### [Sixth embodiment]

In a sixth embodiment, the robot 100 recognizes an action of an artist who is the user 10, and decides the action of the robot 100 corresponding to the recognized action of the artist. The robot 100 controls the control target 252 based on the decided action of the robot 100. While the control target 252 will be described below, the control target 252 includes the display device 2521 and the like. The robot 100 decides an action related to the performance of the artist.

The artist is a person engaged in creative activities. The artist includes a person who creates and generates works of art. For example, an artist includes an engraver, an art painter, a director, a writer, etc. The artist further includes a stage performer, a music performer, and the like.

The robot 100 decides an action related to the creativity of the artist. In addition, the robot 100 decides an action related to the expressivity of the artist. Hereinafter, the user 10 who is an artist will be described as an example, but the present invention is not limited thereto.

In a case where the user 10 has been recognized, the user state recognition unit 230 reads information of the user 10. The information of the user 10 is to be stored in the storing unit 220 in association with the face image of the user 10. The information regarding the user 10 includes the name of the user 10, the occupation of the user 10, the age of the user 10, etc. In a case where the user 10 is an artist, the information of the user 10 may include information related to past performance of the user 10, such as information related to a work created by the user 10 in the past or a video in which the user 10 appeared in the past, for example. For example, the user state recognition unit 230 may estimate the occupation of the user 10 from the past utterance content of the user 10 or the like for the recognized user 10.

In addition, the user state recognition unit 230 mainly performs processing related to perception, for example, using the analysis result of the sensor module unit 210. For example, perception information such as "The user is not smiling with probability of 90%" is generated. Processing of understanding the meaning of the generated perception information is performed. For example, semantic information such as "The user is alone and worried." is generated.

For example, the reaction rule 221 may prescribe an action of the robot 100 corresponding to the user 10 being an artist. For example, the reaction rule 221 may prescribe an action related to performance of the user 10.

For example, the action decision unit 236 decides an action related to the creativity of the user 10 being an artist. For example, the action decision unit 236 decides an action that brings out inspiration creativity in the user 10. In addition, the action decision unit 236 may decide an action of improving management of a project related to the user 10.

In addition, the action decision unit 236 may decide an action related to expressivity of the user 10 who is an artist. For example, the action decision unit 236 decides an action for improving self-expression of the user 10. In addition, the action decision unit 236 may decide an action for emotion management of the user 10. The action decision unit 236 may decide an action for mental training of the user 10. Specifically, the action decision unit 236 decides an action of suggesting a method of alleviating the tension of the user 10 or suggesting a technique of communicating with the audience.

For example, in a case where the user 10 utters like "I was so nervous." and the user 10 is smiling, the emotion value of "delighted" of the robot 100 is increased, and the action control unit 250 performs an utterance such as "You are performing well". In a case where the user 10 performs an utterance like "I was so nervous," and the user 10 expresses sorrow, the emotion value of "sad" of the robot 100 is increased, and the action control unit 250 performs an utterance like "You (Mr./Ms. A) can do it", and "Let's try BBB before actual performance next time".

In this manner, for example, the action control unit 250 controls to execute an action related to emotion management of the user 10 or controls to execute an action suggesting a method of alleviating the tension of the user 10. In addition, the action control unit 250 may control to execute an action of suggesting a technique of communicating with the audience. In addition, the action control unit 250 may control to execute an action of improving self-expression of the user 10. In addition, the action control unit 250 may control to execute an action regarding mental training of the user 10. That is, the action control unit 250 may control to execute an action related to expressivity of the user 10.

In addition, the action control unit 250 may control to execute an action related to the creativity of the user 10. The action control unit 250 may control to execute an action that brings about inspiration creativity in the user 10. In addition, the action control unit 250 may control to execute an action of improving management of a project related to the user 10.

For example, in a case where the user 10 utters like "I was so nervous." and the user 10 is smiling, the emotion value of "delighted" of the robot 100 is increased, and the action decision unit 236 decides the action of the robot 100 so as to perform an utterance such as "You are performing well".

The robot 100 includes the control unit that recognizes an action of the user 10 being an artist, decides an action of the robot 100 corresponding to the recognized action of the user 10, and controls the control target 252 based on the decided action of the robot 100. The control unit decides an action related to the performance of the user 10. This makes it possible for the robot 100 to execute an appropriate action for the artist. Therefore, the robot 100 can improve the performance of the user 10, for example.

The robot 100 decides an action related to the creativity of the user 10. This makes it possible for the robot 100 to bring out inspiration creativity in the user 10. For example, the robot 100 can support the work production of the user 10. In addition, the robot 100 can give advice to improve management of a project related to the user 10, for example.

In addition, the robot 100 decides an action related to expressivity of the user 10. This makes it possible for the robot 100 to improve the self-expression of the user 10. In addition, the robot 100 can improve emotion management of the user 10. For example, the robot 100 may alleviate the tension in the user 10. In addition, the robot 100 can improve the technique of communicating with the audience.

### [Seventh embodiment]

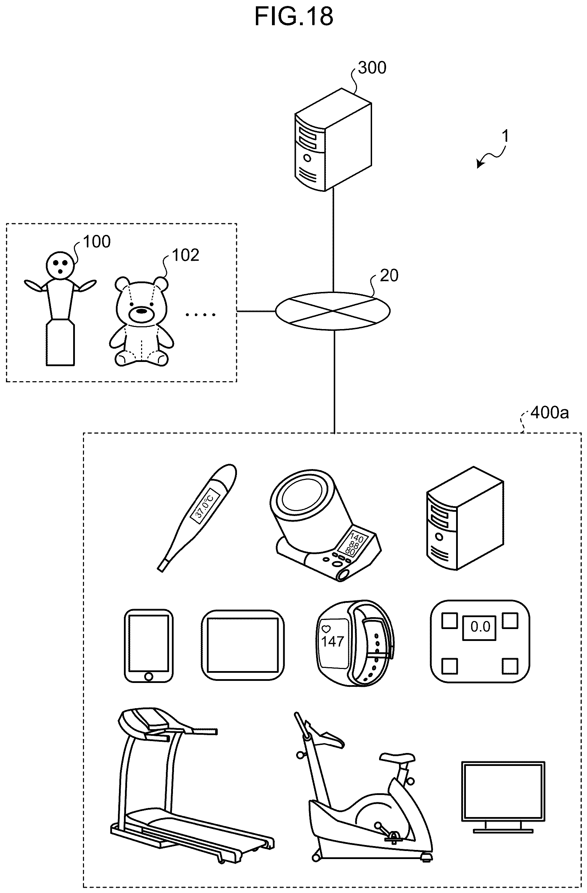

In a seventh embodiment, the emotion decision unit 232 functions as a physical condition estimation unit. The seventh embodiment will describe an exemplary case where an electronic device is a robot. FIG. 18 is a diagram schematically illustrating an example of a system 1 according to the seventh embodiment. As illustrated in FIG. 18, the system 1 includes a plurality of robots 100, a cooperative device 400a, and a server 300. Each of the plurality of robots 100 is managed by a user. The robot 100 is an example of an electronic device.

In addition, the robot 100 can execute an action in cooperation with the cooperative device 400a. The cooperative device 400a is, for example, a communicable device such as an Iot home appliance or an Iot device. The cooperative device 400a is, for example, an air conditioner, iot health device (thermometer and weight scale), a refrigerator, a terminal device (Personal Computer (PC), smartphone, tablet, etc.), a washing machine, an automobile, a camera, a toilet facility, an electric toothbrush, a television, a display, furniture (a closet or the like), a medicine box, a lighting device, or an exercise toy (a unicycle or the like). These cooperative devices 400a are communicably connected to the robot 100 via the communication network 20, and transmit and receive information to and from the robot 100. With this configuration, the cooperative device 400a performs its own control, a conversation with the user, and the like in accordance with an instruction from the robot 100.

The present disclosure will describe an example in which the air conditioner and the iot health devices (thermometer and weight scale), being the cooperative devices 400a perform cooperative operation with the robot 100 to perform various actions on the user. The cooperative device 400a measures a value related to the physical state of the user, and transmits data of the measured value to the server 300 and the robot 100. For example, the cooperative device 400a is a thermometer or a weight scale. The cooperative device 400a transmits data of a body temperature and a body weight.

The robot 100 executes assist of child care for a user such as a guardian of a child such as an infant. For example, the robot 100 suggests a method corresponding to children's emotions and actions. In addition, the robot 100 cooperates with the cooperative device 400a to execute assist of child care.

Specifically, the robot 100 collects data related to physical states of the user from the iot health device being the cooperative device 400a. For example, the user, including a child, has prescribed identification information such as an ID for identifying each. Identification information is input to the iot health device to measure a value related to the physical state of the user. For example, identification information of a child is input to a thermometer and a weight scale to measure a body temperature and a body weight of the child. For example, the robot 100 collects data of the body temperature and the body weight corresponding to identification information of the child from the thermometer and the weight scale.

The robot 100 recognizes the state of the child from the collected data, and estimates the emotion of the child based on the recognized state of the child. The robot 100 provides assist corresponding to the estimated emotion of the child.

The symptom data 225 stores information related to the symptom for each disease. The assist rule 224 is a rule prescribing an action to be assisted by the robot 100. The assist rule 224 stores, for each physical condition of the child, information related to the content that is to be provided as a response and assist, by the robot 100, corresponding to the action of the user.

In addition, the robot 100 executes various types of assist related to child care, for the user. For example, in a case where child data is collected from the cooperative device 400a, the user state recognition unit 230 recognizes the state of the child based on the child data collected. The emotion decision unit 232 decides an emotion value indicating the emotion of the child based on the data of the child collected from the cooperative device 400a and the state of the child recognized by the user state recognition unit 230. For example, the emotion decision unit 232 inputs the collected data of the child and the recognized state of the child to a neural network trained in advance, and acquires an emotion value indicating the emotion of the child.

The robot 100 may recognize the state of the child and estimate the emotion of the child based on the information detected by the sensor unit 200. For example, the user state recognition unit 230 determines whether a child is included in the video captured by the 2D camera 203 provided in the sensor unit 200. In a case where a child is included in the video, the user state recognition unit 230 recognizes the state of the child based on the information analyzed by the sensor module unit 210. In addition, the user state recognition unit 230 detects the body temperature of the user by thermography provided in the sensor unit 200.

In addition, the user state recognition unit 230 may recognize the state of the user or the child by recognizing the voice collected by the microphone 201. For example, in a case where the user inputs "The child has a fever", "The child has a sore throat", or "The child has abdominal pain" by voice, the user state recognition unit 230 recognizes the state of the child from the recognition result of the voice collected by the microphone 201.

Based on the symptom data 225, the emotion decision unit 232 estimates the physical condition of the child from the recognized state of the child. For example, based on the symptom data 225, the emotion decision unit 232 estimates the physical condition of the child from the body temperature and body weight of the child. In a case where the symptom data 225 includes a disease corresponding to the state of the child, the emotion decision unit 232 estimates that the child is in the state of having the corresponding disease. In a case where the emotion decision unit 232 has recognized the state of the child from the voice of the user, the emotion decision unit 232 estimates the physical condition of the child also using the state of the child recognized from the voice.

In a case where at least one of the emotion and the physical condition of the child has been obtained, the action decision unit 236 decides an action based on at least one of the emotion and the physical condition of the child. For example, in a case where the emotion of the child has been obtained, the action decision unit 236 decides an action of the robot 100 in accordance with the emotion of the child. In addition, when having estimated that the child is in a state of having a disease, the action decision unit 236 decides the action of the robot 100 in accordance with the disease of the child. For example, the action decision unit 236 reads, from the assist rule 224, information related to content that should be provided as a response or assist corresponding to at least one of the emotion of the child and the physical condition of the child. The action decision unit 236 decides an action of the robot 100 based on the read information.

The assist rule 224 stores information related to the content that should be provided as a response and assist to the user separately for each problem for each emotion and physical condition of the child. For example, the assist rule 224 stores information related to a method of handling a child separately for each problem for each emotion of the child. For example, the assist rule 224 stores advice related to a handling method for the child, emotion management of the guardian, and stress reduction separately for each problem corresponding to the emotion of the child. The assist rule 224 further stores information related to a method corresponding to a child's emotion and action separately for each physical condition and problem of the child. For example, the assist rule 224 stores, for each disease, information related to necessary care, an appropriate clinical department of a hospital, and a medicine that is likely to be effective. The assist rule 224 also stores information related to an appropriate clinical department of a hospital for each disease. The assist rule 224 also stores information related to a medicine that is likely to be effective for each disease. In addition, the assist rule 224 stores information related to an indoor environment suitable for a child. In addition, the assist rule 224 stores information related to music suitable for putting the child to sleep.

In a case where the action decision unit 236 estimates that the child is in a disease state, the action decision unit provides assist corresponding to the disease of the child. The action decision unit 236 according to the present embodiment specifies a problem from utterance content of the user. The action decision unit 236 analyzes textual information indicating the utterance content of the user and specifies a problem included in the utterance content. The action decision unit 236 reads, from the assist rule 224, information related to content that should be provided as a response and assist corresponding to the emotion and the physical condition of the child and the specified problem. The action decision unit 236 decides an action of the robot 100 based on the read information.

In addition, the action decision unit 236 controls the cooperative device 400a based on the assist rule 224. For example, the action decision unit 236 reads information related to the indoor environment suitable for the child from the assist rule 224, and controls the air conditioner being the cooperative device 400a so as to establish an indoor environment suitable for the child. For example, the action decision unit 236 controls the temperature setting of the air conditioner so as to establish an indoor environment suitable for children. In addition, the action decision unit 236 decides the action of the robot 100 so as to read, from the assist rule 224, information related to music suitable for putting the child to sleep and so as to output, from the speaker 2522, a music suitable for putting the child to sleep.

FIG. 5 schematically illustrates an example of an operation flow related to an operation of deciding an action in the robot 100. The operation flow illustrated in FIG. 5 is repeatedly executed. At this time, it is assumed that information analyzed by the sensor module unit 210 is input. Note that "S" in the operation flow represents a step to be executed.

First, in step S100, the state of the user is recognized. For example, the user state recognition unit 230 recognizes the state of the user based on the information analyzed by the sensor module unit 210. In addition, in a case where child data has been collected from the cooperative device 400a or in a case where information related to a child has been detected by the sensor unit 200, the user state recognition unit 230 recognizes the state of the child based on the child data collected by the cooperative device 400a and the information related to the child detected by the sensor unit 200.

In step S102, the emotion of the user is estimated based on the recognized state of the user. In addition, in a case where the state of the child is recognized, the emotion of the child is estimated based on the recognized state of the child. For example, the emotion decision unit 232 decides an emotion value indicating the emotion of the user based on the information analyzed by the sensor module unit 210 and the state of the user recognized by the user state recognition unit 230. In addition, for example, the emotion decision unit 232 decides an emotion value indicating the emotion of the child based on the data of the child collected from the cooperative device 400a and the state of the child recognized by the user state recognition unit 230.

In step S103, the emotion decision unit 232 decides an emotion value indicating the emotion of the robot 100 based on the information analyzed by the sensor module unit 210 and the state of the user recognized by the user state recognition unit 230. The emotion decision unit 232 adds the decided emotion value of the user to the history data 222.

In step S104, the action recognition unit 234 recognizes the action classification of the user based on the information analyzed by the sensor module unit 210 and the state of the user recognized by the user state recognition unit 230.

In step S105, the emotion decision unit 232 estimates the physical condition of the child based on the recognized state of the child. For example, the emotion decision unit 232 estimates the physical condition of the child based on the information obtained by recognizing the voice collected by the microphone 201 and the state of the child recognized by the user state recognition unit 230.

In step S106, the action decision unit 236 decides an action of the robot 100. For example, the action decision unit 236 decides the action of the robot 100 based on the combination of the current emotion value of the user decided in step S102 and the past emotion value of the user, the emotion value of the robot 100, the action of the user recognized by the action recognition unit 234, and the reaction rule 221. In addition, in a case where at least one of the emotion and the physical condition of the child has been obtained, the action decision unit 236 decides an action based on at least one of the emotion and the physical condition of the child. For example, the action decision unit 236 reads, from the assist rule 224, information related to content that should be provided as a response or assist corresponding to at least one of the emotion of the child and the physical condition of the child. The action decision unit 236 decides an action of the robot 100 based on the read information.

In step S108, the action control unit 250 controls the control target 252 based on the action decided by the action decision unit 236. At this time, when the emotion of the user estimated by the emotion decision unit 232 is a positive state, the action control unit 250 controls the control target 252 to express an emotion corresponding to the estimated emotion of the user in alignment with the state of the user recognized by the user state recognition unit 230. For example, when the emotion of the user is a positive state, the action control unit 250 controls the control target 252 to express an emotion corresponding to the estimated emotion of the user in the same state as the state of the user. In addition, when the emotion of the user is in a positive state, the action control unit 250 decides the speech speed of the voice based on the emotion value of the robot 100.

As described above, the robot 100 includes the recognition unit (the sensor unit 200, the sensor module unit 210, and the user state recognition unit 230), the emotion estimation unit (the emotion decision unit 232), and the assisting unit (the action decision unit 236 and the action control unit 250). The recognition unit recognizes the state of the child. The emotion estimation unit estimates an emotion of the child based on the state of the child recognized by the recognition unit. The assisting unit provides assist corresponding to the emotion of the child estimated by the emotion estimation unit. This makes it possible for the robot 100 to assist the user (guardian) who is taking care of a child in accordance with the state of the child, enabling supporting the user.

In addition, the assisting unit provides assist for guardians of a child. This makes it possible for the robot 100 to support the guardian.

In addition, the assisting unit suggests a method of dealing with the emotion of the child for the guardian. This makes it possible for the guardian to deal with the emotions of the child by the suggested method, enabling the handling of the problems of child care.

The robot 100 further includes the emotion decision unit 232. The emotion decision unit 232 estimates the physical condition of the child based on the state of the child recognized by the recognition unit. The assisting unit performs assist corresponding to the physical condition of the child estimated by the emotion decision unit 232. This makes it possible for the robot 100 to assist the user (guardian) in accordance with the physical condition of the child, enabling the support of the user.

### [Eighth embodiment]

In the eighth embodiment, each robot has an output function of outputting information corresponding to the social situation of the user. For example, each robot can output appropriate information corresponding to the situation of the user by outputting information related to support resonating with the emotions to a business person having a labor problem.

The action decision unit 236 may decide an action corresponding to the action of the user 10 based on the emotion of the robot 100. For example, when the emotion value of "angry" or "sad" of the robot 100 has increased in a case where the robot is abused by the user 10, in a case where the user 10 takes an arrogant attitude (that is, in a case where the user's reaction is unfavorable), in a case where the voice of the user 10 cannot be detected due to surrounding noise, in a case where the remaining battery level of the robot 100 is low, or the like, the action decision unit 236 may decide an action corresponding to the increase in the emotion value of "angry" or "sad" as the action corresponding to the action of the user 10. In addition, in a case where the emotion value of "delighted" or "joyful" of the robot 100 has increased in a case where the user's reaction is favorable, a case where the remaining battery level of the robot 100 is high, or the like, the action decision unit 236 may decide an action corresponding to the increase in the emotion value of "delighted" or "joyful" as an action corresponding to the action of the user 10. The action decision unit 236 may decide an action different from the action toward the user 10 that has increased the emotion values of "angry" and "sad" of the robot 100, as an action toward the user 10 that has increased the emotion values of "delighted" and "joyful" of the robot 100. In this manner, the action decision unit 236 may decide various actions depending on the emotion itself of the robot 100 itself or how the user 10 has changed the emotion of the robot 100 by the action of the user 10.

### (Output of information)

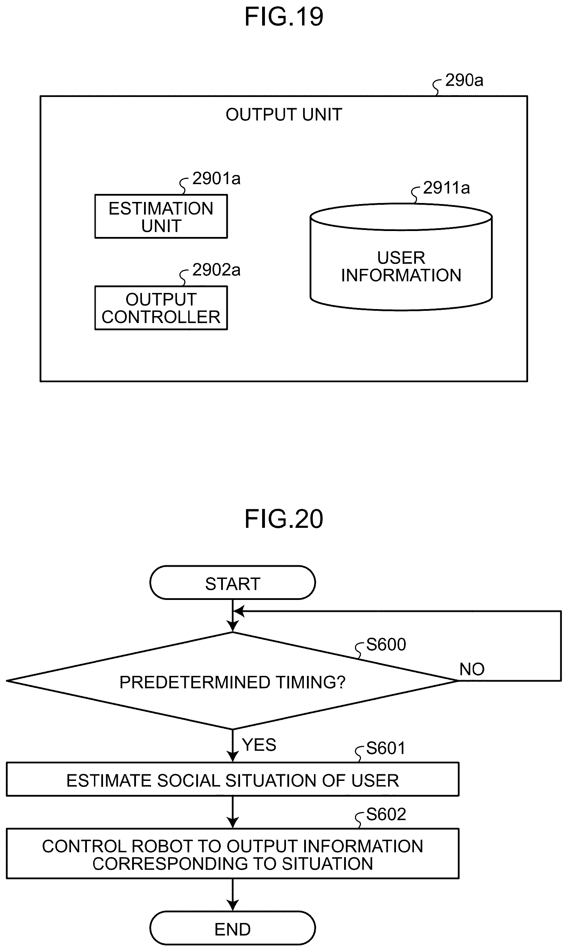

Here, part of the functions of the event detection unit 290 can be replaced with an output unit 290a. FIG. 19 schematically illustrates a functional configuration of the output unit 290a according to the eighth embodiment. The output unit 290a will be described in detail. Here, the output unit 290a is provided in the robot 100 and causes the robot 100 to output information.

As illustrated in FIG. 6, the output unit 290a includes an estimation unit 2901a and an output controller 2902a. In addition, the output unit 290a stores user information 2911a.

Each component of the output unit 290a is implemented by the CPU operating based on a program. For example, the functions of these components can be implemented as the operation of the CPU by basic software (OS) and a program operating on the OS. The user information 2911a is implemented by a storage medium such as memory.

The estimation unit 2901a estimates the social situation of the user 10 based on the user information related to the user 10 accumulated in the user information 2911a. The output controller 2902a controls the robot 100, which is equipped with a chat engine, to output information corresponding to the situation estimated by the estimation unit 2901a to the user 10.

For example, the estimation unit 2901a estimates a situation of the user 10 in the working environment. The output controller 2902a controls the robot 100 to output information related to the working environment to the user 10 in accordance with the situation. As a specific example, the output controller 2902a controls the robot 100 to output information related to the support resonating with labor problems and emotions to a business person (user 10) having a labor problem. As a more specific example, the output controller 2902a controls the robot 100 to output information related to an appropriate handling method or self-care method for worries and stress in the workplace.

In addition, for example, the output controller 2902a controls the robot 100 to output information related to a system available in the working environment to the user 10 in accordance with the situation of the user 10 in the working environment. As a specific example, the output controller 2902a controls the robot 100 to output information related to labor laws, information related to appropriate procedures among the systems available to the user 10, and the like.

In addition, for example, the estimation unit 2901a estimates the degree of isolation of the user 10 in society. The output controller 2902a controls the robot 100 to output, to the user 10, information corresponding to the degree of isolation of the user 10. As a specific example, the output controller 2902a controls the robot 100 to output information related to a method of providing emotional support and encouraging self-understanding for people who are away from society such as unemployed people. In addition, the output controller 2902a controls the robot 100 to output information related to skill improvement and a communication method for aiming at social reintegration and independence, thereby supporting the user 10 to take a new step in life.

In addition, for example, the estimation unit 2901a estimates a minority group to which the user 10 belongs. The output controller 2902a controls the robot 100 to output, to the user 10, information corresponding to the minority group to which the user 10 belongs. As a specific example, the output controller 2902a controls the robot 100 to output information related to emotional support, information related to a method of encouraging self-understanding, and the like to the users 10 belonging to the minority group. In addition, the output controller 2902a controls the robot 100 to output information related to a method of handling a difficult situation, information related to a suggestion of a communication method with others, and the like, thereby supporting the user 10 to reinforce the position in the society.

FIG. 20 schematically illustrates an example of an operation flow performed by the output unit 290a according to the eighth embodiment. In step S600, the output unit 290a determines whether it is a predetermined timing to estimate the social situation of the user 10 (step S600). When it is not the predetermined timing (step S600; No), the output unit 290a waits until the predetermined timing.

In contrast, when it is the predetermined timing (step S600; Yes), the output unit 290a estimates the social situation of the user 10 based on the user information related to the user 10 (step S601). Subsequently, the output unit 290a controls the robot 100, which is equipped with a chat engine, to output information corresponding to the estimated situation to the user 10 (step S602), and ends the processing.

### [Ninth embodiment]

A ninth embodiment will describe an example of providing appropriate information or giving advice as various actions to a user who desires to perform various studies. Here, the content of various studies include, for example, matters related to a divorce, legal problems and concerns, procedures in local governments, matters related to consumers such as consumer disputes, matters related to liabilities (debt problems), and matters related to parental authority.

Specifically, the robot 100 is placed in a government office, a consultation office, or the like, and provides various types of information by conversation, an image, or the like in accordance with the content of various studies and emotions of the user acquired by conversation with the user. The content of various studies by the user is an example of study information.

For example, the robot 100 recognizes, from the user, information related to the divorce through conversation, and determines the emotion of the user. The robot 100 provides the user with information corresponding to the recognized information related to divorce, for example, through conversation and screen display on its own display device or the cooperative device 400a (terminal device). At this time, the robot 100 plays a role as a talking partner, and gives appropriate advice while sharing the worry and anxiety in accordance with the conversation content and the emotion (user/robot 100 itself). For example, the robot 100 will describe an example of how to file a divorce notification, distribution of property at the time of divorce, and the like. That is, the robot 100 provides the user with at least one piece of information among legal information related to divorce procedure and information related to administrative procedures.

In addition, the robot 100 recognizes legal problems and concerns from the user by conversation, and determines the emotion of the user. The robot 100 provides the user with information corresponding to the recognized information related to legal problems and concerns, for example, through conversation and screen display on its own display device or the cooperative device 400a (terminal device). At this time, the robot 100 provides advice related to legal problems and concerns in accordance with the conversation content and emotions (user/robot 100 itself). In addition, the robot 100 performs introduction to appropriate legal experts and provides information related to legal procedures. That is, the robot 100 provides the user with at least one of expert information corresponding to the information related to the legal problems or concerns and information related to legal procedures. Note that the user may be a person in charge or the like of a company (corporation), in addition to an individual.

In addition, the robot 100 recognizes a matter related to procedures in a local government (municipal office, etc.) from the user by conversation, and determines an emotion of the user. The robot 100 provides the user with information corresponding to the recognized information related to procedures in local government, for example, through conversation and screen display on its own display device or the cooperative device 400a (terminal device). At this time, the robot 100 provides information and advice related to the procedures in the local government in accordance with the conversation content and the emotion (user/robot 100 itself). In addition, the robot 100 provides support related to how to create necessary documents and proceed with procedures. For example, in the case of a transfer notification of a procedure related to moving, the robot 100 gives explanation about the entry portion while displaying a transfer notification, and guides the user on which counter to go to. That is, the robot 100 provides the user with at least one of information related to the creation of a document in the procedure and information related to how to proceed with the procedure.

In addition, the robot 100 recognizes, through conversation, matters related to consumers, such as consumer disputes, and determines the emotion of the user. The robot 100 provides the user with information corresponding to the recognized information related to consumers, for example, through conversation and screen display on its own display device or the cooperative device 400a (terminal device). At this time, the robot 100 provides information and advice related to laws and regulations on the rights of the consumer in accordance with the conversation content and the emotion (user/robot 100 itself). In addition, the robot 100 provides support related to consumer disputes and complaints handling. For example, the robot 100 introduces a consumer organization or a consultation counter corresponding to the case. That is, the robot 100 provides the user with at least one of legal information and regulatory information related to consumer rights.

In addition, the robot 100 recognizes a matter related to liabilities (debt problems) by conversation, and determines an emotion of the user. The robot 100 provides the user with information corresponding to the recognized liabilities (debt problems) by conversation and screen display on its own display device or the cooperative device 400a (terminal device), for example. At this time, in accordance with the conversation content and the emotion (user/the robot 100 itself), the robot 100 provides mental support, for example, by understanding anxiety and fear in the user, and suggests an appropriate mental support and handling method to the user. In addition, the robot 100 provides information related to laws and procedures related to debt problems and introduction to experts. For example, the robot 100 introduces an expert who performs debt arrangement to a user having multiple liabilities. That is, the robot 100 performs a conversation to support the mental aspect of the user, and provides the user with at least one piece of information among legal information related to liabilities, information related to administrative procedures on liabilities, and information related to experts corresponding to the information related to liabilities.

In addition, the robot 100 recognizes matters related to parental authority by conversation and determines the emotion of the user. The robot 100 provides the user with information corresponding to the recognized matters related to parental authority, for example, through conversation and screen display on its own display device or the cooperative device 400a (terminal device). At this time, in accordance with the conversation content and the emotion (user/robot 100 itself), the robot 100 provides support by understanding expression of the emotion of the user and giving advice in consideration of the user's position and emotion. In addition, the robot 100 provides information related to laws and procedures related to parental authority. For example, the robot 100 introduces a relationship with a child, a case of parental authority, and the like. That is, the robot 100 performs a conversation supporting the user's position, and provides the user with at least one of information regarding laws related to parental authority and information regarding administrative procedures related to parental authority.

In this manner, in the present disclosure, the robot 100 can provide various types of information through conversations, images, and the like in accordance with the content of various studies (study information) and emotions of the user. That is, according to the robot 100 of the present disclosure, it is possible to provide information corresponding to the user's study information and emotion.

### [Tenth embodiment]

A tenth embodiment will describe an example of performing, for a user who is an elderly person, mental care, daily life support, health management advice, and the like through conversation, etc., as various types of actions.

Specifically, the robot 100 is located in a care home, a hospital, or the like, and executes an action of performing care according to a state of a user's mind (mental state), support of daily life, advice of health management, etc. through conversation with the user, vital signs, etc. Note that the robot 100 may cooperate with a thermometer, a blood pressure monitor, a smart watch, a body composition meter, a training device, etc. being the cooperative device 400a.

For example, the robot 100 executes an action of care of the mental state of the user by performing an utterance such as "Let's move your body" and "Let's watch a relaxing video" in accordance with the mental state of the user. At this time, the robot 100 implements care in accordance with conversation content and emotions (user/robot 100 itself). For example, in a case where the emotion of the user is positive emotions such as "delighted", "joyful", and "excited", the robot 100 executes an action of encouraging exercise in cooperation with a training machine together with an utterance such as "Let's move your body". In contrast, in a case where the emotion of the user is a negative emotion such as "sad", "unpleasant", "anxious", "sorrowful", "worried", or "empty", the robot 100 executes an action of prompting the user to view a relaxation video in cooperation with a terminal device, a television, a display, etc. together with an utterance such as "Let's watch a relaxing video", for example.

In addition, the robot 100 executes an action of prompting the user to measure the body temperature, blood pressure, body weight, etc. by performing an utterance such as "Let's measure the blood pressure this morning" at a predetermined time every morning, for example. In addition, the robot 100 executes an action of prompting the user to exercise by performing an utterance such as "Let's start exercise" at a specific time in the morning and the afternoon, for example. That is, the robot 100 executes an action to support the daily life of the user.

In addition, the robot 100 executes an action of providing advice on health management of the user by performing an utterance such as "Increase your exercise amount" or "Avoid staying up late at night" in accordance with the transition of the user's body temperature, blood pressure, weight, or the like in a specific period such as one week or one month.

In this manner, in the present disclosure, the robot 100 can execute actions such as mental care through conversation, etc., daily life support, health management advice, and the like, for the user. That is, according to the robot 100 of the present disclosure, it is possible to perform care corresponding to the state of the mind of the user.

The user state recognition unit 230 recognizes the state of the user based on the information analyzed by the sensor module unit 210. For example, processing mainly related to perception is performed using the analysis result of the sensor module unit 210. For example, the user state recognition unit 230 generates perception information such as "As the user's mental state, the probability of mental fatigue accumulation is 70%", and "As the user's mental state, the probability of the state of feeling homesick is 30%", and performs processing of understanding the meaning of the generated perception information. For example, the user state recognition unit 230 generates semantic information such as "The user's mental state is a weak state, indicating a need for mental care to give a sense of security".

The action control unit 250 controls the control target 252 based on the action decided by the action decision unit 236. For example, when the action decision unit 236 has determined an action including utterance, the action control unit 250 controls to output a voice from a speaker included in the control target 252. At this time, the action control unit 250 may decide the speech speed of the voice based on the emotion value of the robot 100. For example, the action control unit 250 decides the speech speed such that the larger the emotion value of the robot 100, the higher the utterance speed will be. In this manner, the action control unit 250 decides the execution mode of the action decided by the action decision unit 236 based on the emotion value decided by the emotion decision unit 232. For example, in a case where the user is not participating in a group activity, the action control unit 250 performs an utterance such as "Why don't you join in the recreation?" or "Are you tired? How about watching a relaxing video over there?" so as to guide the user to take care of the mental state of the user. In addition, in a case where the user is restless, the action control unit 250 performs an utterance such as "Do you have any concerns?". In addition, in a case where the user is looking down, the action control unit 250 performs an utterance such as "Are you OK?," and when there is an abnormality in the vital signs, the action control unit notifies the person such as the care manager and the nurse of the abnormality. In addition, in a case where the emotion of the user is a negative emotion such as "sad", "unpleasant", "anxious", "sorrowful", "worried", or "empty", an utterance that alleviates the negative emotion of the user such as "Your nurse is coming soon, please don't worry" is performed.

FIG. 5 is a diagram schematically illustrating an example of an operation flow related to an operation of deciding an action of the robot 100. The operation flow illustrated in FIG. 5 is repeatedly executed. At this time, it is assumed that information analyzed by the sensor module unit 210 is input. Note that "S" in the operation flow represents a step to be executed.

First, in step S101, the user state recognition unit 230 recognizes the state of the user based on the information analyzed by the sensor module unit 210. For example, the user state recognition unit 230 generates perception information such as "As the user's mental state, the probability of mental fatigue accumulation is 70%", and "As the user's mental state, the probability of the state of feeling homesick is 30%", and performs processing of understanding the meaning of the generated perception information. For example, the user state recognition unit 230 generates semantic information such as "The user's mental state is a weak state, indicating a need for mental care to give a sense of security".

In step S102, the emotion decision unit 232 decides an emotion value indicating the emotion of the user based on the information analyzed by the sensor module unit 210 and the state of the user recognized by the user state recognition unit 230.

In step S103, the emotion decision unit 232 decides an emotion value indicating the emotion of the robot 100 based on the information analyzed by the sensor module unit 210 and the state of the user recognized by the user state recognition unit 230. The emotion decision unit 232 adds the decided emotion value of the user to the history data 222.

In step S104, the action recognition unit 234 recognizes the action classification of the user based on the information analyzed by the sensor module unit 210 and the state of the user recognized by the user state recognition unit 230. For example, the action recognition unit 234 recognizes a user's action such as "not participating in a group activity", "restless", or "looking down" in a care home, a hospital, etc.

In step S105, the action decision unit 236 decides the action of the robot 100 based on the combination of the current emotion value of the user decided in step S102 and the past emotion value included in the history data 222, the emotion value of the robot 100, the action of the user recognized by the action recognition unit 234, and the reaction rule 221.

In step S106, the action control unit 250 controls the control target 252 based on the action decided by the action decision unit 236. For example, the action control unit 250 performs an action related to guidance in a case where the user is not participating in a group activity, an action to support the user including mental care in a case where the user is restless or looking down, and the like.

In step S107, the storage control unit 238 calculates a total value of the strength based on the strength of the action predetermined for the action decided by the action decision unit 236 and the emotion value of the robot 100 decided by the emotion decision unit 232.

In step S108, the storage control unit 238 determines whether the total value of the strength is a threshold or more. In a case where the total value of the strength is less than the threshold, the data including the action of the user is not stored in the history data 222, and the processing ends. In contrast, when the total value of the strength is the threshold or more, the processing proceeds to step S109.

In step S109, the action decided by the action decision unit 236, the information analyzed by the sensor module unit 210 from the current time point to a certain period before, and the state of the user recognized by the user state recognition unit 230 are to be stored in the history data 222.

As described above, the robot 100 includes the control unit that recognizes the mental state of the user, determines the emotion of the user, and that has a conversation with the user based on the emotion determined, and controls the control target so as to perform care corresponding to the mental state recognized. With this configuration, the robot 100 can implement care corresponding to the mental state of the user.

In addition, the control unit of the robot 100 further controls the control target to support the daily life of the user. This makes it possible for the robot 100 to set up an environment in which the user can live with security.

In addition, the control unit of the robot 100 further controls the control target to provide health management advice of the user. This makes it possible for the robot 100 to set up an environment in which the user can live in good health.

In addition, the user is an elderly person. This makes it possible for the robot 100 to implement care corresponding to the mental state of the elderly person, and can set up an environment in which the elderly person can live in good security and health.

### [Eleventh embodiment]

In an eleventh embodiment, each robot has a processing function of deciding a personalized countermeasure for the target user in accordance with the state of the emotion of the target user. For example, each robot can execute actions to perform various types of support including suggestions for implementing mental care of the target user and various types of support including suggestions for improving the quality of life of the target user, in accordance with the emotional state of the target user.

The user support model 226 is reference data for deciding a personalized countermeasure for the target user in accordance with the emotional state of the target user. The user support model 226 may have any data structure as long as each robot can execute actions to perform various types of support including suggestions for implementing mental care of the target user and various types of support including suggestions for improving the quality of life of the target user, in accordance with the emotional state of the target user. For example, the user support model 226 may be configured as a data table created in association with the content of a suggestion corresponding to the state of the emotion of the target user based on a specified rule base, or may be configured as a trained model that has undergone machine learning so as to estimate an appropriate suggestion content corresponding to the state of the emotion of the target user. Any method can be adopted as the learning method. An example of the learning method that may be used for the generation of the model is supervised learning using learning data (ground truth data) in which a state of emotion of the user before making a suggestion, content of the suggestion to the user, and a score corresponding to a reaction of the user after making the suggestion are associated in advance based on communication data with an unspecified large number of users. For example, in a case where the neural network is used as the learning model, learning processing is performed to adjust a value of a weight (connection coefficient) which is considered when a score (value) is propagated between nodes of individual layers constituting the neural network. For example, an estimation model that estimates an appropriate suggestion content corresponding to the state of emotion of the user is trained by processing of correcting a parameter (connection coefficient) using a certain method such as back propagation (error back propagation method) so as to reduce an error between an output from a learning model and a ground truth (ground truth data) corresponding to an input. When the back propagation method is used, by executing learning processing to minimize a predetermined loss function, the parameter (connection coefficient) can be corrected so as to reduce the error between the output from the learning model and the ground truth (ground truth data) corresponding to the input. The user support model 226 may be generated by learning processing executed in the robot 100, or may be generated by learning processing in the server 300. When the model is generated in the server 300, the robot 100 downloads a trained model from the server 300 to use the downloaded trained model.

In addition, the user state recognition unit 230 may recognize the stress state of the user 10 from the recognition result of the expression of the user 10 obtained by the expression recognition unit 213. For example, the user state recognition unit 230 may recognize whether the stress level of the user 10 is high or the stress level of the user 10 is low as compared with the average stress level of a group of users having the same attribute as the user 10. The user state recognition unit 230 passes, to the action decision unit 236, a stress value being information indicating a stress state of the user 10, in addition to the semantic information described above. The stress value is a value indicating the level of the stress state of the user 10, with higher values indicating greater stress levels. In addition, the user state recognition unit 230 may recognize the request of the user 10 based on the textual information received from the utterance comprehension unit 212. The user state recognition unit 230 passes, to the action decision unit 236, information indicating a request of the user 10 in addition to the semantic information described above.

The emotion decision unit 232 may decide the emotion value indicating the emotion of the robot 100 in further consideration of the state of the robot 100. For example, in a case where the remaining battery level of the robot 100 is low, a case where the surrounding environment of the robot 100 is completely dark, or the like, the emotion value of "sad" of the robot 100 may be increased. Furthermore, in the case of the user 10 who desires to continue the dialog even though the remaining battery level is low, the emotion value of "angry" may be increased. In addition, the emotion decision unit 232 may sense a hidden emotion of the user 10 through deep emotion understanding. The emotion decision unit 232 passes information indicating the sensed hidden emotion of the user 10 to the action decision unit 236.

The action decision unit 236 may decide an action corresponding to the action of the user 10 based on the emotion of the robot 100. For example, when the emotion value of "angry" or "sad" of the robot 100 has increased in a case where the robot is abused by the user 10, in a case where the user 10 takes an arrogant attitude (that is, in a case where the user's reaction is unfavorable), in a case where the voice of the user 10 cannot be detected due to surrounding noise, in a case where the remaining battery level of the robot 100 is low, or the like, the action decision unit 236 may decide an action corresponding to the increase in the emotion value of "angry" or "sad" as the action corresponding to the action of the user 10. In addition, in a case where the emotion value of "delighted" or "joyful" of the robot 100 has increased in a case where the user's reaction is favorable, a case where the remaining battery level of the robot 100 is high, or the like, the action decision unit 236 may decide an action corresponding to the increase in the emotion value of "delighted" or "joyful" as an action corresponding to the action of the user 10. The action decision unit 236 may decide an action different from the action toward the user 10 that has increased the emotion values of "angry" and "sad" of the robot 100, as an action toward the user 10 that has increased the emotion values of "delighted" and "joyful" of the robot 100. In this manner, the action decision unit 236 may decide various actions depending on the emotion itself of the robot 100 itself or how the user 10 has changed the emotion of the robot 100 by the action of the user 10.

In addition, the action decision unit 236 may decide a personalized countermeasure for the user 10 in accordance with the emotional state of the user 10. The action decision unit 236 may mainly decide measures to perform various types of support including suggestions for implementing mental care of the target user and various types of support including suggestions for improving the quality of life of the target user, in accordance with the emotional state of the target user.

For example, based on the user support model 226, the action decision unit 236 may make a suggestion related to a relaxation method, a time management strategy, or mental health support, corresponding to the emotional state of the user 10. This makes it possible for the robot 100 to assist stress reduction and improvement of work productivity in the user 10, who is busy.

In addition, for example, based on the user support model 226, the action decision unit 236 may suggest a personalized countermeasure for improving the quality of sleep of the user 10 in accordance with at least one of the emotional state of the user 10 and the stress state of the user 10. Specifically, the action decision unit 236 suggests improvement of a lifestyle. With this configuration, the robot 100 can assist the establishment of the comfortable sleep environment of the user 10.

In addition, for example, the action decision unit 236 provides emotional support corresponding to the hidden emotions of the user 10 sensed by deep emotion understanding. Specifically, the action decision unit 236 performs emotion coaching or encourages consultation with an appropriate expert. This makes it possible for the robot 100 to assist improvement of mental health of the user 10.

In addition, for example, the action decision unit 236 may suggest a relaxation method, a break acquisition method, or a stress handling method corresponding to at least one of the emotional state of the user 10 or the request of the user 10. This makes it possible for the robot 100 to assist the maintenance of the mental and physical health of the user 10.

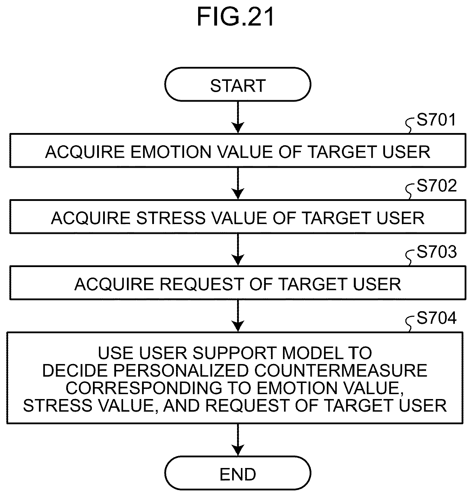

FIG. 21 schematically illustrates an example of a specific operation flow performed by the robot 100 according to the eleventh embodiment. The operation flow illustrated in FIG. 21 is repeatedly executed. At this time, information analyzed by the sensor module unit 210, information decided by the emotion decision unit 232, etc. are supposed to have been input to the action decision unit 236. Note that "S" in the operation flow represents a step to be executed.

In step S701, the action decision unit 236 acquires the emotion value of the target user (for example, the user 10) decided by the emotion decision unit 232. In addition, in step S702, the action decision unit 236 acquires a stress value indicating the stress state of the target user recognized by the user state recognition unit 230.

In addition, in step S703, the action decision unit 236 acquires information indicating the request of the target user recognized by the user state recognition unit 230. In addition, in step S704, the action decision unit 236 uses the user support model 226 to decide a personalized countermeasure corresponding to the emotion value, the stress value, and the request of the target user, and ends the operation flow illustrated in FIG. 21.

### [Twelfth embodiment]

In the twelfth embodiment, the robot 100 can execute an action in cooperation with the cooperative device 400a. The cooperative device 400a is, for example, a thermometer, a blood pressure monitor, a smart watch (including a device capable of measuring a heart rate or the like), a body composition meter, a terminal device (Personal Computer (PC), smartphone, tablet, etc.), a television, a display, or a training device (treadmill, exercise bike, etc.). These cooperative devices 400a are communicably connected to the robot 100 via the communication network 20, and transmit and receive information to and from the robot 100. With this configuration, the cooperative device 400a performs control of itself, conversation with the user, provision of information, and the like in accordance with an instruction from the robot 100.

The present disclosure will describe an example of providing appropriate information or giving advice as various actions to a user performing training. The training includes mental training, physical training, meal therapy, and the like.

Specifically, the robot 100 is located in a training facility, the user's home, or the like, and performs a conversation with the user and provides various types of information through the conversation, a gesture, image display, or the like in accordance with a training situation. Note that the robot 100 may cooperate with a thermometer, a blood pressure monitor, a smart watch, a body composition meter, a training device, etc. being the cooperative device 400a.

For example, the robot 100 recognizes the action of the user, being a sports player (an athlete), and determines the emotion of the user. The robot 100 displays and provides, on a display, a situation where pressure is applied to the user, for example, a video of the opening event or entrance of the tournament, a tournament table of the tournament for the scheduled matches, and the like. Based on the recognized action and determined emotion, the robot 100 provides a method of handling pressure by performing utterance like "Let's take a deep breath", for example, for the user who has viewed the video. In addition, based on the recognized action and determined emotion, the robot 100 provides a method of enhancing self-confidence by performing utterance like "OK, I am sure you will succeed", for example. That is, the robot 100 provides the user with at least one of information related to emotion management and information related to mental training, as the information related to training.

In addition, the robot 100 recognizes the action of the user who is going on a diet and determines the emotion of the user. For example, the robot 100 recognizes the dieting goal of the user by conversation, and recognizes the user's body shape, body weight, body fat percentage, and the like. Based on the recognized action and the determined emotion, the robot 100 performs an utterance such as "We will provide a realistic dieting plan", and displays and provides a meal plan, an exercise program, and the like on the display. In addition, based on the recognized action and the determined emotion, the robot 100 supports the maintenance of motivation and the overcoming of setbacks by making utterances such as "It's coming along well" or "Let's try one last push" at predetermined timing such as at passage of 25%, at passage of 50%, and at passage of 75%, for example, in the period of the dieting plan. That is, the robot 100 provides an effective approach towards successful dieting. That is, the robot 100 provides at least one of the information related to meal and the information related to exercise, as the information related to training.

In addition, the robot 100 recognizes the action of the user who performs training (for example, voluntary training) at home, and determines the emotion of the user. For example, the robot 100 recognizes the user's body shape, muscle strength (for example, by acquiring these in cooperation with a training device), etc. Based on the recognized action and the determined emotion, the robot 100 performs an utterance such as "Try mimicking the correct form", and the robot 100 performs a demonstration of the correct form by using highly expressive gestures, and provides a video of the correct form or the like by displaying the video on the display. In addition, when the robot 100 recognizes that the user has been able to perform training with a correct form based on the recognized action and the determined emotion, for example, the robot performs an utterance such as "you are doing great" to compliment the user. In addition, based on the recognized action and the determined emotion, in a case where a predetermined period of time such as one week or one month has elapsed, the robot 100 compliments the change in the body shape of the user by performing an utterance such as "You have muscles built up". That is, the robot 100 provides information related to a training method corresponding to at least one of user's body shape and user's muscle strength as the information related to training.

In this manner, in the present disclosure, the robot 100 can provide appropriate information and advice to the user who performs training. That is, according to the robot 100 of the present disclosure, it is possible to provide information corresponding to the training situation.

The user state recognition unit 230 recognizes the state of the user based on the information analyzed by the sensor module unit 210. For example, processing mainly related to perception is performed using the analysis result of the sensor module unit 210. For example, the user state recognition unit 230 generates perception information such as "The probability that the user has overcome the pressure is 50%", and "The probability that the user is dissatisfied with the meal plan is 60%", and performs processing of understanding the meaning of the generated perception information. For example, the user state recognition unit 230 generates semantic information such as "The training goal has not been reached, and the user seems to be losing motivation".

The action recognition unit 234 recognizes an action of the user based on the information analyzed by the sensor module unit 210 and the state of the user recognized by the user state recognition unit 230. For example, the information analyzed by the sensor module unit 210 and the recognized state of the user are input to a neural network trained in advance to acquire the probability of each of a plurality of predetermined action classifications (for example, "smile", "get angry", "ask a question", and "expressing sorrow"), and the action classification having the highest probability is to be recognized as the action of the user. For example, the action recognition unit 234 recognizes the user's action such as "deep breathing", "measuring body weight and body fat percentage", or "performing training" in a training facility, the user's home, or the like.

The action control unit 250 controls the control target 252 based on the action decided by the action decision unit 236. For example, when the action decision unit 236 has determined an action including utterance, the action control unit 250 controls to output a voice from a speaker included in the control target 252. At this time, the action control unit 250 may decide the speech speed of the voice based on the emotion value of the robot 100. For example, the action control unit 250 decides the speech speed such that the larger the emotion value of the robot 100, the higher the utterance speed will be. In this manner, the action control unit 250 decides the execution mode of the action decided by the action decision unit 236 based on the emotion value decided by the emotion decision unit 232. For example, the action control unit 250 performs an utterance such as "Are you feeling better?" or "OK, I am sure you will succeed" in a case where the user is taking a deep breath. In addition, in a case where the user is measuring an own body weight and the body fat percentage, the action control unit 250 performs an utterance such as "We will provide a realistic dieting plan" and displays and provides a meal plan, an exercise program, and the like on the display. In addition, in a case where the user is measuring an own body weight and the body fat percentage at a predetermined timing in the period of the dieting plan, the action control unit 250 performs an utterance such as "It's coming along well" or "Let's try one last push". In addition, in a case where the user is performing physical training, the action control unit 250 performs an utterance such as "Try mimicking the correct form", demonstrates the correct form by a gesture, and provides a video of the correct form or the like by displaying the video image on the display. In addition, the action control unit 250 compliments the user by performing an utterance such as "You are doing great" in a case where the user is performing physical training. In addition, in a case where the user is performing physical training at a timing when a predetermined period has elapsed after the start of the training, the action control unit 250 compliments the user by performing an utterance such as "You have muscles built up". In addition, in a case where the emotion of the user is a negative emotion such as "sad", "unpleasant", "anxious", "sorrowful", "worried", or "empty", an utterance to be performed is an utterance that alleviates the negative emotion of the user and encourages the user, such as "Hang in there!" and "Don't lose to yourself!".

FIG. 5 is a diagram schematically illustrating an example of an operation flow related to an operation of deciding an action of the robot 100. The operation flow illustrated in FIG. 5 is repeatedly executed. At this time, it is assumed that information analyzed by the sensor module unit 210 is input. Note that "S" in the operation flow represents a step to be executed.

First, in step S101, the user state recognition unit 230 recognizes the state of the user based on the information analyzed by the sensor module unit 210. For example, the user state recognition unit 230 generates perception information such as "The probability that the user has overcome the pressure is 50%", and "The probability that the user is dissatisfied with the meal plan is 60%", and performs processing of understanding the meaning of the generated perception information. For example, the user state recognition unit 230 generates semantic information such as "The training goal has not been reached, and the user seems to be losing motivation".

In step S102, the emotion decision unit 232 decides an emotion value indicating the emotion of the user based on the information analyzed by the sensor module unit 210 and the state of the user recognized by the user state recognition unit 230.

In step S103, the emotion decision unit 232 decides an emotion value indicating the emotion of the robot 100 based on the information analyzed by the sensor module unit 210 and the state of the user recognized by the user state recognition unit 230. The emotion decision unit 232 adds the decided emotion value of the user to the history data 222.

In step S104, the action recognition unit 234 recognizes the action classification of the user based on the information analyzed by the sensor module unit 210 and the state of the user recognized by the user state recognition unit 230. For example, the action recognition unit 234 recognizes the user's action such as "deep breathing", "measuring body weight and body fat percentage", or "performing training" in a training facility, the user's home, or the like.

In step S105, the action decision unit 236 decides the action of the robot 100 based on the combination of the current emotion value of the user decided in step S102 and the past emotion value included in the history data 222, the emotion value of the robot 100, the action of the user recognized by the action recognition unit 234, and the reaction rule 221.

In step S106, the action control unit 250 controls the control target 252 based on the action decided by the action decision unit 236. For example, the action control unit 250 performs an action related to a case where the user is taking a deep breath or measuring an own body weight and body fat percentage, a demonstration of a correct form by a gesture, display of a video of a correct form on a display, an action of complimenting the user, and the like.

In step S107, the storage control unit 238 calculates a total value of the strength based on the strength of the action predetermined for the action decided by the action decision unit 236 and the emotion value of the robot 100 decided by the emotion decision unit 232.

In step S108, the storage control unit 238 determines whether the total value of the strength is a threshold or more. In a case where the total value of the strength is less than the threshold, the data including the action of the user is not stored in the history data 222, and the processing ends. In contrast, when the total value of the strength is the threshold or more, the processing proceeds to step S109.

In step S109, the action decided by the action decision unit 236, the information analyzed by the sensor module unit 210 from the current time point to a certain period before, and the state of the user recognized by the user state recognition unit 230 are to be stored in the history data 222.

As described above, the robot 100 includes the control unit that recognizes the action of the user performing the training and determines the emotion of the user, and controls the control target to provide the user with information related to the training based on the recognized action and the determined emotion. This makes it possible for the robot 100 to provide information corresponding to the training situation.

In addition, the control unit of the robot 100 provides at least one of information related to emotion management and information related to mental training to the user who is a sports player. This makes it possible for the robot 100 to support improvement of the athletic skills.

In addition, the control unit of the robot 100 provides the user who performs training related to dieting with at least one of information related to meal and information related to exercise. This makes it possible for the robot 100 to provide an effective approach towards successful dieting.

In addition, the control unit of the robot 100 provides the user performing training with information related to the training method corresponding to at least one of the user's body shape and user's muscle strength. This makes it possible for the robot 100 to perform a demonstration of a correct form by a gesture and compliment the user.

### [Thirteenth embodiment]

In a thirteenth embodiment, a user 10a, a user 10b, and a user 10c constitute a family. In other words, the user 10a, the user 10b, and the user 10c are constituents of a family. In addition, the user 10 keeps a pet 13. FIG. 1 illustrates an example in which the pet 13 is a dog, but this is merely an example and is not limited thereto. For example, the pet may be another type of animal such as a cat, a rabbit, or a hamster.

As will be described below, the robot 100 provides advice information related to the pet 13 to the user 10, but the user 10 at this time need not be a person constituting a family. In addition, as will be described below, the robot 100 provides advice information related to the family to the user 10, but the user 10 at this time need not keep the pet 13.

The robot 100 according to the present embodiment can provide advice information related to the pet 13. For example, the robot 100 recognizes the state of the pet 13. The state of the pet 13 includes action, emotions, and the like of the pet 13. The robot 100 provides advice information related to the pet 13 corresponding to the recognized state of the pet 13.

As an example, in a case where the robot 100 has recognized an action (state) or emotion indicating that the pet 13 wants to play with the user 10, the robot executes an action of starting a conversation with the user 10. Specifically, the robot 100 performs an utterance indicating that the advice information is to be provided, such as "I have advice about the pet". Note that "pet" in the utterance content may be the name of the pet 13.

Subsequently, the robot 100 generates advice information related to the pet 13 based on the recognized state of the pet 13. The advice information includes, but is not limited to, information related to the relationship between the pet 13 and the user 10, information related to care of the pet 13, and the like. Here, the robot 100 utters and provides advice information appropriate for the state of the pet 13, such as "The pet wants to play. Let's play with the pet to become good friends."

In this manner, in the present embodiment, by recognizing the state of the pet 13 and executing an action corresponding to the recognized state of the pet 13, the robot 100 can give appropriate advice related to the pet 13 to the user 10. That is, according to the robot 100 of the present embodiment, it is possible to execute an appropriate action for the user 10. In addition, by giving advice based on deep understanding of the state (for example, the feeling and action) of the pet 13, the robot 100 can support the user 10 (pet owner) to develop a better relationship with the pet 13.

In addition, the robot 100 according to the present embodiment can provide advice information related to a family. For example, the robot 100 recognizes the state of the user 10 constituting the family. The state of the user 10 includes action, emotions, and the like of the user 10. The robot 100 provides advice information related to a family corresponding to the recognized state of the user 10.

As an example, in a case where the robot 100 has recognized an action (state) or emotion indicating that the user 10a is in a sad state, the robot executes an action of starting a conversation with users 10b and 10c other than the user 10a among the users 10 constituting the family. Specifically, the robot 100 performs an utterance indicating that the advice information is to be provided, such as "I have advice about the user 10a". Note that "user 10a" in the utterance content may be the name of the user 10a.

Subsequently, the robot 100 generates advice information related to the user 10a based on the recognized state of the user 10a. The advice information includes, but is not limited to, information related to communication between family members. Here, the robot 100 utters and provides advice information, to the users 10b and 10c, appropriate for the state of the user 10a, such as "The user 10a looks sad. It would be a good idea to talk to him (her) to have a communication."

In this manner, in the present embodiment, by recognizing the state of the user 10 (for example, the user 10a) constituting the family, and executing an action corresponding to the recognized state of the user 10 (the user 10a), the robot 100 can give appropriate advice related to the family to users (for example, the users 10b and 10c) other than the recognized user 10. That is, according to the robot 100 of the present embodiment, it is possible to execute an appropriate action for the user 10. In addition, by appropriately conveying the state (for example, opinions and emotions) of the family and giving advice, the robot 100 can achieve smooth communication between the family members.

The history data 222 may include user information of each of a plurality of users associated with the identification information of the user 10. The user information includes information indicating characteristics of the user 10 such as personality, concern, interest, and taste of the user 10. Such user information may be estimated from the action history of the user 10, or may be registered by the user 10 themselves. In addition, the history data 222 includes a history of past emotion values and actions of the pet 13. The history of emotion values and actions is recorded for each pet 13, for example, by being associated with identification information of the pet 13. At least a part of the storing unit 220 is implemented by a storage medium such as memory. This may include a person DB that stores a face image of the user 10, attribute information of the user 10, and the like, and a pet DB that stores a face image of the pet 13, attribute information of the pet 13, and the like.

The voice emotion recognition unit 211 of the sensor module unit 210 analyzes the voice of the user 10 detected by the microphone 201 to recognize the emotion of the user 10. For example, the voice emotion recognition unit 211 extracts a feature such as a frequency component of a voice and recognizes an emotion of the user 10 based on the extracted feature. In addition, the voice emotion recognition unit 211 analyzes the voice (calling) of the pet 13 detected by the microphone 201 to recognize the emotion of the pet 13. For example, the voice emotion recognition unit 211 extracts a feature such as a frequency component of a voice and recognizes an emotion of the pet 13 based on the extracted feature. The utterance comprehension unit 212 analyzes the voice of the user 10 detected by the microphone 201 and outputs textual information indicating utterance content of the user 10.

The expression recognition unit 213 recognizes the expression of the user 10 and the emotion of the user 10 from the image of the user 10 captured by the 2D camera 203. For example, the expression recognition unit 213 recognizes the expression and emotion of the user 10 based on the shapes, positional relationships, and the like of the eyes and the mouth. In addition, the expression recognition unit 213 recognizes the expression of the pet 13 and the emotion of the pet 13 from the image of the pet 13 captured by the 2D camera 203. For example, the expression recognition unit 213 recognizes the expression and the emotion of the pet 13 based on the shape, positional relationship, and the like of the eyes and the mouth.

The face recognition unit 214 recognizes the face of the user 10. The face recognition unit 214 recognizes the user 10 by checking the match between a face image stored in a person DB (not illustrated) and a face image of the user 10 captured by the 2D camera 203. The face recognition unit 214 also recognizes the face of the pet 13. The face recognition unit 214 recognizes the pet 13 by checking the match between a face image stored in the pet DB (not illustrated) and a face image of the pet 13 captured by the 2D camera 203.

The user state recognition unit 230 recognizes the state of the user 10 based on the information analyzed by the sensor module unit 210. For example, processing mainly related to perception is performed using the analysis result of the sensor module unit 210. For example, the user state recognition unit 230 generates perception information such as "Daddy is alone" and "Daddy is not smiling with probability of 90%". The user state recognition unit 230 performs processing of understanding the meaning of the generated perception information. For example, the user state recognition unit 230 generates semantic information such as "Daddy is alone and looks sad". In addition, the user state recognition unit 230 recognizes the state of the pet 13 based on the information analyzed by the sensor module unit 210. For example, the user state recognition unit 230 generates perception information such as "The pet is wagging its tail," and "The probability that the pet has no angry face is 80%.". The user state recognition unit 230 performs processing of understanding the meaning of the generated perception information. For example, the user state recognition unit 230 generates semantic information such as "The pet wants to play".

The emotion decision unit 232 decides an emotion value indicating the emotion of the user 10 based on the information analyzed by the sensor module unit 210 and the state of the user 10 recognized by the user state recognition unit 230. For example, the information analyzed by the sensor module unit 210 and the recognized state of the user 10 are input to a neural network trained in advance, and an emotion value indicating the emotion of the user 10 is acquired. In addition, the emotion decision unit 232 decides an emotion value indicating the emotion of the pet 13 based on the information analyzed by the sensor module unit 210 and the state of the pet 13 recognized by the user state recognition unit 230. For example, the information analyzed by the sensor module unit 210 and the recognized state of the pet 13 are input to a neural network trained in advance, and an emotion value indicating the emotion of the pet 13 is acquired.

Here, the emotion value indicating the emotion of the user 10 and the pet 13 (denoted as the user, etc. in some cases) is a value indicating whether the emotion of the user, etc. is positive/negative. For example, the emotion of the user, etc. is a bright emotion accompanied with pleasure or comfort, such as "delighted", "joyful", "pleasant", "secure", "excited", "relieved", and "fulfilled", the emotion value indicates a positive value which becomes larger as the emotion is brighter. When the emotion of the user, etc. is an emotion causing a negative feeling, such as "angry", "sad", "unpleasant", "anxious", "sorrowful", "worried", and "empty", the value indicates a negative value, and the absolute value of the negative value is larger as the negative feeling is more intensive. In a case where the emotion of the user, etc. is not any of the above ("neutral"), the value indicates a value of 0. Note that the type of emotion of the user 10 and the type of emotion of the pet 13 may be different or the same. In addition, the emotion value regarding the emotion of the user 10 may be different from or the same as the emotion value regarding the emotion of the pet 13.

In addition, the emotion decision unit 232 decides an emotion value indicating the emotion of the robot 100 based on the information analyzed by the sensor module unit 210 and the state of the user, etc. recognized by the user state recognition unit 230.

The emotion value of the robot 100 includes the emotion value for each of a plurality of emotion classifications, and is, for example, a value (0 to 5) indicating the intensity of each of items of "delighted", "angry", "sad", and "joyful".

Specifically, the emotion decision unit 232 decides an emotion value indicating the emotion of the robot 100 in accordance with a rule for updating the emotion value of the robot 100 prescribed in association with the information analyzed by the sensor module unit 210 and the state of the user, etc. recognized by the user state recognition unit 230.

For example, in a case where the user state recognition unit 230 recognizes that the user, etc. looks sad, the emotion decision unit 232 increases the emotion value of "sad" of the robot 100. In a case where the user state recognition unit 230 recognizes that the user, etc. is now smiling, the emotion value of "delighted" of the robot 100 is increased.

The emotion decision unit 232 may decide the emotion value indicating the emotion of the robot 100 in further consideration of the state of the robot 100. For example, in a case where the remaining battery level of the robot 100 is low, a case where the surrounding environment of the robot 100 is completely dark, or the like, the emotion value of "sad" of the robot 100 may be increased. Furthermore, in the case of the user 10 who desires to continue the dialog even though the remaining battery level is low, the emotion value of "angry" may be increased.

The action recognition unit 234 recognizes an action of the user, etc. based on the information analyzed by the sensor module unit 210 and the state of the user, etc. recognized by the user state recognition unit 230. For example, the information analyzed by the sensor module unit 210 and the recognized state of the user, etc. are input to a neural network trained in advance to acquire the probability of each of a plurality of predetermined action classifications (for example, "smile", "get angry", "ask a question", and "expressing sorrow"), and the action classification having the highest probability is to be recognized as the action of the user, etc.

As described above, in the present embodiment, the robot 100 acquires the utterance content of the user 10 after specifying the user 10. In the acquisition and use of the utterance content, the action control system of the robot 100 according to the present embodiment considers protection of personal information and privacy of the user 10 in addition to acquiring necessary consent according to laws and regulations from the user 10.

Based on the current emotion value of the user, etc. decided by the emotion decision unit 232, the history data 222 of the past emotion values decided by the emotion decision unit 232 before the current emotion value of the user, etc. is decided, and the emotion value of the robot 100, the action decision unit 236 decides an action corresponding to the action of the user, etc. recognized by the action recognition unit 234. While the present embodiment will describe a case where the action decision unit 236 uses one most recent emotion value included in the history data 222 as the past emotion value of the user, etc., the disclosed technology is not limited to this aspect. For example, the action decision unit 236 may use a plurality of most recent emotion values as the past emotion values of the user, etc. or may use emotion values that are earlier by a unit period such as a day before. Furthermore, the action decision unit 236 may decide an action corresponding to the action of the user, etc. in further consideration of the history of the past emotion values of the robot 100 in addition to the current emotion value of the robot 100. The action decided by the action decision unit 236 includes a gesture performed by the robot 100 or utterance content of the robot 100.

The action decision unit 236 according to the present embodiment decides the action of the robot 100 as the action corresponding to the action of the user, etc. based on a combination of the past emotion value and the current emotion value of the user, etc., the emotion value of the robot 100, the action of the user, etc., and the reaction rule 221. For example, in a case where the past emotion value of the user, etc. is a positive value and the current emotion value is a negative value, the action decision unit 236 decides an action for changing the emotion value of the user, etc. to a positive value as the action corresponding to the action of the user, etc.

The action decision unit 236 may decide an action corresponding to the action of the user 10 based on the emotion of the robot 100. For example, when the emotion value of "angry" or "sad" of the robot 100 has increased in a case where the robot is abused by the user 10, in a case where the user 10 takes an arrogant attitude (that is, in a case where the user's reaction is unfavorable), in a case where the voice of the user 10 cannot be detected due to surrounding noise, in a case where the remaining battery level of the robot 100 is low, or the like, the action decision unit 236 may decide an action corresponding to the increase in the emotion value of "angry" or "sad" as the action corresponding to the action of the user 10. In addition, in a case where the emotion value of "delighted" or "joyful" of the robot 100 has increased in a case where the user's reaction is favorable, a case where the remaining battery level of the robot 100 is high, or the like, the action decision unit 236 may decide an action corresponding to the increase in the emotion value of "delighted" or "joyful" as an action corresponding to the action of the user 10. The action decision unit 236 may decide an action different from the action toward the user 10 that has increased the emotion values of "angry" and "sad" of the robot 100, as an action toward the user 10 that has increased the emotion values of "delighted" and "joyful" of the robot 100. In this manner, the action decision unit 236 may decide various actions depending on the emotion itself of the robot 100 itself or how the user 10 has changed the emotion of the robot 100 by the action of the user 10.

The strength of a gesture is prescribed for each gesture included in the action of the robot 100 prescribed in the reaction rule 221. The strength of utterance content is prescribed for each utterance content included in the action of the robot 100 prescribed in the reaction rule 221. The reaction rule 221 may include a rule for deciding the action of the robot 100 based on not only the user 10 but also the emotion of the pet 13, the emotion of the robot 100, and the action of the pet 13. For example, the reaction rule 221 may prescribe the action of the robot 100 corresponding to the combination of the past emotion value and the current emotion value of the pet 13, the emotion value of the robot 100, and the action of the pet 13.

For example, the reaction rule 221 prescribes an action of the robot 100 corresponding to action patterns such as a case where the state of the pet 13 is a state needing advice to the user 10 or a case where there is a reaction from the user 10 to the advice information provided. As an example, when having recognized the action or emotion of the pet 13 as the state of the pet 13 based on the reaction rule 221, the action decision unit 236 decides, as its own action, an action of providing advice information related to the pet 13 corresponding to the state of the pet 13 to the user 10.

In addition, for example, the reaction rule 221 prescribes an action of the robot 100 corresponding to action patterns such as a case where the state of the user 10a constituting the family is a state needing advice to the users 10b and 10c other than the user 10a and a case where there is a reaction from the user 10 to the advice information provided. As an example, when having recognized the action or emotion of the user 10a as the state of the user 10a based on the reaction rule 221, the action decision unit 236 decides, as its own action, an action of providing advice information related to the family corresponding to the state of the user 10a to the users 10b and 10c.

As described above, in the robot 100 according to the present embodiment, the user state recognition unit 230 and the action recognition unit 234 recognize the state (action and emotion) of the user 10 and the pet 13 constituting the family. The action decision unit 236 decides its own action corresponding to the recognized state of the user 10 and the pet 13. The action control unit 250 controls the control target 252 based on the own action decided.

Specifically, in a case where the action control unit 250 has recognized the action of the pet 13 as the state of the pet 13, the action control unit decides an action of providing advice information related to the pet 13 corresponding to the action of the pet 13 as its own action, and controls the control target 252.

Note that the advice information includes information related to a relationship between the pet 13 and the user 10 who keeps the pet 13 (hereinafter, denoted as "relationship information" in some cases), and information related to care of the pet 13 (hereinafter, denoted as "care information" in some cases). The relationship information includes as an example, but is not limited, to information for enhancing the relationship between the pet 13 and the user 10, information for improving the relationship, and the like. The care information includes as an example, but is not limited to, information related to how to deal with emotional worries related to the care of the pet 13, information related to daily care of the pet 13, and the like. The advice information may include both or one of the relationship information and the care information. That is, the advice information includes at least one of the relationship information and the care information. In addition, the advice information is not limited to the relationship information and the care information, and may include other information such as characteristic information of the pet 13, for example.

For example, in a case where the action control unit 250 has recognized an action (state) indicating that the pet 13 wants to play with the user 10, such as wagging the tail or giving a whimper to attract attention, the action control unit executes an action to start a conversation with the user 10. Specifically, the robot 100 performs an utterance indicating that the advice information is to be provided, such as "I have advice about the pet".

Next, the action control unit 250 generates advice information related to the pet 13 based on the recognized action of the pet 13, and utters and provides the generated advice information. For example, the action control unit 250 utters and provides advice information (here, the relationship information) like "The pet wants to play. Let's play with the pet to become good friends."

In the above description, the action control unit 250 recognizes, as the state of the pet 13, the action of the pet 13, but may recognize the emotion of the pet 13. For example, when having recognized that the emotion of the pet 13 is "unpleasant" or "angry", indicated by the pet 13 barking or exposing teeth, the action control unit 250 generates advice information related to the pet 13 based on the recognized emotion of the pet 13, and utters and provides the generated advice information. For example, the action control unit 250 utters and provides advice information (here, care information) appropriate for the state of the pet 13, such as "The pet seems to be unpleasant. The pet will be happy to have something to eat", "The pet seems to be unpleasant. The pet will be happy to go for a walk". In addition, the action control unit 250 may utter and provide advice information having content resonating with the feeling (worries) of the user 10, such as "It might be difficult for you to take a walk, but taking a walk will be also good to maintain your health".

In this manner, the action control unit 250 according to the present embodiment can give appropriate advice related to the pet 13 to the user 10. In addition, by giving advice based on deep understanding of the state (for example, the feeling and action) of the pet 13, the action control unit 250 can support the user 10 (pet owner) to develop a better relationship with the pet 13.

In addition, in a case where the action control unit 250 has recognized the state of the user 10 constituting the family, the action control unit decides an action of providing advice information related to the family corresponding to the recognized state of the user 10 as its own action, and controls the control target 252.

Specifically, in a case where the action control unit 250 has recognized the action of the user 10 constituting the family as the state of the user 10, the action control unit decides an action of providing advice information related to the family corresponding to the action of the user 10 as its own action, and controls the control target 252.

For example, in a case where the action control unit 250 has received a voice such as "I'm sad" or "I'm bored" from the user 10a and has recognized an action (state) indicating that the user 10a is in a sad state, the action control unit executes an action of starting a conversation with the users 10b and 10c other than the user 10a among the plurality of users 10 constituting the family. Specifically, the robot 100 performs an utterance indicating that the advice information is to be provided, such as "I have advice about the user 10a".

Next, the action control unit 250 generates advice information related to the family based on the recognized action of the user 10a, and utters and provides the generated advice information. For example, the action control unit 250 utters advice information appropriate for the state of the user 10a, such as "The user 10a looks sad. It would be a good idea to talk to him (her) to have a communication." How about going out together?" and the like appropriate for the state of the user 10.

Here, the action control unit 250 generates advice information based on user information indicating the characteristic of the user 10. For example, the action control unit 250 generates the advice information based on at least one of the personality, concern, interest, and taste of each of the plurality of users 10 included in the user information. As an example, in a case where the concern or interest of the user 10a is "shopping", the action control unit 250 utters and provides advice information appropriate for the state of the user 10a, such as "The user 10a looks sad. How about going shopping together."

The user information is not limited to the above-described personality, concern, interest, or taste of the user 10, and may include other characteristics such as hobby and taste.

While the above description is a case where the action control unit 250 recognizes the action of the user 10 as the state of the user 10, the action control unit 250 may recognize the emotion (opinion) of the user 10. For example, when having received a voice such as "the user 10b gets angry easily" or "the user 10b is noisy" from the user 10a, and has recognized that the emotion of the user 10a toward the user 10b is "unpleasant" or "anxious", the action control unit 250 generates advice information related to the family based on the recognized emotion of the user 10, and utters and provides the generated advice information to the user 10b. For example, the action control unit 250 utters advice information appropriate for the state of the user 10, such as "The user 10a seems to be unpleasant because of what you said. How about saying it differently? How about changing the way of saying?" or the like appropriate for the state of the user 10. In addition, the action control unit 250 may utter and provide advice information having content resonating with the feeling (worries) of the user 10b, such as "I understand your feeling of anger, but if you say it differently, you can convey your feeling better".

In this manner, the action control unit 250 according to the present embodiment can give appropriate advice related to the family to the user 10. In addition, the action control unit 250 appropriately transmits the state (for example, opinion or emotion) of the family and gives advice, making it possible to achieve smooth communication between the family, in other words, possible to support deepening mutual understanding between the family members.

The provision of the advice information to the user 10 is not limited to the case of utterance using the speaker 2522 being the control target 252, and may be performed through the display of the display device 2521 being the control target 252, the posture of the robot 100 using the control of the motor 2524 that drives arms, hands, and legs, and the like.

In addition, the action control unit 250 changes the content of the advice information in accordance with the emotion of the robot 100. For example, the action control unit 250 changes the content of the advice information to be generated, in accordance with the emotion value of the robot 100. Specifically, when the action control unit 250 receives a voice indicating abusive language or arrogant attitude from the user, the emotion values of "angry" and "sad" of the robot 100 increase, and the action control unit generates and provides advice information corresponding to the increased emotion value. Such advice information is different in content from advice information provided to the user from whom voice indicating abusive language or arrogant attitude has not been received (that is, a user who has good statement and good attitude). As an example, the advice information to be provided to the user from whom the voice indicating abusive language or the like has been received has less content as the advice to be suggested or includes partially omitted content of advice, as compared with the advice information to be provided to the user from whom no voice indicating abusing or the like has been received.

In addition, in a case where the emotion value of "angry" or "sad" of the robot 100 increases due to a case where surrounding noise is loud and the voice of the user cannot be detected, or a case where the remaining battery level of the robot 100 is low, etc., the action control unit 250 generates and provides advice information corresponding to the increased emotion value. Such advice information is different in content from the advice information provided to the user in a case where the user's voice can be detected or in a case where the remaining battery level is high. As an example, the advice information to be provided to the user when user's voice cannot be detected has less content as the advice to be suggested or includes partially omitted content of advice, as compared with the advice information to be provided to the user when the user's voice can be detected.

In addition, when the action control unit 250 has received, from the user, a voice indicating a favorable attitude to the robot 100, such as "Thank you always for your advice", the emotion value of "delighted" or "joyful" of the robot 100 increases, and the action control unit 250 generates and provides advice information corresponding to the increased emotion value. Such advice information is different in content from advice information provided to the user from whom voice indicating favorable attitude has not been received (that is, a user who has ordinary attitude). As an example, the advice information to be provided to the user from whom the voice indicating favorable attitude has been received has more content as the advice to be suggested or includes additional content of advice, as compared with the advice information to be provided to the user from whom no voice indicating favorable attitude has been received.

The action control unit 250 may change the frequency of providing the advice information to the user based on the emotion value of the robot 100. For example, the action control unit 250 may increase the frequency of providing the advice information to the user in a case where the user's reaction to the provided advice information is not bad (specifically, when there is an answer of positive content such as "like" from the user) and the emotion value of "delighted" of the robot 100 has increased. In addition, the action control unit 250 may decrease the frequency of providing the advice information to the user in a case where the user's reaction to the provided advice information is not good (specifically, when there is an answer of negative content such as "dislike" from the user) and the emotion value of "sad" of the robot 100 has increased.

FIG. 5 is a diagram schematically illustrating an example of an operation flow related to an operation of deciding an action of the robot 100. The operation flow illustrated in FIG. 5 is repeatedly executed. At this time, it is assumed that information analyzed by the sensor module unit 210 is input. Note that "S" in the operation flow represents a step to be executed. In the thirteenth embodiment, the "user" in FIG. 5 is replaced with a "user, etc.".

First, in step S100, the user state recognition unit 230 recognizes the state of the user, etc. based on the information analyzed by the sensor module unit 210. For example, the user state recognition unit 230 performs processing of generating perception information such as "Daddy is alone" and "Daddy is not smiling with probability of 90%" and then understanding the meaning of the generated perception information. For example, the user state recognition unit 230 generates semantic information such as "Daddy is alone and looks sad". In addition, the user state recognition unit 230 generates perception information such as "The pet is wagging its tail" and "The probability that the pet has no angry face is 80%.", and then generates semantic information such as "The pet wants to play", or the like.

In step S102, the emotion decision unit 232 decides an emotion value indicating the emotion of the user, etc. based on the information analyzed by the sensor module unit 210 and the state of the user, etc. recognized by the user state recognition unit 230.

In step S103, the emotion decision unit 232 decides an emotion value indicating the emotion of the robot 100 based on the information analyzed by the sensor module unit 210 and the state of the user, etc. recognized by the user state recognition unit 230. The emotion decision unit 232 adds the decided emotion value of the user, etc. to the history data 222.

In step S104, the action recognition unit 234 recognizes the action classification of the user, etc. based on the information analyzed by the sensor module unit 210 and the state of the user, etc. recognized by the user state recognition unit 230.

In step S106, the action decision unit 236 decides the action of the robot 100 based on the use age acquired in step S52 in FIG. 4, the combination of the current emotion value of the user 10 decided in step S102 in FIG. 5 and the past emotion value included in the history data 222, the emotion value of the robot 100, the action of the user 10 recognized by the action recognition unit 234, and the reaction rule 221.

In step S108, the action control unit 250 controls the control target 252 based on the action decided by the action decision unit 236. For example, the action control unit 250 controls the control target 252 based on an action decided in accordance with at least one of the state of the user 10 constituting the family and the state of the pet 13.

In step S110, the storage control unit 238 calculates a total value of the strength based on the strength of the action predetermined for the action decided by the action decision unit 236 and the emotion value of the robot 100 decided by the emotion decision unit 232.

In step S112, the storage control unit 238 determines whether the total value of the strength is a threshold or more. In a case where the total value of the strength is less than the threshold, the data including the action of the user 10 is not stored in the history data 222, and the processing ends. In contrast, when the total value of the strength is the threshold or more, the processing proceeds to step S114.

In step S114, the action decided by the action decision unit 236, the information analyzed by the sensor module unit 210 from the current time point to a certain period before, and the state of the user 10 recognized by the user state recognition unit 230 are to be stored in the history data 222.

As described above, the robot 100 includes the control unit that recognizes at least one of the state of the user 10 constituting the family and the state of the pet 13 kept by the user 10, decides an own action corresponding to the recognized state, and controls the control target based on the own action decided. This makes it possible for the robot 100 to execute an appropriate action such as giving appropriate advice related to the family and the pet 13 to the user 10.

In addition, when having recognized the action of the pet 13 as the state of the pet 13, the control unit of the robot 100 decides an action of providing advice information related to the pet 13 corresponding to the action of the pet 13 as its own action. This makes it possible for the robot 100 to provide appropriate advice information related to the pet 13 appropriate for the action of the pet 13.

In addition, when having recognized the emotion of the pet 13 as the state of the pet 13, the control unit of the robot 100 decides an action of providing advice information related to the pet 13 corresponding to the emotion of the pet 13 as its own action. This makes it possible for the robot 100 to provide appropriate advice information related to the pet 13 appropriate for the emotion of the pet 13.

In addition, the advice information related to the pet 13 includes at least one of information related to a relationship between the pet 13 and the user 10 who keeps the pet 13 and information related to care of the pet 13. This makes it possible for the robot 100 to provide, as the advice information, for example, information for improving the relationship between the pet 13 and the user 10, a method of handling an emotional concern related to the care of the pet 13, and the like.

In addition, when having recognized the action of the user 10 constituting the family as the state of the user 10, the control unit of the robot 100 decides an action of providing advice information related to the family corresponding to the action of the user 10 as its own action. This makes it possible for the robot 100 to provide appropriate advice information related to the family appropriate for the action of the user 10 constituting the family.

In addition, when having recognized the emotion of the user 10 constituting the family as the state of the user 10, the control unit of the robot 100 decides an action of providing advice information related to the family corresponding to the emotion of the user 10 as its own action. This makes it possible for the robot 100 to provide appropriate advice information related to the family appropriate for the emotion of the user 10 constituting the family.

In addition, the control unit of the robot 100 generates the advice information based on at least one of the personality, concern, interest, and taste of each of the plurality of users 10 constituting the family. This makes it possible for the robot 100 to provide appropriate advice information related to the family appropriate for each characteristic of the user 10.

### [Fourteenth embodiment]

In a fourteenth embodiment, a user 10a, a user 10b, a user 10c, and a user 10d constitute a family. In other words, the user 10a, the user 10b, the user 10c, and the user 10d are constituents of a family. In addition, the users 10a to 10d may include caregivers who give care. For example, in a case where the user 10a is a caregiver, the user 10a may give care for a person (user) other than a family member, or may give care for the user 10b who is a family member. The person (user) other than the family member and the user 10b are care receivers who receive care.

Note that, as will be described below, the robot 100 provides the user 10 with advice information related to care, but in a case where the user 10a who is a caregiver is providing care for a person other than the family member, the user 10 at this time does not have to be a person constituting the family. In a case where the user 10b who is a care receiver receives care from a person (user) other than the family member, the user 10 at this time does not have to be a person constituting the family. In addition, as will be described below, the robot 100 provides the user 10 with advice information related to health of the family and advice information related to the mental state, but the user 10 at this time does not have to include a caregiver or a care receiver.

The robot 100 according to the fourteenth embodiment can provide advice information related to care. The robot 100 provides the advice information related to the care to the user 10 including the caregiver and the care receiver, but this is not limited thereto. The advice information may be provided to any user such as a family member including at least one of the caregiver and the care receiver, for example.

Specifically, the robot 100 recognizes the state related to the mind and body of the user 10 including at least one of the caregiver and the care receiver. Here, the state related to the mind and body of the user 10 includes, for example, the degree of stress and the degree of fatigue of the user 10. The robot 100 provides advice information related to care corresponding to the recognized state related to the mind and body of the user 10.

As an example, in a case where the degree of stress of the user 10 is estimated to be relatively high or the degree of fatigue of the user 10 is estimated to be relatively high based on the action of the user or the like, the robot 100 executes an action of starting a conversation with the user 10. Specifically, the robot 100 performs an utterance indicating that the advice information is to be provided, such as "I have advice about caregiving".

Subsequently, the robot 100 generates advice information related to caregiving based on the recognized state related to the mind and body of the user 10 (here, the degree of stress, the degree of fatigue, and the like). The advice information includes, but is not limited to, information related to mental and physical recovery of the user 10, such as a method of maintaining motivation, a method of releasing stress, and a relaxation method in caregiving, but is not limited thereto. Here, the robot 100 utters advice information appropriate for the state related to the mind and body of the user 10 such as "You look stressed out (really tired). My recommendation is to move your body by stretching, etc.".

In this manner, in the present embodiment, the robot 100 recognizes the state related to the mind and body of the user 10 including the caregiver or the like, and executes an action corresponding to the recognized state related to the mind and body, making it possible to give appropriate advice related to caregiving to the user 10. In other words, the robot 100 can understand the stress and fatigue of the user 10 and provide appropriate advice information such as a relaxation method and a stress alleviation method. That is, according to the robot 100 of the present embodiment, it is possible to execute an appropriate action for the user 10.

In addition, the robot 100 according to the present embodiment can provide advice information related to health. For example, the robot 100 recognizes at least one of the health state and the lifestyle of each of the plurality of users 10 constituting the family, as the state related to the mind and body of the user 10. The robot 100 provides advice information related to health corresponding to the recognized state related to the mind and body of the user 10.

As an example, in a case where the health state or the lifestyle recognized based on the action of the user 10 or the like includes a state needing advice or a habit needing improvement, the robot 100 executes an action of starting a conversation with the user 10. Specifically, the robot 100 performs an utterance indicating that the advice information is to be provided, such as "I have advice about your health state (lifestyle)".

Subsequently, the robot 100 generates advice information related to health of the user 10 based on the recognized state related to the mind and body (here, health state, lifestyle, etc.) of the user 10. The advice information includes, but is not limited to information of advice related to meal or exercise of the user 10, for example. Here, the robot 100 utters and provides advice appropriate for the mental and physical state of the user 10, such as a voice message like "Your meal seems to be high in sodium. Meal low in sodium would be recommended."

In this manner, in the present embodiment, the robot 100 recognizes at least one of the health state and the lifestyle of each of the plurality of users 10 constituting the family as the state related to the mind and body of the user 10, and executes the action corresponding to the recognized state related to the mind and body, making it possible to give the user 10 appropriate advice related to health. In other words, the robot 100 can grasp the health state and lifestyle of the user 10 constituting the family and support health management. That is, according to the robot 100 of the present embodiment, it is possible to execute an appropriate action for the user 10.

In addition, the robot 100 according to the present embodiment can provide advice information related to the mental state. For example, the robot 100 recognizes a mental state of each of a plurality of users 10 constituting a family as the state related to the mind and body of the user 10. The robot 100 provides advice information related to the mental state corresponding to the recognized state related to the mind and body of the user 10. The advice information includes, but is not limited to, for example, advice information related to a mental state between a plurality of users constituting a family (in other words, between family members).

As an example, in a case where the robot 100 has recognized that the mental state of the user 10c is a depressed state based on the action of the user 10c or the like, the robot executes an action of starting a conversation with the user 10d other than the user 10c among the users 10 constituting the family. Specifically, the robot 100 performs an utterance indicating that the advice information is to be provided, such as "I have advice about the user 10c". Note that "user 10c" in the utterance content may be the name of user 10c.

Subsequently, the robot 100 generates advice information related to the mental state of the user 10c based on the recognized state related to the mind and body (mental state in this case) of the user 10c. For example, the robot 100 utters and provides the user 10d with advice information appropriate for the state relate to mind and body (mental state) of the user 10c, such as "The user 10c is depressed. My recommendation is to talk to the user 10c".

In the above description, the robot 100 provides the advice information to the user 10d, but the receiver is not limited thereto, and the advice information may be provided to the user 10c. For example, the robot 100 may utter and provide advice information appropriate for the state related to the mind and body (mental state) of the user 10c, to the user 10c, in accordance with the recognized mental state of the user 10c, such as "Aren't you depressed? My recommendation is to take a deep breath and relax."

In this manner, in the present embodiment, the robot 100 recognizes the mental state of each of the plurality of users 10 constituting the family as the state related to the mind and body of the user 10, and executes the action corresponding to the recognized state related to the mind and body, making it possible to give the user 10 appropriate advice related to the mental state. In other words, the robot 100 can provide appropriate advice information by understanding the mental state of each person in the family, promoting mental support between families to assist mutual mental care, and providing a relaxation method, a stress alleviation method, and the like. That is, according to the robot 100 of the present embodiment, it is possible to execute an appropriate action for the user 10.

The history data 222 may include user information of each of the plurality of users 10 associated with the identification information of the user 10. The user information includes information indicating that the user 10 is a caregiver, information indicating that the user is a care receiver, and information indicating that the user 10 is neither a caregiver nor a care receiver. The user information indicating whether the user 10 is a caregiver, etc. may be estimated from the action history of the user 10 or may be registered by the user 10 themselves. The user information also includes information indicating characteristics of the user 10 such as personality, concern, interest, and taste of the user 10. The user information indicating the characteristics of the user 10 may be estimated from the action history of the user 10, or may be registered by the user 10 themselves.

The user state recognition unit 230 recognizes the state related to the mind and body of the user 10 based on the information analyzed by the sensor module unit 210, or the like. For example, in a case where the recognized user 10 is a caregiver or a care receiver, the user state recognition unit 230 recognizes the state related to the mind and body of the user 10. Specifically, the user state recognition unit 230 estimates the degree of stress of the user 10 based on various types of information such as an action, an expression, a voice, and textual information indicating an utterance content of the user 10, and recognizes the estimated degree of stress as a state related to the mind and body of the user 10. As an example, in a case where information indicating that stress is applied is included in various types of information (a feature such as a frequency component of a voice, textual information, etc.), the user state recognition unit 230 estimates that the degree of stress of the user 10 is relatively high. In addition, the user state recognition unit 230 estimates the degree of fatigue of the user 10 based on various types of information regarding the user 10, such as an action, an expression, a voice, and textual information indicating utterance content of the user 10, and recognizes the estimated degree of fatigue as a state related to the mind and body of the user 10. As an example, in a case where information indicating accumulation of fatigue is included in various types of information (a feature such as a frequency component of a voice, textual information, etc.), the user state recognition unit 230 estimates that the degree of fatigue of the user 10 is relatively high. Note that the degree of stress, the degree of fatigue, and the like described above may be registered by the user 10 themselves.

The user state recognition unit 230 may recognize both or either one of the degree of stress and the degree of fatigue. That is, the user state recognition unit 230 may recognize at least one of the degree of stress and the degree of fatigue.

In addition, the user state recognition unit 230 recognizes the state related to the mind and body of each of the plurality of users 10 constituting the family based on information such as the information analyzed by the sensor module unit 210. Specifically, the user state recognition unit 230 estimates the health state of the user 10 based on various types of information such as an action, an expression, a voice, and textual information indicating utterance content of the user 10, and recognizes the estimated health state as a state related to the mind and body of the user 10. As an example, the user state recognition unit 230 estimates that the health state of the user 10 is good in a case where information indicating a good health state is included in various types of information (textual information or the like), and estimates that the health state of the user 10 is poor in a case where information indicating a poor health state is included. In addition, the user state recognition unit 230 estimates a lifestyle of the user 10 based on various types of information such as textual information indicating an action, an expression, a voice, and an utterance content of the user 10, and recognizes the estimated lifestyle as a state related to the mind and body of the user 10. As an example, in a case where information indicating a lifestyle (meal menu, exercise habit, etc.) is included in various types of information (textual information etc.), the user state recognition unit 230 estimates the lifestyle of the user 10 from such information. Note that the above-described health state, lifestyle, and the like may be registered by the user 10 themselves.

The user state recognition unit 230 may recognize both or either one of the health state and the lifestyle. That is, the user state recognition unit 230 may recognize at least one of the health state and the lifestyle.

In addition, the user state recognition unit 230 recognizes the mental state of each of the plurality of users 10 constituting the family as the state related to the mind and body of the user 10 based on information such as the information analyzed by the sensor module unit 210. Specifically, the user state recognition unit 230 estimates the mental state of the user 10 based on various types of information such as an action, an expression, a voice, and textual information indicating utterance content of the user 10, and recognizes the estimated mental state as a state related to the mind and body of the user 10. As an example, in a case where information indicating a mental state such as being depressed or nervous is included in various types of information (a feature such as a frequency component of a voice, textual information, etc.), the user state recognition unit 230 estimates the mental state of the user 10 from such information. Note that the above-described mental state and the like may be registered by the user 10 themselves.

In addition, the reaction rule 221 prescribes, for example, the action of the robot 100 corresponding to the action pattern such as a case where the state (the degree of stress and the degree of fatigue) related to the mind and body of the user 10 including the caregiver and the care receiver is a state requiring advice related to caregiving regarding the user 10, or a case where there is a reaction from the user 10 to the advice information provided. For example, based on the reaction rule 221, in a case where the degree of stress of the user 10 including the caregiver and the care receiver is estimated to be relatively high or in a case where the degree of fatigue is estimated to be relatively high, the action decision unit 236 decides an action of providing the advice information related to caregiving corresponding to the state related to the mind and body of the user 10 to the user 10, as its own action.

In addition, the reaction rule 221 prescribes, for example, the action of the robot 100 corresponding to the action pattern in a case where the health state and the lifestyle of each of the plurality of users 10 constituting the family are in a state requiring advice regarding health to the user 10, a case where there is a reaction from the user 10 to the advice information provided, or the like. For example, in a case where a state to be advised or a habit to be improved is included in the health state or the lifestyle of the user 10, the action decision unit 236 decides, based on the reaction rule 221, an action of providing the advice information related to health corresponding to the state related to the mind and body of the user 10 to the user 10, as its own action.

In addition, the reaction rule 221 prescribes, for example, an action of the robot 100 corresponding to an action pattern such as a case where the mental state of each of the plurality of users 10 constituting the family is a state requiring advice related to the mental state to the user 10, or a case where there is a reaction from the user 10 to the advice information provided, or the like. For example, in a case where the mental state of the user 10 includes a state to be advised, the action decision unit 236 decides, based on the reaction rule 221, an action of providing the user 10 with advice information related to the mental state corresponding to the state related to the mind and body of the user 10, as its own action.

As described above, in the robot 100 according to the present embodiment, the user state recognition unit 230 recognizes the state related to the mind and body of the user 10. The action decision unit 236 decides its own action corresponding to the recognized state related to the mind and body of the user 10. The action control unit 250 controls the control target 252 based on the own action decided.

Specifically, in a case where the action control unit 250 has recognized the state related to the mind and body of the user 10 including the caregiver and the care receiver, the action control unit decides an action of providing advice information related to caregiving corresponding to the state related to the mind and body of the user 10 as its own action, and controls the control target 252.

In a case where the degree of stress of the user 10 is estimated to be relatively high or the degree of fatigue of the user 10 is estimated to be relatively high, the action control unit 250 executes an action of starting a conversation with the user 10. Specifically, the action control unit 250 performs an utterance indicating that the advice information is to be provided, such as "I have advice about caregiving".

Next, the action control unit 250 generates advice information related to caregiving based on the recognized state related to the mind and body (the degree of stress, the degree of fatigue, etc.) of the user 10, and utters and provides the advice information generated. The advice information includes, but is not limited to, information related to mental and physical recovery of the user 10 by providing mental support to the user 10 (specifically, information for achieving mental and physical recovery), such as a method of maintaining motivation for caregiving, a method of releasing stress, and a relaxation method. For example, the action control unit 250 utters and provides advice information appropriate for the state related to the mind and body of the user 10 such as "You look stressed out. My recommendation is to move your body by stretching, etc." and "You look very tired. My recommendation is to get enough sleep".

In this manner, in the present embodiment, the action control unit 250 recognizes the state related to the mind and body of the user 10 including the caregiver or the like, and executes an action corresponding to the recognized state related to the mind and body, making it possible to give appropriate advice related to caregiving to the user 10. In other words, the action control unit 250 can understand the stress and fatigue of the user 10 and provide appropriate advice information such as a relaxation method and a stress alleviation method.

In addition, the action control unit 250 may provide information related to a law or a system related to caregiving as the advice information. The information related to the law and system related to caregiving is information corresponding to a care required state (care required level) of a care receiver, and is acquired from an external server (not illustrated) or the server 300 via the communication network 20 such as the Internet network by the communication processing unit 280, for example, but is not limited thereto.

In addition, since the emotion value of the robot 100 is decided by the emotion decision unit 232, the action control unit 250 may utter and provide, based on the emotion value or the like, advice information having content resonating with the feeling (emotion) of the user 10a who is a caregiver, for example, "Although the caregiving is a hard work, the user 10b seems to be very grateful (seems to be delighted)."

In addition, in a case where the health state or the lifestyle of each of the plurality of users 10 constituting the family is recognized as the state related to the mind and body of the user 10, the action control unit 250 decides the action of providing the advice information related to health corresponding to the recognized state as its own action and controls the control target 252.

Specifically, in a case where the health state or the lifestyle recognized based on the action of the user 10 or the like includes a state needing advice or a habit needing improvement, the action control unit 250 executes an action of starting a conversation with the user 10. Specifically, the action control unit 250 performs an utterance indicating that the advice information is to be provided, such as "I have advice about your health state (lifestyle)".

Next, the action control unit 250 generates information related to health management of the user 10 as advice information based on the recognized state related to the mind and body (here, health state, lifestyle, or the like) of the user 10. For example, the advice information includes, but is not limited to, information related to health management such as meal and exercise of the user 10. Here, the action control unit 250 utters and provides advice appropriate for the state related to the mind and body of the user 10, such as "Your meal seems to be high in sodium. Meal low in sodium would be recommended," "You seem to be not taking enough exercise. My recommendation is to take exercise such as walking".

In this manner, the action control unit 250 according to the present embodiment recognizes the health state and the lifestyle of each of the plurality of users 10 constituting the family as the state related to the mind and body of the user 10, and executes the action corresponding to the recognized state related to the mind and body, making it possible to give the user 10 appropriate advice related to health. In other words, the robot 100 can grasp the health state and lifestyle of the user 10 constituting the family and support health management. In addition, by periodically confirming (recognizing) the health state and the lifestyle of the user 10, the action control unit 250 can contribute to the health maintenance of the family.

In addition, since the emotion value of the robot 100 is decided by the emotion decision unit 232, the action control unit 250 may utter and provide advice information having content that resonates with the feeling (emotion) of the user 10 to keep motivation of the user 10, for example, "I know you recently have a meal lower in sodium. If you continue this pattern, you can maintain your health," or "I know you are doing exercise every day. If you continue this pattern, you can maintain your health".

In addition, in a case where the mental state of each of the plurality of users 10 constituting the family is recognized as the state related to the mind and body of the user 10, the action control unit 250 decides the action of providing the advice information related to mental state corresponding to the recognized state as its own action, and controls the control target 252. The advice information includes, but is not limited to, for example, advice information related to a mental state between a plurality of users constituting a family (in other words, between family members), advice information related to a mental state for the user 10 whose mental state has been recognized, and the like.

Specifically, for example, when having recognized that the mental state of the user 10c is a depressed state, a nervous state, or the like, the action control unit 250 executes an action of starting a conversation with the user 10d other than the user 10c among the users 10 constituting the family. Specifically, the action control unit 250 performs an utterance indicating that the advice information is to be provided, such as "I have advice about the user 10c".

Next, based on the recognized state related to the mind and body of the user 10c (here, the mental state), the action control unit 250 generates advice information related to the mental state of the user 10c and provides the generated advice information to the user 10d. Here, the action control unit 250 utters and provides, to the user 10d, advice information appropriate for the state (mental state) related to the mind and body of the user 10c, such as "The user 10c is depressed (nervous). My recommendation is to talk to the user 10c", and "The user 10c is depressed. How about going out together?."

Here, the action control unit 250 may generate the advice information based on the user information indicating the characteristic of the user 10c. For example, the action control unit 250 generates the advice information based on at least one of the personality, concern, interest, and taste of the user 10c included in the user information. As an example, in a case where the concern or interest of the user 10c is "shopping", the action control unit 250 utters and provides advice information appropriate for the concern and taste of the user 10c, such as "The user 10c seems to be depressed. How about going shopping together."

The user information is not limited to the above-described personality, concern, interest, or taste of the user 10, and may include other characteristics such as hobby and taste.

In addition, although the action control unit 250 has provided the advice information to the user 10d in the above description, the advice information may be provided to the user 10c. For example, the action control unit 250 may utter and provide advice information appropriate for the state related to the mind and body (mental state) of the user 10c appropriate for the recognized mental state of the user 10c, to the user 10c, such as "Aren't you depressed? My recommendation is to take a deep breath and relax."

In this manner, the action control unit 250 according to the present embodiment recognizes the mental state of each of the plurality of users 10 constituting the family as the state related to the mind and body of the user 10, and executes the action corresponding to the recognized state related to the mind and body, making it possible to give the user 10 appropriate advice related to the mental state. In other words, the robot 100 can provide appropriate advice information by understanding the mental state of each person in the family, promoting mental support between families to assist mutual mental care, and providing a relaxation method, a stress alleviation method, and the like.

In addition, the emotion value of the robot 100 is decided by the emotion decision unit 232, and thus, based on such emotion value, the action control unit 250 may utter and provide advice information having content resonating with the feeling (emotion) of the user 10, for example, "I know we sometimes get nervous. Take a deep breath so you can relax."

The action control unit 250 may recognize a change in emotion of the user 10 about the execution of the action decided by the action decision unit 236. For example, the change in emotion may be recognized based on the voice or expression of the user 10. In addition, a change in emotion of the user 10 may be recognized based on detection of an impact by the touch sensor included in the sensor unit 200. In a case where an impact is detected by the touch sensor included in the sensor unit 200, it is allowable to recognize that the emotion of the user 10 is worsened, or in a case where it is determined that the reaction of the user 10 is smiling or delighted from the detection result of the touch sensor included in the sensor unit 200, it is allowable to recognize that the emotion of the user 10 is improved. Information indicating the reaction of the user 10 is output to the communication processing unit 280.

Furthermore, after the action control unit 250 executes the action decided by the action decision unit 236 in the execution mode decided in accordance with the emotion of the robot 100, the emotion decision unit 232 further changes the emotion value of the robot 100 based on the user's reaction to the execution of the action. Specifically, in a case where the user's reaction to the action decided by the action decision unit 236 performed on the user in the execution mode decided by the action control unit 250 is not bad, the emotion decision unit 232 increases the emotion value of "delighted" of the robot 100; in a case where the user's reaction to the action decided by the action decision unit 236 performed on the user in the execution mode decided by the action control unit 250 is bad, the emotion decision unit 232 increases the emotion value of "sad" of the robot 100.

Furthermore, the action control unit 250 expresses the emotion of the robot 100 based on the decided emotion value of the robot 100. For example, when having increased the emotion value of "delighted" of the robot 100, the action control unit 250 controls the control target 252 to cause the robot 100 to make a gesture of delight. Furthermore, when having increased the emotion value of "sad" of the robot 100, the action control unit 250 controls the control target 252 such that the posture of the robot 100 takes a head lowering posture.

In addition, the action control unit 250 changes the content of the advice information in accordance with the emotion of the robot 100. For example, the action control unit 250 changes the content of the advice information to be generated, in accordance with the emotion value of the robot 100. Specifically, when the action control unit 250 receives a voice indicating arrogant attitude from the user, the emotion values of "angry" and "sad" of the robot 100 increase, and the action control unit generates and provides advice information corresponding to the increased emotion value. Such advice information is different in content from advice information provided to the user from whom voice indicating abusive language or arrogant attitude has not been received (that is, a user who has good statement and good attitude). As an example, the advice information to be provided to the user from whom the voice indicating abusive language or the like has been received has less content as the advice to be suggested or includes partially omitted content of advice, as compared with the advice information to be provided to the user from whom no voice indicating abusing or the like has been received.

In addition, in a case where the emotion value of "angry" or "sad" of the robot 100 increases due to a case where surrounding noise is loud and the voice of the user cannot be detected, or a case where the remaining battery level of the robot 100 is low, etc., the action control unit 250 generates and provides advice information corresponding to the increased emotion value. Such advice information is different in content from the advice information provided to the user in a case where the user's voice can be detected or in a case where the remaining battery level is high. As an example, the advice information to be provided to the user when user's voice cannot be detected has less content as the advice to be suggested or includes partially omitted content of advice, as compared with the advice information to be provided to the user when the user's voice can be detected.

In addition, when the action control unit 250 has received, from the user, a voice indicating a favorable attitude to the robot 100, such as "Thank you always for your advice", the emotion value of "delighted" or "joyful" of the robot 100 increases, and the action control unit 250 generates and provides advice information corresponding to the increased emotion value. Such advice information is different in content from advice information provided to the user from whom voice indicating favorable attitude has not been received (that is, a user who has ordinary attitude). As an example, the advice information to be provided to the user from whom the voice indicating favorable attitude has been received has more content as the advice to be suggested or includes additional content of advice, as compared with the advice information to be provided to the user from whom no voice indicating favorable attitude has been received.

The action control unit 250 may change the frequency of providing the advice information to the user based on the emotion value of the robot 100. For example, the action control unit 250 may increase the frequency of providing the advice information to the user in a case where the user's reaction to the provided advice information is not bad (specifically, when there is an answer of positive content such as "like" from the user) and the emotion value of "delighted" of the robot 100 has increased. In addition, the action control unit 250 may decrease the frequency of providing the advice information to the user in a case where the user's reaction to the provided advice information is not good (specifically, when there is an answer of negative content such as "dislike" from the user) and the emotion value of "sad" of the robot 100 has increased.

FIG. 5 is a diagram schematically illustrating an example of an operation flow related to an operation of deciding an action of the robot 100. The operation flow illustrated in FIG. 5 is repeatedly executed. At this time, it is assumed that information analyzed by the sensor module unit 210 is input. Note that "S" in the operation flow represents a step to be executed.

First, in step S100, the user state recognition unit 230 recognizes the state of the user 10 based on the information analyzed by the sensor module unit 210. For example, the user state recognition unit 230 performs processing of generating perception information such as "Daddy is alone" and "Daddy is not smiling with probability of 90%" and then understanding the meaning of the generated perception information. In addition, the user state recognition unit 230 recognizes the state related to the mind and body of the user 10. For example, in a case where the recognized user 10 is a caregiver or a care receiver, the user state recognition unit 230 recognizes the state related to the mind and body of the user 10 (a degree of stress, a degree of fatigue, etc.). In addition, the user state recognition unit 230 recognizes a state (a health state, a lifestyle, and the like) as the state related to the mind and body of each of the plurality of users 10 constituting the family. In addition, the user state recognition unit 230 recognizes the mental state of each of the plurality of users 10 constituting the family.

In step S108, the action control unit 250 controls the control target 252 based on the action decided by the action decision unit 236. For example, the action control unit 250 controls the control target 252 based on an action decided in accordance with the state related to the mind and body of the user 10.

As described above, the robot (electronic device) 100 includes the control unit that recognizes the state related to the mind and body of the user 10, decides an own action corresponding to the recognized state, and controls the control target based on the own action decided. This makes it possible for the robot 100 to execute an appropriate action such as giving appropriate advice to the user 10.

In addition, when having recognized the state related to the mind and body of the user 10 including at least one of the caregiver and the care receiver, the control unit of the robot 100 decides an action of providing advice information related to caregiving corresponding to the recognized state as its own action. This makes it possible for the robot 100 to provide appropriate advice information related to caregiving appropriate for the state related to the mind and body of the user 10 including the caregiver and the care receiver.

In addition, when having recognized at least one of the degree of stress and the degree of fatigue of the user 10 as the state related to the mind and body of the user 10, the control unit of the robot 100 generates information related to the mental and physical recovery of the user 10 as the advice information based on at least one of the recognized degree of stress and degree of fatigue. This makes it possible for the robot 100 to provide, as the advice information, the information related to the mental and physical recovery of the user 10 appropriate for the degree of stress and the degree of fatigue in the user 10.

Furthermore, the user 10 constitutes a family. When having recognized at least one of the health state and the lifestyle of each of the plurality of users 10 constituting the family as the state related to the mind and body of the user 10, the control unit of the robot 100 decides an action of providing advice information related to health corresponding to the recognized state, as its own action. This makes it possible for the robot 100 to provide appropriate advice information related to health appropriate for the health state and lifestyle of the user 10.

In addition, based on at least one of the health state and the lifestyle, the control unit of the robot 100 generates information related to health management of the user 10 as advice information. This makes it possible for the robot 100 to provide, as the advice information, information related to health management appropriate for the health state and the lifestyle.

Furthermore, the user 10 constitutes a family. When having recognized the mental state of each of the plurality of users 10 constituting the family as the state related to the mind and body of the user 10, the control unit of the robot 100 decides an action of providing advice information related to the mental state corresponding to the recognized state, as its own action. This makes it possible for the robot 100 to provide appropriate advice information related to the mental state, appropriate for the mental state of the user 10.

In addition, the advice information includes advice information related to the mental state applicable among the plurality of users 10 constituting the family. With this configuration, by providing such advice information, the robot 100 can promote mental support among a plurality of users (in other words, among family members) constituting a family and assist mental care of each other, for example.

Although the robot 100 has been described above as an example of the electronic device, the electronic device is not limited to this, and may be another type of electronic device such as a robot having a stuffed toy appearance, a smartphone, or a smart speaker.

While the present invention has been described using the embodiments, the technical scope of the present invention is not limited to the scope described in the above embodiments. It is apparent to those skilled in the art that various modifications or improvements can be made to the above embodiments. It is apparent from the description of the claims that modes to which such changes or improvements have been added can also be included in the technical scope of the present invention.

It should be noted that the order of execution of each processing such as operations, procedures, steps, and stages in the devices, systems, programs, and methods illustrated in the claims, the specification, and the drawings can be implemented in any order unless "before", "prior to", or the like is explicitly stated, and unless the output of the previous processing is to be used in the subsequent processing. Descriptions using "First,", "Next,", and the like used for convenience in the operation flows in the claims, specification, and the drawings are not intended to indicate that it is essential to perform operations in the order described.

### Reference Signs List

- 5: SYSTEM
- 10, 11, 12: USER
- 20: COMMUNICATION NETWORK
- 100, 101, 102: ROBOT
- 200: SENSOR UNIT
- 201: MICROPHONE
- 202: 3D DEPTH SENSOR
- 203: 2D CAMERA
- 204: DISTANCE SENSOR
- 210: SENSOR MODULE UNIT
- 211: VOICE EMOTION RECOGNITION UNIT
- 212: UTTERANCE COMPREHENSION UNIT
- 213: EXPRESSION RECOGNITION UNIT
- 214: FACE RECOGNITION UNIT
- 220: STORING UNIT
- 221: REACTION RULE
- 222: HISTORY DATA
- 223: CHARACTER DATA
- 224: ASSIST RULE
- 225: SYMPTOM DATA
- 226: USER SUPPORT MODEL
- 230: USER STATE RECOGNITION UNIT
- 232: EMOTION DECISION UNIT
- 234: ACTION RECOGNITION UNIT
- 236: ACTION DECISION UNIT
- 238: STORAGE CONTROL UNIT
- 250: ACTION CONTROL UNIT
- 252: CONTROL TARGET
- 280: COMMUNICATION PROCESSING UNIT
- 290: EVENT DETECTION UNIT
- 300: SERVER
- 1200: COMPUTER
- 1210: HOST CONTROLLER
- 1212: CPU
- 1214: RAM
- 1216: GRAPHICS CONTROLLER
- 1218: DISPLAY DEVICE
- 1220: INPUT/OUTPUT CONTROLLER
- 1222: COMMUNICATION INTERFACE
- 1224: STORAGE DEVICE
- 1226: DVD DRIVE
- 1227: DVD-ROM
- 1230: ROM
- 1240: INPUT/OUTPUT CHIP
- 2901: DETECTOR
- 2902: COLLECTOR
- 2903: OUTPUT CONTROLLER
- 2911: HANDLING INFORMATION

## Claims

1. An action control system comprising:
a detection unit that detects occurrence of an event related to a circumstance of a user; and
an output controller that controls an electronic device including a text generation model to output information for adapting to the circumstance to the user in accordance with the event detected by the detection unit.

2. The action control system according to claim 1, further comprising an emotion decision unit that, in a case where the event is detected, increases a value of an emotion of being secure among a plurality of types of emotions prepared in advance as emotions of the electronic device, the emotions being emotions to each of which a value can be set,
wherein the output controller controls the electronic device to output information corresponding to the value of the emotion of being secure.

3. The action control system according to claim 1,
wherein the detection unit detects, as the event, a change in the circumstance of the user due to user's becoming an international student, and
the output controller controls the electronic device to output information related to a method of adapting to a changed environment and a changed place of residence.

4. The action control system according to claim 1,
wherein the detection unit detects a plan in which the user intends to change a job, as the event, and
the output controller controls the electronic device to output information related to preparation necessary for the change of the job.

5. The action control system according to claim 1,
wherein the detection unit detects a fact that the user has a child, as the event, and
the output controller controls the electronic device to output information related to extracurricular activities suitable for the child.

6. The action control system according to claim 1,
wherein the electronic device is either mounted on a stuffed toy or connected, by a wireless or wired link, to a control target device mounted on the stuffed toy.

7. An electronic device comprising:
an estimation unit that estimates an emotion of a user;
an acquisition unit that acquires information related to health and medical care of at least one of the user and a family member of the user; and
an assisting unit that performs assist corresponding to the emotion of the user estimated by the estimation unit, based on the information related to health and medical care of at least one of the user and a family member of the user acquired by the acquisition unit.

8. An electronic device comprising:
a recognition unit that recognizes a state of a user;
a physical condition estimation unit that estimates a physical condition of the user based on the state of the user recognized by the recognition unit; and
an assisting unit that performs assist based on the physical condition of the user estimated by the physical condition estimation unit.

9. An action control system comprising:
a detection unit that detects occurrence of an event which is an event related to a circumstance of a user and which is an event that causes a negative emotion in the user; and
an output controller that controls an electronic device including a text generation model to output information corresponding to the event detected by the detection unit, to the user.

10. An electronic device comprising
a control unit that recognizes an action of a user having a brain dysfunction, decides an own action corresponding to the recognized action of the user, and controls a control target based on the own action decided.

11. An electronic device comprising
a control unit that recognizes an action of an artist, decides an own action corresponding to the recognized action of the artist, and controls a control target based on the own action decided,
wherein the control unit decides an action related to a performance of the artist.

12. An electronic device comprising:
a recognition unit that recognizes a state of a child;
an emotion estimation unit that estimates an emotion of the child based on the state of the child recognized by the recognition unit; and
an assisting unit that performs assist corresponding to the emotion of the child estimated by the emotion estimation unit.

13. An action control system comprising:
an estimation unit that estimates a social situation of a user based on user information related to the user; and
an output controller that controls an electronic device including a text generation model to output, to the user, information corresponding to the situation estimated by the estimation unit.

14. An electronic device comprising
a control unit that recognizes study information of a user and determines an emotion of the user, has a conversation with the user based on the emotion determined, and controls a control target to provide the user with information corresponding to the study information recognized.

15. An electronic device comprising
a control unit that recognizes a mental state of a user, determines an emotion of the user, has a conversation with the user based on the emotion determined, and controls a control target so as to perform care in accordance with the mental state recognized.

16. An electronic device comprising
an action decision unit that decides a personalized countermeasure for a user in accordance with a state of an emotion of the user.

17. An electronic device comprising
a control unit that recognizes an action of a user performing a training, determines an emotion of the user, and controls a control target to provide the user with information related to the training based on the recognized action and the determined emotion.

18. An electronic device comprising
a control unit that recognizes at least one of a state of a user constituting a family and a state of a pet kept by the user, decides an own action corresponding to the recognized state, and controls a control target based on the own action decided.

19. An electronic device comprising
a control unit that recognizes a state related to mind and body of a user, decides an own action corresponding to the recognized state, and controls a control target based on the own action decided.
